# EUROPEAN PATENT APPLICATION

(11) **EP 4 850 751 A2**
(43) Date of publication of application: **09.12.2026**
(21) Application number: 26207469.3
(22) Date of filing: 22.07.2019
(51) Int. Cl.: A61P 3/10

(54) **GIP/GLP1 CO-AGONIST COMPOUNDS**

(30) Priority: 23.07.2018 US 201862702072 P; 13.09.2018 US 201862730563 P; 03.10.2018 US 201862740596 P
(62) Document number(s) of the earlier application(s) in accordance with Art. 76 EPC: 25155884.7 / 4 549 454; 19753218.7 / 3 827 015
(71) Applicant: Eli Lilly and Company, Indianapolis, IN 46285 (US)
(72) Inventor: ABRAHAM, Milata Mary, Indianapolis, 46206-6288 (US); ABURUB, Aktham, Indianapolis, 46206-6288 (US); ALSINA-FERNANDEZ, Jorge, Indianapolis, 46206-6288 (US); BROWN, Robert Andrew, Indianapolis, 46206-6288 (US); CABRERA, Over, Indianapolis, 46206-6288 (US); COSKUN, Tamer, Indianapolis, 46206-6288 (US); CUMMINS, Robert Chadwick, Indianapolis, 46206-6288 (US); DATTA-MANNAN, Amita, Indianapolis, 46206-6288 (US); ELSAYED, Mohamed ElSayed Hamed, Indianapolis, 46206-6288 (US); LAI, Xianyin, Indianapolis, 46206-6288 (US); PATEL, Phenil Jayantilal, Indianapolis, 46206-6288 (US); QU, Hongchang, Indianapolis, 46206-6288 (US); SLOOP, Kyle Wynn, Indianapolis, 46206-6288 (US); TRAN, Thi Thanh Huyen, Indianapolis, 46206-6288 (US); WALLIS, James Lincoln, Indianapolis, 46206-6288 (US); WILLARD, Francis Stafford, Indianapolis, 46206-6288 (US)
(74) Representative: Eli Lilly and Company Limited

(57) **Abstract**

The present invention relates to compounds having activity at both the human glucosedependent insulinotropic polypeptide (GIP) and glucagon-like peptide-1 (GLP-1) receptors. The present invention also relates to compounds having an extended duration of action at each of these receptors. Furthermore, the present invention relates to compounds that may be administered orally. Compounds may be useful in the treatment of type 2 diabetes mellitus ("T2DM"). Also, the compounds may be useful in the treatment of obesity.

## Description

The present invention relates to compounds having activity at both the human glucose-dependent insulinotropic polypeptide (GIP) and glucagon-like peptide-1 (GLP-1) receptors. The present invention also relates to compounds having an extended duration of action at each of these receptors. Furthermore, the present invention relates to compounds that may be administered orally. Compounds may be useful in the treatment of type 2 diabetes mellitus ("T2DM"). Also, the compounds may be useful in the treatment of obesity.

Over the past several decades, the prevalence of diabetes has continued to rise. T2DM is the most common form of diabetes accounting for approximately 90% of all diabetes. T2DM is characterized by high blood glucose levels associated mainly with insulin resistance. The current standard of care for T2DM includes diet and exercise, treatment with oral medications, and injectable glucose lowering drugs, including incretin-based therapies, such as GLP-1 receptor agonists. A variety of GLP-1 receptor agonists are currently available for treatment of T2DM, although currently marketed GLP-1 receptor agonists are generally dose-limited by gastrointestinal side effects such as nausea and vomiting. Subcutaneous injection is the typical route of administration for the available GLP-1 receptor agonists. When treatment with oral medications and incretin-based therapies are insufficient, insulin treatment is considered. Despite the advances in treatment available today, many patients with T2DM are unable to reach their glycemic control goals. Uncontrolled diabetes leads to several conditions associated with increased morbidity and mortality of patients. There is a need for a treatment to enable more patients with T2DM to reach their glycemic treatment goal.

Obesity is a complex medical disorder resulting in excessive accumulation of adipose tissue mass. Today obesity is a global public health concern that is associated with undesired health outcomes and morbidities. Desired treatments for patients with obesity strive to reduce excess body weight, improve obesity-related co-morbidities, and maintain long-term weight reduction. Available treatments for obesity are particularly unsatisfactory for patients with severe obesity. There is a need for alternative treatment options to induce therapeutic weight loss in patients in need of such treatment.

WO2016/111971 describes peptides stated to have GLP-1 and GIP activity. WO2013/164483 also discloses compounds stated to have GLP-1 and GIP activity.

There is a need for T2DM treatments capable of providing effective glucose control for a larger portion of the patients in need of such treatment. There is a further need for T2D treatments capable of providing effective glucose control and with a favorable side effect profile. There is a need for alternate treatment options to provide therapeutic weight loss in a patient in need of such treatment. There is a need for an alternate treatment option for a patient in need of treatment for severe obesity.

There is a desire for compounds having agonist activity at the GIP and GLP-1 receptors that are suitable for oral administration. Compounds with extended duration of action at each of the GIP and GLP-1 receptors are desirable to allow for less frequent dosing of the compound.

Accordingly, the present invention provides a compound of Formula I: 
R1X1X2X3GTX6TSDX10X11X12X13X14DX16X17AX19X20X21X22X23X24X25X26X27
X28X29X30X31 (SEQ ID NO:3)
 wherein:
R₁ is a modification of the N-terminal amino group wherein the modification is selected from the group consisting of Ac and absent;
X₁ is selected from the group consisting of Y, H, D-Tyr, F, desH, and desY,
X₂ is selected from the group consisting of Aib, αMeP, A, P, and D-Ala_{;}
or X₁ and X₂ combine to form desH-*ψ*[NHCO]-Aib;
X₃ is selected from the group consisting of E, N, Aad, and cTA;
X₆ is selected from the group consisting of F, αMeF, and αMeF(2F);
X₁₀ is selected from the group consisting of A, L, H, 3Pal, 4Pal, V, Y, E, αMeF, αMeF(2F), I, αMeY, Q, D-His, D-Tyr, cTA, and K(2-[2-(2-amino-ethoxy)-ethoxy]-acetyl)₂-(γ-Glu)-CO-(CH₂)qCO₂H;
X₁₁ is selected from the group consisting of S, αMeS, and D-Ser;
X₁₂ is selected from the group consisting of I, S, D-Ile, and K(2-[2-(2-amino-ethoxy)-ethoxy]-acetyl)₂-(γ-Glu)-CO-(CH₂)qCO₂H;
X₁₃ is selected from the group consisting of Nle, Aib, L, αMeL, and K(2-[2-(2-amino-ethoxy)-ethoxy]-acetyl)₂-(γ-Glu)-CO-(CH₂)qCO₂H;
X₁₄ is selected from the group consisting of L and K, wherein K is conjugated to a C₁₆-C₂₂ fatty acid wherein said fatty acid is optionally conjugated to said K via a linker;
X₁₆ is selected from the group consisting of K, E, Orn, Dab, Dap, S, T, H, Aib, αMeK, R, and K(2-[2-(2-amino-ethoxy)-ethoxy]-acetyl)₂-(γ-Glu)-CO-(CH₂)qCO₂H;
X₁₇ is selected from the group consisting of K, Q, I, and an amino acid conjugated to a C₁₆-C₂₂ fatty acid wherein said fatty acid is optionally conjugated to said amino acid via a linker;
X₁₉ is selected from the group consisting of Q, A, and K(2-[2-(2-amino-ethoxy)-ethoxy]-acetyl)₂-(γ-Glu)-CO-(CH₂)qCO₂H;
X₂₀ is selected from the group consisting of Aib, Q, H, R, K, αMeK, and K(2-[2-(2-amino-ethoxy)-ethoxy]-acetyl)₂-(γ-Glu)-CO-(CH₂)qCO₂H;
X₂₁ is selected from the group consisting of H, Aad, D, Aib, T, A, E, I, and K(2-[2-(2-amino-ethoxy)-ethoxy]-acetyl)₂-(γ-Glu)-CO-(CH₂)qCO₂H;
X₂₂ is selected from the group consisting of F and αMeF;
X₂₃ is selected from the group consisting of I, L, A, G, F, H, E, V, and K(2-[2-(2-amino-ethoxy)-ethoxy]-acetyl)₂-(γ-Glu)-CO-(CH₂)qCO₂H;
X₂₄ is selected from the group consisting of S, Aad, D-Glu, E, Aib, H, V, A, Q, D, P, and K(2-[2-(2-amino-ethoxy)-ethoxy]-acetyl)₂-(γ-Glu)-CO-(CH₂)qCO₂H;
X₂₅ is selected from the group consisting of Y and αMeY;
X₂₆ is selected from the group consisting of L, αMeL, and K(2-[2-(2-amino-ethoxy)-ethoxy]-acetyl)₂-(γ-Glu)-CO-(CH₂)qCO₂H;
X₂₇ is selected from the group consisting of L, I, and K(2-[2-(2-amino-ethoxy)-ethoxy]-acetyl)₂-(γ-Glu)-CO-(CH₂)qCO₂H;
X₂₈ is selected from the group consisting of E, A, S, D-Glu, and K(2-[2-(2-amino-ethoxy)-ethoxy]-acetyl)₂-(γ-Glu)-CO-(CH₂)qCO₂H;
X₂₉ is selected from the group consisting of Aib, G, A, and K(2-[2-(2-amino-ethoxy)-ethoxy]-acetyl)₂-(γ-Glu)-CO-(CH₂)qCO₂H;
X₃₀ is selected from the group consisting of C, G, G-R₂ and K(2-[2-(2-amino-ethoxy)-ethoxy]-acetyl)₂-(γ-Glu)-CO-(CH₂)_{q}-CO₂H;
X₃₁ is absent or is selected from the group consisting of PX₃₂X₃₃X₃₄-R₂ (SEQ ID NO:4), PX₃₂X₃₃X₃₄X₃₅X₃₆X₃₇X₃₈X₃₉-R₂ (SEQ ID NO:5),
PX₃₂X₃₃X₃₄X₃₅X₃₆X₃₇X₃₈X₃₉X₄₀-R₂ (SEQ ID NO:6)_{,} K[(2-[2-(2-amino-ethoxy)-ethoxy]-acetyl)₂-(γ-Glu)-CO-(CH₂)_{q}-CO₂H] X₃₂X₃₃X₃₄-R₂ (SEQ ID NO:7)_{,} K[(2-[2-(2-amino-ethoxy)-ethoxy]-acetyl)₂-(γ-Glu)-CO-(CH₂)_{q}-CO₂H]
X₃₂X₃₃X₃₄X₃₅X₃₆X₃₇X₃₈X₃₉-R₂ (SEQ ID NO:8)_{,} and K[(2-[2-(2-amino-ethoxy)-ethoxy]-acetyl)₂-(γ-Glu)-CO-(CH2)q-CO2H] X₃₂X₃₃X₃₄X₃₅X₃₆X₃₇X₃₈X₃₉X₄₀-R₂ (SEQ ID NO:9); wherein:
   X₃₂ is S or K[(2-[2-(2-amino-ethoxy)-ethoxy]-acetyl)₂-(γ-Glu)-CO-(CH₂)_{q}-CO₂H];
   X₃₃ is S or K[(2-[2-(2-amino-ethoxy)-ethoxy]-acetyl)₂-(γ-Glu)-CO-(CH₂)_{q}-CO₂H];
   X₃₄ is selected from the group consisting of G, C, and K[(2-[2-(2-amino-ethoxy)-ethoxy]-acetyl)₂-(γ-Glu)-CO-(CH₂)_{q}-CO₂H];
   X₃₅ is A or K[(2-[2-(2-amino-ethoxy)-ethoxy]-acetyl)₂-(γ-Glu)-CO-(CH₂)_{q}-CO₂H];
   X₃₆ is P or K[(2-[2-(2-amino-ethoxy)-ethoxy]-acetyl)₂-(γ-Glu)-CO-(CH₂)_{q}-CO₂H];
   X₃₇ is P or K[(2-[2-(2-amino-ethoxy)-ethoxy]-acetyl)₂-(γ-Glu)-CO-(CH₂)_{q}-CO₂H];
   X₃₈ is P or K[(2-[2-(2-amino-ethoxy)-ethoxy]-acetyl)₂-(γ-Glu)-CO-(CH₂)_{q}-CO₂H];
   X₃₉ is selected from the group consisting of C, S, and K[(2-[2-(2-amino-ethoxy)-ethoxy]-acetyl)₂-(γ-Glu)-CO-(CH₂)_{q}-CO₂H];
   X₄₀ is selected from the group consisting of C and K[(2-[2-(2-amino-ethoxy)-ethoxy]-acetyl)₂-(γ-Glu)-CO-(CH₂)_{q}-CO₂H];
q is selected from the group consisting of 14, 15, 16, 17, 18, 19, and 20; and R₂ is a modification of the C-terminal group, wherein the modification is NH₂ or absent;
or a pharmaceutically acceptable salt thereof;
wherein if X₃₀ is G-R₂, then X₃₁ is absent;
wherein no more than one of X₁₀, X₁₂, X₁₃, X₁₄, X₁₆, X₁₇, X₁₉, X₂₀, X₂₁, X_{23,} X₂₄, X₂₆, X₂₇, X₂₈, X₂₉, X₃₀, X₃₁, X₃₂, X₃₃, X₃₄, X₃₅, X₃₆, X₃₇, X₃₈, X₃₉, and X₄₀ may be a substituent that contains a fatty acid; and
wherein no more than one of X_{30,} X_{34,} X_{39,} and X₄₀ may be C; and
wherein if one of X_{30,} X_{34,} X_{39,} and X₄₀ is C, then none of X₁₀, X₁₂, X₁₃, X₁₄, X₁₆, X₁₇, X19, X20, X21, X23, X24, X26, X27, X28, X29, X30, X31, X32, X33, X34, X35, X36, X37, X38, X_{39,} and X₄₀ is a substituent that contains a fatty acid.

In an embodiment is a compound of Formula I, or a pharmaceutically acceptable salt thereof, wherein q is 16. In an embodiment is a compound of Formula I, or a pharmaceutically acceptable salt thereof, wherein X₃₁ is selected from the group consisting of SEQ ID NO:5 and SEQ ID NO:8. In an embodiment is a compound of Formula I, or a pharmaceutically acceptable salt thereof, wherein the X₁₇ amino acid that is conjugated to a fatty acid is a natural amino acid. In an embodiment is a compound of Formula I, or a pharmaceutically acceptable salt thereof, wherein X₁₇ is selected from the group consisting of K, Q and I.

In an embodiment is a compound of Formula I, or a pharmaceutically acceptable salt thereof, wherein K is conjugated to a C₁₆-C₂₂ fatty acid wherein said fatty acid is optionally conjugated to said K via a linker.

In an embodiment is a compound of Formula I, or a pharmaceutically acceptable salt thereof, wherein X₁₄ or X₁₇ is selected from the group consisting of K(2-[2-(2-Amino-ethoxy)-ethoxy]-acetyl)₂-(γ-Glu)-CO-(CH₂)₁₆-CO₂H, K(2-[2-(2-Amino-ethoxy)-ethoxy]-acetyl)₂-(γ-Glu)-CO-(CH₂)₁₈-CO₂H, K(2-[2-(2-Amino-ethoxy)-ethoxy]-acetyl)₂-(γ-Glu)-CO-(CH₂)₁₄-CO₂H, K(2-[2-(2-Amino-ethoxy)-ethoxy]-acetyl)₂-CO-(CH₂)₁₈-CO₂H, K(2-[2-(2-Amino-ethoxy)-ethoxy]-acetyl)-(γ-Glu)-(Trx)-CO-(CH₂)₁₈-CO₂H, K(2-[2-(2-Amino-ethoxy)-ethoxy]-acetyl)-(Trx)-(γ-Glu)-CO-(CH₂)₁₈-CO₂H, K(2-[2-(2-Amino-ethoxy)-ethoxy]-acetyl)-(εK)-(γ-Glu)-CO-(CH₂)₁₈-CO₂H, K(2-[2-(2-Amino-ethoxy)-ethoxy]-acetyl)-(εK)-(εK)-CO-(CH₂)₁₈-CO₂H, K(2-[2-(2-Amino-ethoxy)-ethoxy]-acetyl)₂-(γ-Glu)₂-CO-(CH₂)₁₈-CO₂H, K(2-[2-(2-Amino-ethoxy)-ethoxy]-acetyl)₂-CO-(CH₂)₁₈-CO₂H, K(2-[2-(2-Amino-ethoxy)-ethoxy]-acetyl)₂-(eK)-CO-(CH₂)₁₆-CO₂H, K(2-[2-(2-Amino-ethoxy)-ethoxy]-acetyl)₂-(εK)-CO-(CH₂)₁₄-CO₂H, and KDab-(2-[2-(2-Amino-ethoxy)-ethoxy]-acetyl)-Dab-(2-[2-(2-Amino-ethoxy)-ethoxy]-acetyl)-CO-(CH₂)₁₈-CO₂H.

In an embodiment is a compound of Formula I, or a pharmaceutically acceptable salt thereof, wherein X₁₄ or X₁₇ is selected from the group consisting of K(2-[2-(2-Amino-ethoxy)-ethoxy]-acetyl)₂-(γ-Glu)-CO-(CH₂)₁₆-CO₂H, K(2-[2-(2-Amino-ethoxy)-ethoxy]-acetyl)₂-(γ-Glu)-CO-(CH₂)₁₈-CO₂H, K(2-[2-(2-Amino-ethoxy)-ethoxy]-acetyl)₂-(γ-Glu)-CO-(CH₂)₁₄-CO₂H, and K(2-[2-(2-Amino-ethoxy)-ethoxy]-acetyl)₂-CO-(CH₂)₁₈-CO₂H.

In an embodiment is a compound of Formula I, or a pharmaceutically acceptable salt thereof, wherein X₁₄ or X₁₇ is selected from the group consisting of K(2-[2-(2-Amino-ethoxy)-ethoxy]-acetyl)₂-(γ-Glu)-CO-(CH₂)₁₆-CO₂H, K(2-[2-(2-Amino-ethoxy)-ethoxy]-acetyl)₂-(γ-Glu)-CO-(CH₂)₁₈-CO₂H, and K(2-[2-(2-Amino-ethoxy)-ethoxy]-acetyl)₂-(γ-Glu)-CO-(CH₂)₁₄-CO₂H. In an embodiment is a compound of Formula I, or a pharmaceutically acceptable salt thereof, wherein X₁₄ or X₁₇ is selected from the group consisting of K(2-[2-(2-Amino-ethoxy)-ethoxy]-acetyl)₂-(γ-Glu)-CO-(CH₂)₁₈-CO₂H and K(2-[2-(2-Amino-ethoxy)-ethoxy]-acetyl)₂-(γ-Glu)-CO-(CH₂)₁₆-CO₂H. In an embodiment is a compound of Formula I, or pharmaceutically acceptable salt thereof, wherein X₁₄ or X₁₇ is K(2-[2-(2-amino-ethoxy)-ethoxy]-acetyl)ₐ-(γ-Glu)_{b}-CO-(CH₂)_{q}-CO₂H, wherein a is 2, b is 1, and q is selected from the group consisting of 18 and 20. In an embodiment is a compound of Formula I, or pharmaceutically acceptable salt thereof, wherein X₁₄ or X₁₇ is K(2-[2-(2-amino-ethoxy)-ethoxy]-acetyl)ₐ-(γ-Glu)_{b}-CO-(CH₂)_{q}-CO₂H, wherein a is 2, b is 1 and q is 18. In an embodiment is a compound of Formula I, or pharmaceutically acceptable salt thereof, wherein X₁₄ or X₁₇ is K(2-[2-(2-amino-ethoxy)-ethoxy]-acetyl)ₐ-(γ-Glu)_{b}-CO-(CH₂)_{q}-CO₂H, wherein, a is 2, b is 1, and q is 20.

In an embodiment is a Formula I compound, or pharmaceutically acceptable salt thereof, wherein X₁ and X₂ do not combine to form desH-*ψ*[NHCO]-Aib (hereafter a "Formula II" compound).

In an embodiment is a compound of Formula I, or pharmaceutically acceptable salt thereof, wherein:
X₁₇ is an amino acid conjugated to a C₁₆-C₂₂ fatty acid wherein said fatty acid is optionally conjugated to said amino acid via a linker; and
X₃₀ is selected from the group consisting of G-R₂ and G;
wherein if X₃₀ is G, then X₃₁ is selected from the group consisting of PX₃₂X₃₃X₃₄-R₂ (SEQ ID NO:4), wherein X₃₂ is S, X₃₃ is S and X₃₄ is G (SEQ ID NO:297), and PX₃₂X₃₃X₃₄X₃₅X₃₆X₃₇X₃₈X₃₉-R₂ (SEQ ID NO:5), wherein X₃₂ is S, X₃₃ is S, X₃₄ is G, X₃₅ is A, X₃₆ is P, X₃₇ is P, X₃₈ is P and X₃₉ is S (SEQ ID NO:298) (hereafter a "Formula III" compound).

In an embodiment is a compound of Formula III, or a pharmaceutically acceptable salt thereof, wherein the X₁₇ amino acid is conjugated to the fatty acid via a linker (hereafter a "Formula IIIa" compound).

In an embodiment is a compound of Formula III and IIIa, or a pharmaceutically acceptable salt thereof, wherein:
X₁₀ is selected from the group consisting of A, L, H, 3Pal, 4Pal, V, Y, E, αMeF, αMeF(2F), I, αMeY, Q, D-His, D-Tyr, and cTA;
X₁₂ is selected from the group consisting of I, S, and D-Ile;
X₁₃ is selected from the group consisting of Nle, Aib, L, and αMeL;
X₁₄ is selected from the group consisting of L and K;
X₁₆ is selected from the group consisting of K, E, Orn, Dab, Dap, S, T, H, Aib, αMeK, and R;
X₁₉ is selected from the group consisting of Q, and A;
X₂₀ is selected from the group consisting of Aib, Q, H, R, K, and αMeK;
X₂₁ is selected from the group consisting of H, Aad, D, Aib, T, A, E, and I;
X₂₃ is selected from the group consisting of I, L, A, G, F, H, E, and V;
X₂₄ is selected from the group consisting of S, Aad, D-Glu, E, Aib, H, V, A, Q, D, and P;
X₂₆ is selected from the group consisting of L, and αMeL;
X₂₇ is selected from the group consisting of L, and I;
X₂₈ is selected from the group consisting of E, A, S, and D-Glu;
X₂₉ is selected from the group consisting of Aib, G, and A;
X₃₀ is selected from the group consisting of G and G-R₂;
wherein if X₃₀ is G; then X₃₁ is selected from the group consisting of PX₃₂X₃₃X₃₄-R₂ (SEQ ID NO:4), wherein X₃₂ is S, X₃₃ is S and X₃₄ is G (SEQ ID NO:297) and PX₃₂X₃₃X₃₄X₃₅X₃₆X₃₇X₃₈X₃₉-R₂ (SEQ ID NO:5), wherein X₃₂ is S, X₃₃ is S, X₃₄ is G, X₃₅ is A, X₃₆ is P, X₃₇ is P, X₃₈ is P and X₃₉ is S (SEQ ID NO:298) (hereafter a "Formula IIIb" compound).

In an embodiment, is a compound of Formula III, IIIa and IIIb, or a pharmaceutically acceptable salt thereof, wherein the linker comprises from 1 to 2 amino acids, and in a further embodiment of these particular Formula III, IIIa and IIIb compounds are those wherein the linker amino acids are independently selected from the group consisting of Glu and γ-Glu. In another embodiment is a compound of Formula III, IIIa and IIIb, or a pharmaceutically acceptable salt thereof, wherein the linker comprises from one or two (2-[2-(2-amino-ethoxy)-ethoxy]-acetyl) moieties and in a further embodiment of these particular formula III, IIIa and IIIb compounds are those where the linker is (2-[2-(2-amino-ethoxy)-ethoxy]-acetyl)ₐ-(γ-Glu)_{b}, wherein a is selected from the group consisting of 1 or 2; and b is selected from the group consisting of 1 or 2.

In an embodiment is a compound of Formula III, or a pharmaceutically acceptable salt thereof, wherein X₁₇ is an amino acid conjugated to a C₁₆-C₂₂ fatty acid, wherein the amino acid is K and wherein said fatty acid is optionally conjugated to said amino acid via a linker.

In an embodiment is a compound of Formula III, or pharmaceutically acceptable salt thereof, wherein:
R₁ is absent;
X₁ and X₂ do not combine to form desH-*ψ*[NHCO]-Aib;
X₁₇ is K conjugated to a C₁₆-C₂₂ fatty acid wherein said fatty acid is optionally conjugated to said amino acid via a linker.

In an embodiment is a compound of Formula III, or pharmaceutically acceptable salt thereof, wherein:
X₁ is Y;
X₂ is Aib;
X₃ is E;
X₁₀ is selected from the group consisting of A, L, H, 3Pal, 4Pal, V, and Y;
X₁₁ is S;
X₁₂ is I;
X₁₄ is L;
X₁₆ is selected from the group consisting of K, E, Orn, Dab, and Dap;
X₁₇ is K conjugated to a C₁₆-C₂₂ fatty acid wherein said fatty acid is optionally conjugated to said amino acid via a linker;
X₁₉ is Q;
X₂₀ is Aib;
X₂₁ is selected from the group consisting of H, Aad, D, Aib, T, A, and E;
X₂₂ is F;
X₂₃ is I;
X₂₄ is selected from the group consisting of S, Aad, D-Glu, and E;
X₂₆ is L; and
X₂₈ is selected from the group consisting of E and A.

In an embodiment is a compound of Formula III, or pharmaceutically acceptable salt thereof, wherein:
X₁ is Y;
X₂ is Aib;
X₃ is E;
X₆ is αMeF(2F);
X₁₀ is selected from the group consisting of Y, 4-PaI, and V;
X₁₁ is S;
X₁₂ is I;
X₁₃ is selected from the group consisting of L, Aib, and αMeL;
X₁₄ is L;
X₁₆ is selected from the group consisting of E, K, and Orn;
X₁₇ is K conjugated to a C₁₆-C₂₂ fatty acid wherein said fatty acid is optionally conjugated to said amino acid via a linker;
X₁₉ is Q;
X₂₀ is Aib
X₂₁ is selected from the group consisting of E, A, and T;
X₂₂ is F;
X₂₃ is I;
X₂₄ is D-Glu;
X₂₅ is selected from the group consisting of Y and αMeY;
X₂₆ is L;
X₂₇ is I;
X₂₈ is E;
X₂₉ is G;
X₃₀ is G; and
X₃₁ is PX₃₂X₃₃X₃₄X₃₅X₃₆X₃₇X₃₈X₃₉-R₂ (SEQ ID NO:5), wherein X₃₂ is S, X₃₃ is S, X₃₄ is G, X₃₅ is A, X₃₆ is P, X₃₇ is P, X₃₈ is P, X₃₉ is S (SEQ ID NO:298).

In an embodiment is a compound of Formula III, IIIa and IIIb, or a pharmaceutically acceptable salt thereof, wherein R₂ is absent.

In an embodiment is a compound of Formula III, IIIa and IIIb, or a pharmaceutically

acceptable salt thereof, wherein R₂ is NH₂.

In an embodiment is a compound of Formula III, IIIa and IIIb, or a pharmaceutically acceptable salt thereof, wherein X₁₃ is αMeL.

In an embodiment is a compound of Formula III, IIIa and IIIb, or a pharmaceutically acceptable salt thereof, wherein X₂₅ is Y and X₁₃ is αMeL.

In an embodiment is a compound of Formula III, IIIa and IIIb, or a pharmaceutically acceptable salt thereof, wherein X₁₇ is K conjugated to a fatty acid via a linker to the epsilon-amino group of the K side-chain wherein said fatty acid and linker have the following formula:
(2-[2-(2-amino-ethoxy)-ethoxy]-acetyl)ₐ-(γ-Glu)_{b}-CO-(CH₂)_{q}-CO₂H, wherein a is 1 or 2; b is 1 or 2; and q is selected from the group consisting of 14 to 20.

In an embodiment is a compound of Formula III, IIIa and IIIb, or a pharmaceutically acceptable salt thereof, wherein X₁₆ is Orn, X₁₃ is αMeL, and X₂₅ is Y. In an embodiment is a compound of Formula III, IIIa and IIIb, or a pharmaceutically acceptable salt thereof, wherein X₁₆ is E, X₁₃ is αMeL, and X₂₅ is Y. In an embodiment, is a compound of Formula III, IIIa and IIIb, or a pharmaceutically acceptable salt thereof, wherein X₁₆ is E, X₁₃ is αMeL, X₁₀ is Y, and X₂₅ is αMeY. In an embodiment is a compound of Formula III, IIIa and IIIb, or a pharmaceutically acceptable salt thereof, wherein X₁₆ is Orn, X₁₃ is αMeL, X₁₀ is 4Pal, and X₂₅ is Y. In an embodiment is a compound of Formula III, IIIa and IIIb, or a pharmaceutically acceptable salt thereof, wherein X₁₆ is Orn, X₁₃ is αMeL, X₁₀ is V, and X₂₅ is Y. In an embodiment is a compound of Formula III, IIIa and IIIb, or a pharmaceutically acceptable salt thereof, wherein X₁₆ is E, X₁₃ is αMeL, X₂₅ is Y, and X₁₇ is K(2-[2-(2-Amino-ethoxy)-ethoxy]-acetyl)ₐ-(γ-Glu)_{b}-CO-(CH₂)_{q}-CO₂H, wherein a is 2; b is 1; and q is selected from the group consisting of 14 to 20. In an embodiment is a compound of Formula III, IIIa and IIIb, or a pharmaceutically acceptable salt thereof, wherein X₁₆ is E, X₁₃ is αMeL, X₁₀ is Y, and X₂₅ is Y and and X₁₇ is K(2-[2-(2-Amino-ethoxy)-ethoxy]-acetyl)ₐ-(γ-Glu)_{b}-CO-(CH₂)_{q}-CO₂H, wherein a is 2; b is 1; and q is selected from the group consisting of 16 to 20.

In an embodiment is a compound of Formula I selected from the group consisting of SEQ ID NO:10, SEQ ID NO:11, SEQ ID NO:12, SEQ ID NO:13, and SEQ ID NO:14, or a pharmaceutically acceptable salt thereof. In an embodiment is a compound of Formula I that is SEQ ID NO:10, or a pharmaceutically acceptable salt thereof. In an embodiment is a compound of Formula I that is SEQ ID NO:11, or a pharmaceutically acceptable salt thereof. In an embodiment is a compound of Formula I that is SEQ ID NO:12, or a pharmaceutically acceptable salt thereof. In an embodiment is a compound of Formula I that is SEQ ID NO:13, or a pharmaceutically acceptable salt thereof. In an embodiment is a compound of Formula I that is SEQ ID NO:14, or a pharmaceutically acceptable salt thereof.

In an embodiment is a compound of Formula I, or a pharmaceutically acceptable salt thereof, wherein X₁ is selected from the group consisting of Y, F, and D-Tyr; X₆ is F; and X₁₃ is selected from the group consisting of Aib, L, and αMeL.

In an embodiment, is a compound of Formula I, or a pharmaceutically acceptable salt thereof, wherein R₁ is absent; X₁ is selected from the group consisting of Y, F, and D-Tyr; X₆ is F; X₁₃ is selected from the group consisting of Aib, L, and αMeL; X₂ is Aib; X₃ is E; X₁₀ is Y; X₁₁ is S; X₁₂ is I; X₁₄ is L; X₁₆ is selected from the group consisting of K, E, Orn, Dab, Dap, S, T, H, Aib, αMeK, and R; X₁₇ is an amino acid conjugated to a C₁₆-C₂₂ fatty acid wherein said fatty acid is optionally conjugated to said amino acid via a linker; X₁₉ is Q; X₂₀ is selected from the group consisting of Aib, Q, H, and K; X₂₁ is selected from the group consisting of H, D, T, A, and E; X₂₂ is F; X₂₃ is I; X₂₄ is selected from the group consisting of D-Glu and E; X₂₆ is L; X₂₇ is I; X₂₈ is selected from the group consisting of E, A, S, and D-Glu; X₂₉ is selected from the group consisting of Aib, G, and A; X₃₀ is selected from the group consisting of C, G, and G-R₂; X₃₁ is absent or is selected from the group consisting of PX₃₂X₃₃X₃₄-R₂ (SEQ ID NO:4), PX₃₂X₃₃X₃₄X₃₅X₃₆X₃₇X₃₈X₃₉-R₂ (SEQ ID NO:5)_{,} and PX₃₂X₃₃X₃₄X₃₅X₃₆X₃₇X₃₈X₃₉X₄₀-R₂ (SEQ ID NO:6); wherein: X₃₂ is S; X₃₃ is S; X₃₄ is selected from the group consisting of G and C; X₃₅ is A; X₃₆ is P; X₃₇ is P; X₃₈ is P; X₃₉ is selected from the group consisting of C and S; and X₄₀ is C.

In an embodiment, is a compound of Formula I, or a pharmaceutically acceptable salt thereof, wherein X₁ is selected from the group consisting of Y, F, and D-Tyr; X₆ is F; and X₁₃ is selected from the group consisting of Aib, L, and αMeL; X₂₈ is A; X₂₉ G; X₃₀ is G; X₃₁ is PX₃₂X₃₃X₃₄X₃₅X₃₆X₃₇X₃₈X₃₉-R₂ (SEQ ID NO:5); X₃₄ is G; and X₃₉ is S_{.}

In an embodiment, is a compound of Formula I, or a pharmaceutically acceptable salt thereof, wherein X₁ is selected from the group consisting of Y and D-Tyr; and X₁₃ αMeL.

In an embodiment is a compound of Formula I selected from the group consisting of SEQ ID NO:303, SEQ ID NO:304, SEQ ID NO:305, SEQ ID NO:306, SEQ ID NO:307, and SEQ ID NO:308, or a pharmaceutically acceptable salt thereof. In an embodiment is a compound of Formula I that is SEQ ID NO:303, or a pharmaceutically acceptable salt thereof. In an embodiment is a compound of Formula I that is SEQ ID NO:304, or a pharmaceutically acceptable salt thereof. In an embodiment is a compound of Formula I that is SEQ ID NO:305, or a pharmaceutically acceptable salt thereof. In an embodiment is a compound of Formula I that is SEQ ID NO:306, or a pharmaceutically acceptable salt thereof. In an embodiment is a compound of Formula I that is SEQ ID NO:307, or a pharmaceutically acceptable salt thereof. In an embodiment is a compound of Formula I that is SEQ ID NO:308, or a pharmaceutically acceptable salt thereof. In an embodiment is a compound of Formula I that is SEQ ID NO:386, or a pharmaceutically acceptable salt thereof.

In an embodiment is a compound of Formula I, or a pharmaceutically acceptable salt thereof, wherein:
X₁₀ is selected from the group consisting of A, L, H, 3Pal, 4Pal, V, Y, αMeF, αMeF(2F), I, αMeY, Q, D-His, E, cTA, and D-Tyr;
X₁₂ is selected from the group consisting of I, D-Ile, and S;
X₁₃ is selected from the group consisting of Nle, Aib, L, and αMeL;
X₁₄ is L;
X₁₆ is selected from the group consisting of K, E, Orn, Dab, Dap, S, T, H, Aib, αMeK, and R;
X₁₇ is selected from the group consisting of K, Q, and I;
X₁₉ is selected from the group consisting of Q and A;
X₂₀ is selected from the group consisting of Aib, Q, H, R, K, and αMeK;
X₂₁ is selected from the group consisting of H, Aad, D, Aib, T, A, E, and I;
X₂₃ is selected from the group consisting of I, L, A, G, F, H, E, and V;
X₂₄ is selected from the group consisting of S, Aad, D-Glu, E, Aib, H, V, A, Q, D, and P;
X₂₆ is selected from the group consisting of L and αMeL;
X₂₇ is selected from the group consisting of L and I;
X₂₈ is selected from the group consisting of E, A, S, and D-Glu; and
X₂₉ is selected from the group consisting of Aib, G, and A (hereafter a "Formula IV" compound).

In an embodiment of is a compound of Formula IV, or a pharmaceutically acceptable salt thereof wherein X₃₉ is C. In an embodiment is a compound of Formula IV, or a pharmaceutically acceptable salt thereof wherein X₄₀ is C.

In an embodiment is a compound of Formula IV, or a pharmaceutically acceptable salt thereof, wherein one, and only one, of X_{30,} X_{34,} X_{39,} and X₄₀ is C. In an embodiment is a compound of Formula IV, or a pharmaceutically acceptable salt thereof, wherein one, and only one, of X_{30,} X_{34,} X_{39,} and X₄₀ is C modified using time-extension technology. In an embodiment is a compound of Formula IV, or pharmaceutically acceptable salt thereof, wherein C is modified using time-extension technology wherein the time-extension technology is XTEN. In an embodiment is a compound of Formula IV, or pharmaceutically acceptable salt thereof, wherein C is modified using time-extension technology wherein the time-extension technology is a (Glu)ₘ biotin wherein m is 0, 1, 2, or 3. In an embodiment is a compound of Formula IV, or a pharmaceutically acceptable salt thereof, wherein:
X₁ is Y;
X₂ is Aib;
X₃ is E;
X₁₀ is selected from the group consisting of A, L, H, 3Pal, 4Pal, V, and Y;
X₁₁ is S;
X₁₂ is I;
X₁₆ is selected from the group consisting of K, E, Orn, Dab, and Dap;
X₁₉ is Q;
X₂₀ is selected from the group consisting of Aib and K;
X₂₁ is selected from the group consisting of H, Aad, D, Aib, T, A, and E;
X₂₂ is F;
X₂₃ is I;
X₂₄ is selected from the group consisting of S, Aad, D-Glu, and E;
X₂₆ is L; and
X₂₈ is selected from the group consisting of E and A;
or a pharmaceutically acceptable salt thereof.

In an embodiment is a compound of Formula IV, or a pharmaceutically acceptable salt thereof, wherein
X₁ is Y;
X₂ is Aib;
X₃ is E;
X₁₀ is selected from the group consisting of A, L, H, 3Pal, 4Pal, V, and Y;
X₁₁ is S;
X₁₂ is I;
X₁₆ is selected from the group consisting of K, E, Orn, Dab, and Dap;
X₂₀ is Aib;
X₂₁ is selected from the group consisting of H, Aad, D, Aib, T, A, and E;
X₂₂ is F;
X₂₄ is selected from the group consisting of S, Aad, D-Glu, and E;
X₂₇ is I; and
X₂₈ is selected from the group consisting of E and A.

In an embodiment is a compound of Formula I, or a pharmaceutical salt thereof, wherein:
X₁₄ is L;
X₁₇ is selected from the group consisting of K, Q, and I;
X₃₀ is selected from the group consisting of G-R₂ and G; and
q is selected from the group consisting of 16, 18, and 20;
wherein if X₃₀ is G, then X₃₁ is selected from the group consisting of:
   PX₃₂X₃₃X₃₄-R₂ (SEQ ID NO:4), wherein:
      X₃₂ is S, X₃₃ is S, X₃₄ is G and R₂ is absent (SEQ ID NO:299) or
      X₃₂ is S, X₃₃ is S, X₃₄ is G and R₂ is NH₂ (SEQ ID NO:300);
         and
   PX₃₂X₃₃X₃₄X₃₅X₃₆X₃₇X₃₈X₃₉-R₂ (SEQ ID NO:5), wherein:
      X₃₂ is S, X₃₃ is S, X₃₄ is G, X₃₅ is A, X₃₆ is P, X₃₇ is P, X₃₈ is P, X₃₉ is S and R₂ is absent (SEQ ID NO:301) or
      X₃₂ is S, X₃₃ is S, X₃₄ is G, X₃₅ is A, X₃₆ is P, X₃₇ is P, X₃₈ is P, X₃₉ is S and R₂ is NH₂ (SEQ ID NO:302); and
   wherein one of X₁₀, X₁₂, X₁₃, X_{14,} X₁₆, X₁₉, X₂₀, X₂₁, X_{23,} X₂₄, X₂₆, X₂₇, X₂₈, and X₂₉ is K(2-[2-(2-amino-ethoxy)-ethoxy]-acetyl)₂-γGlu-CO-(CH₂)qCO₂H (hereafter a "Formula V" compound).

In an embodiment is a compound of Formula V, or a pharmaceutically acceptable salt
thereof, wherein:
X₁ is Y;
X₂ is Aib;
X₃ is E;
X₁₀ is selected from the group consisting of A, L, H, 3Pal, 4Pal, V, Y, E, cTA, and K(2-[2-(2-amino-ethoxy)-ethoxy]-acetyl)₂-(γ-Glu)-CO-(CH₂)qCO₂H;
X₁₁ is S;
X₁₂ is selected from the group consisting of I, D-Ile, and K(2-[2-(2-amino-ethoxy)-ethoxy]-acetyl)₂-(γ-Glu)-CO-(CH₂)qCO₂H;
X₁₆ is selected from the group consisting of K, E, Orn, Dab, Dap, and K(2-[2-(2-amino-ethoxy)-ethoxy]-acetyl)₂-(γ-Glu)-CO-(CH₂)qCO₂H;
X₁₇ is selected from the group consisting of K and I;
X₁₉ is selected from the group consisting of Q and K(2-[2-(2-amino-ethoxy)-ethoxy]-acetyl)₂-(γ-Glu)-CO-(CH₂)qCO₂H;
X₂₀ is selected from the group consisting of Aib and K(2-[2-(2-amino-ethoxy)-ethoxy]-acetyl)₂-(γ-Glu)-CO-(CH₂)qCO₂H;
X₂₁ is selected from the group consisting of H, Aad, D, Aib, T, A, E, and K(2-[2-(2-amino-ethoxy)-ethoxy]-acetyl)₂-(γ-Glu)-CO-(CH₂)qCO₂H;
X₂₂ is F;
X₂₄ is selected from the group consisting of S, Aad, D-Glu, E, and K(2-[2-(2-amino-ethoxy)-ethoxy]-acetyl)₂-(γ-Glu)-CO-(CH₂)qCO₂H;
X₂₆ is selected from the group consisting of L and K(2-[2-(2-amino-ethoxy)-ethoxy]-acetyl)₂-(γ-Glu)-CO-(CH₂)qCO₂H;
X₂₇ is selected from the group consisting of L and I; and
X₂₈ is selected from the group consisting of E, A, and K(2-[2-(2-amino-ethoxy)-ethoxy]-acetyl)₂-(γ-Glu)-CO-(CH₂)qCO₂H.

In an embodiment is a compound of Formula V, or a pharmaceutically acceptable salt thereof, wherein X₂₀ is K(2-[2-(2-amino-ethoxy)-ethoxy]-acetyl)₂-(γ-Glu)-CO-(CH₂)qCO₂H, wherein q is 16 or 18. In an embodiment is a compound of Formula V, or a pharmaceutically acceptable salt thereof, wherein X₃₁ is SEQ ID NO:301 or SEQ ID NO:302.

An embodiment provides a method of treating a condition selected from the group consisting of T2DM, obesity, nonalcoholic fatty liver disease (NAFLD), nonalcoholic steatohepatitis (NASH), dyslipidemia and metabolic syndrome, comprising administering to a subject in need thereof, an effective amount of a compound of Formula I or a pharmaceutically acceptable salt thereof. An embodiment provides a method for providing therapeutic weight loss comprising administering to a subject in need thereof, an effective amount of a compound of Formula I, or a pharmaceutically acceptable salt thereof. In one embodiment, the condition is NAFLD. In one embodiment, the condition is NASH.

An embodiment provides a compound of Formula I, or a pharmaceutically acceptable salt thereof, for use in therapy. An embodiment provides a compound of Formula I, or a pharmaceutically acceptable salt thereof, for use in therapy to treat a condition selected from the group consisting of T2DM, obesity, NAFLD, NASH, dyslipidemia and metabolic syndrome. In an embodiment, the condition is T2DM. In an embodiment, the condition is obesity. In an embodiment, the condition is NAFLD. In an embodiment, the condition is NASH. In an embodiment, the condition is metabolic syndrome.

The compounds of Formula I, or a pharmaceutically acceptable salt thereof, may be useful in the treatment of a variety of symptoms or disorders. For example, certain embodiments, provide a method for treatment of T2DM in a patient comprising administering to a subject in need of such treatment an effective amount of a compound of Formula I, or a pharmaceutically acceptable salt thereof. In an embodiment, is a method for treatment of obesity in a patient comprising administering to a subject in need of such treatment an effective amount of a compound of Formula I, or a pharmaceutically acceptable salt thereof. In an embodiment, the method is inducing non-therapeutic weight loss in a subject, comprising administering to a subject in need of such treatment an effective amount of a compound of Formula I, or a pharmaceutically acceptable salt thereof.

In certain embodiments, the present invention provides a method for treatment of metabolic syndrome in a patient comprising administering to a subject in need of such treatment an effective amount of a compound of Formula I, or a pharmaceutically acceptable salt thereof. In an embodiment, the method is treatment of NASH comprising administering to a subject in need of such treatment an effective amount of a compound of Formula I, or a pharmaceutically acceptable salt thereof.

Also provided herein is a compound of the present invention for use in simultaneous, separate and sequential combinations with one or more agents selected from metformin, a thiazolidinedione, a sulfonylurea, a dipeptidyl peptidase 4 inhibitor, a sodium glucose co-transporter, a SGLT-2 inhibitor, a growth differentiation factor 15 modulator ("GDF15"), a peptide tyrosine tyrosine modulator ("PYY"), a modified insulin, amylin, a dual amylin calcitonin receptor agonist, and oxyntomodulin agonist ("OXM") in the treatment of a condition selected from the group consisting of T2DM, obesity, NAFLD, NASH, dyslipidemia and metabolic syndrome. In an embodiment, a compound of the present invention is provided in a fixed dose combination with one or more agents selected from metformin, a thiazolidinedione, a sulfonylurea, a dipeptidyl peptidase 4 inhibitor, a sodium glucose co-transporter, a SGLT-2 inhibitorGDF15, PYY, a modified insulin, amylin, a dual amylin calcitonin receptor agonist, and OXM. In an embodiment is a compound of the present invention for use in simultaneous, separate and sequential combinations with one or more agents selected from metformin, a thiazolidinedione, a sulfonylurea, a dipeptidyl peptidase 4 inhibitor, a sodium glucose co-transporter, a SGLT-2 inhibitor, GDF15, PYY, a modified insulin, amylin, a dual amylin calcitonin receptor agonist, and OXM in the treatment of a condition selected from the group consisting of T2DM and obesity. In an embodiment is a compound of the present invention for use in simultaneous, separate and sequential combinations with one or more agents selected from metformin, a thiazolidinedione, a sulfonylurea, a dipeptidyl peptidase 4 inhibitor, a sodium glucose co-transporter, and a SGLT-2 inhibitor in the treatment of a condition selected from the group consisting of T2DM and obesity.

In other embodiments, the compounds, or a pharmaceutically acceptable salt thereof, may be useful to improve bone strength in subjects in need thereof. The compounds of the present invention, or a pharmaceutically acceptable salt thereof, may be useful in the treatment of other disorders such as Parkinson's disease or Alzheimer's disease. Incretins and incretin analogs having activity at one or more of the GIP, GLP-1 and/or glucagon receptors have been described as having the potential to have therapeutic value in a number of other diseases or conditions, including for example obesity, NAFLD and NASH, dyslipidemia, metabolic syndrome, bone related disorders, Alzheimer's disease, and Parkinson's disease. *See, e.g*., Jall S., et. al, Monomeric GLP-1/GIP/glucagon triagonism corrects obesity, hepatosteatosis, and dyslipidemia in female mice, MOL. METAB. 6(5):440-446 (March 2017); Carbone L.J., et. al., Incretin-based therapies for the treatment of non-alcoholic fatty liver disease: A systematic review and meta-analysis. J.GASTROENTEROL. HEPATOL., 31(1):23-31 (Jan. 2016); B. Finan, et. al, Reappraisal of GIP Pharmacology for Metabolic Diseases. TRENDS MOL. MED., 22(5):359-76 (May 2016); Choi, I.Y., et al., Potent body weight loss and efficacy in a NASH animal model by a novel long-acting GLP-1/Glucagon/GIP triple-agonist (HM15211), ADA 2017 Poster 1139-P; Ding, K.H., Impact of glucose-dependent insulinotropic peptide on age-induced bone loss, J. BONE MINER. RES., 23(4):536-43 (2008); Tai, J. et. al, Neuroprotective effects of a triple GLP-1/GIP/glucagon receptor agonist in the APP/PS1 transgenic mouse model of Alzheimer's disease, BRAIN RES. 1678, 64-74 (2018); T.D. Müller et al., The New Biology and Pharmacology of Glucagon, PHYSIOL. REV. 97: 721-766 (2017); Finan, B. et. al, Unimolecular Dual Incretins Maximize Metabolic Benefits in Rodents, Monkeys, and Humans, SCI. TRANSL. MED., 5:209 (October 2013); Hölscher C, Insulin, incretins and other growth factors as potential novel treatments for Alzheimer's and Parkinson's diseases. BIOCHEM. SOC. TRANS. 42(2):593-0 (Apr. 2014).

Another embodiment provides the use of a compound of the present invention, or a pharmaceutically acceptable salt thereof, in the manufacture of a medicament for the treatment of a condition selected from the group consisting of T2DM, obesity, NAFLD, NASH, dyslipidemia and metabolic syndrome. In an embodiment, the medicament is for the treatment of T2DM. In an embodiment, the medicament is for the treatment of obesity. In an embodiment, the medicament is for the treatment of NAFLD. In an embodiment, the medicament is for the treatment of NASH.

Another embodiment provides a pharmaceutical composition comprising a compound of Formula I, or a pharmaceutically acceptable salt thereof, and at least one selected from the group consisting of a carrier, diluent, and excipient.

In an embodiment is a pharmaceutical composition comprising a compound of Formula I, or a pharmaceutically acceptable salt thereof, at least one permeation enhancer and at least one protease inhibitor. In an embodiment, is a pharmaceutical composition comprising a compound of Formula I, or a pharmaceutically acceptable salt thereof, at least one permeation enhancer, and at least one selected from the group consisting of carrier, diluent, and excipient.

In an embodiment is a pharmaceutical composition comprising a compound of Formula I, or a pharmaceutically acceptable salt thereof, a permeation enhancer, a protease inhibitor, and at least one selected from the group consisting of carrier, diluent, and excipient. In an embodiment is a pharmaceutical composition comprising a compound of Formula I, or a pharmaceutically acceptable salt thereof, and a permeation enhancer. In an embodiment is a pharmaceutical composition comprising a compound of the present invention, or a pharmaceutically acceptable salt thereof, and a permeation enhancer. In an embodiment the permeation enhancer is selected from the group consisting of sodium decanoate ("C10"), sodium taurodeoxycholate ("NaTDC"), lauroyl carnitine ("LC"), dodecyl maltoside ("C12-maltoside"), dodecyl phosphatidylcholine ("DPC"), sodium N-[8-(2-hydroxybenzoyl) amino] caprylate ("SNAC") and a Rhamnolipid. In an embodiment the permeation enhancer is selected from the group consisting of C10 and LC. In an embodiment a protease inhibitor is selected from the group consisting of soybean trypsin inhibitor ("SBTI"), soybean trypsin-chymotrypsin inhibitor ("SBTCI"), ecotin, sunflower trypsin inhibitor ("SFTI"), leupeptin, citric acid, ethylenediaminetetraacetic acid ("EDTA"), sodium glycocholate and 4-(2-aminoethyl) benzenesulfonyl fluoride hydrochloride ("AEBSF"). In an embodiment, a protease inhibitor is selected from the group consisting of SBTI, SBTCI, and SFTI. In an embodiment, a protease inhibitor is SBTI.

As used herein, the term "treating" or "to treat" includes restraining, slowing, stopping, or reversing the progression or severity of a symptom, condition, or disorder.

Certain compounds of the present invention are generally effective over a wide dosage range. For example, dosages for once weekly parenteral dosing may fall within the range of 0.05 mg to about 30 mg per person per week.

The compounds of the present invention include novel amino acid sequences having affinity for the respective GLP-1 and GIP receptors, with desired potency at each of these receptors. GLP-1 is a 36 amino acid peptide, the major biologically active fragment of which is produced as a 30-amino acid, C-terminal amidated peptide (GLP-1₇₋₃₆) (SEQ ID NO:2).

GIP is a 42 amino acid peptide (SEQ ID NO:1), which, like GLP-1, is also known as an incretin, and plays a physiological role in glucose homeostasis by stimulating insulin secretion from pancreatic beta cells in the presence of glucose.

The compounds provide desired potency at each of the GIP and GLP-1 receptors. In an embodiment, compounds are suitable for oral administration. In an embodiment, compounds have desirable GIP and GLP receptor extended time action. In an embodiment, compounds have desirable GIP and GLP receptor activity wherein the GIP agonist potency is from 2.5 to 5 times the GLP1 receptor potency as measured by the casein cAMP assay described herein below, wherein the potency is normalized against native GIP and GLP on the day the assay is run. In an embodiment, compounds have desirable GIP and GLP receptor activity wherein the GIP agonist potency is from 2.5 to 10 times the GLP1 receptor potency as measured by the casein cAMP assay, wherein the potency is normalized against native GIP and GLP on the day the assay is run.

As used herein the term "amino acid" means both naturally occurring amino acids and unnatural amino acids. The amino acids are typically depicted using standard one letter codes (e.g., L = leucine), as well as alpha-methyl substituted residues of natural amino acids (e.g., α-methyl leucine, or αMeL and α-methyl lysine, or αMeK) and certain other unnatural amino acids, such as alpha amino isobutyric acid, or "Aib," "4Pal," "Orn," and the like. The structures of these amino acids appear below: 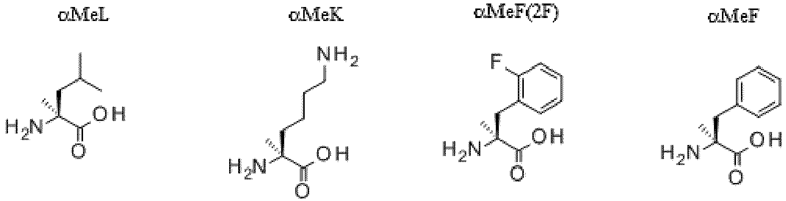 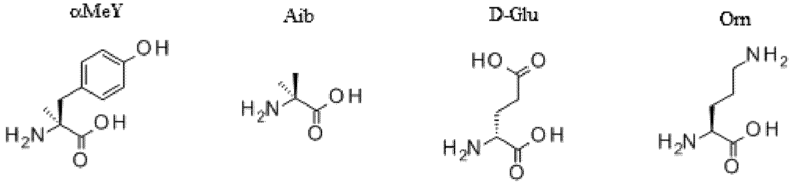 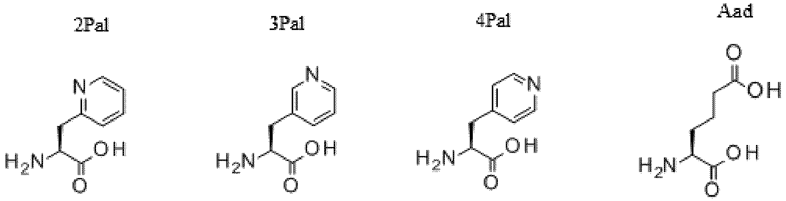 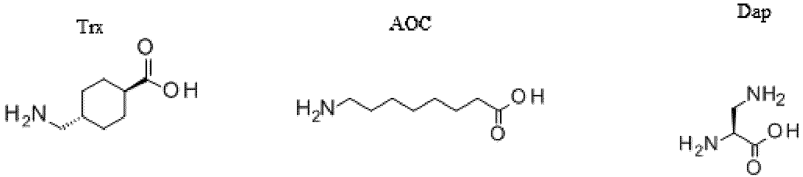 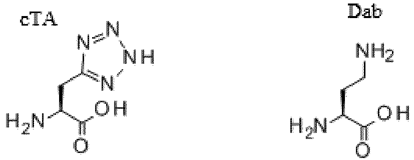 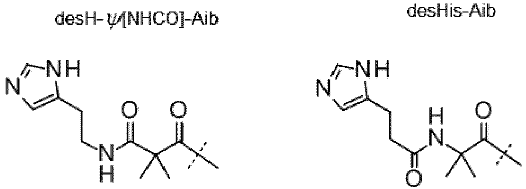 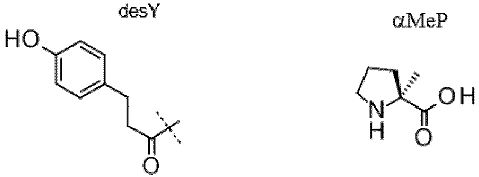 As used herein "Orn" means ornithine. As used herein "4Pal" means 3-(4-Pyridyl)-L-alanine. As used herein "αMeF(2F)" means alpha-methyl 2-F-phenylalanine. As used herein "αMeY," "αMeK," and "αMeL" mean alpha methyl tyrosine, alpha methyl lysine, and alpha methyl leucine, respectively. As used herein, "e" and "D-Glu" mean D-glutamic acid. As used herein "D-His" and "h" each mean D-histidine. As used herein "D-Tyr"and "y" each means D-tyrosine. As used herein "D-Ser" and "s" means means D-serine. As used herein "D-Ala" and "a" each means D-alanine. As used herein, "αMeF(2F)" means alpha-methyl-F(2F) and alpha-methyl-Phe(2F). As used herein, "αMeF", means alpha-methyl-F and alpha-methyl-Phe. As used herein, "αMeY", means alpha-methyl-Tyr. As used herein "αMeK", means alpha-methyl-Lys. As used herein, "αMeL", means alpha-methyl-Leu. As used herein, "αMeS", means alpha-methyl-serine and alpha-methyl-Ser. As used herein "αMeP", means alpha-methyl-proline and alpha-methyl-Pro. As used herein, "desH", means desHis. As used herein, "desY", means desTyr.

When X₁ is DesH and X₂ is Aib, and the DesH and Aib can combine to form a group as illustrated above, DesH-*ψ*[NHCO]-Aib.

When used herein, the term "amino acid conjugated to a C₁₆-C₂₂ fatty acid" refers to any natural or unnatural amino acid with a functional group that has been chemically modified to conjugate to a fatty acid by way of a covalent bond to the fatty acid or, preferably, by way of a linker. Examples of such functional groups include amino, carboxyl, chloro, bromo, iodo, azido, alkynyl, alkenyl, and thiol groups. Examples of natural amino acids which include such functional groups include K (amino), C (thiol), E (carboxyl) and D (carboxyl). In an embodiment the conjugated amino acid is K.

As noted above, in an embodiment of a compound of Formula I, II, III, IV, and V are compounds of the present invention wherein a fatty acid moiety is conjugated via a linker or a direct bond. In an embodiment, compounds of the present invention include a fatty acid moiety conjugated, preferably via a linker, to a K at position 14 or 17. In an embodiment, the conjugation is an acylation. In an embodiment, the conjugation is to the epsilon-amino group of the K side-chain. In an embodiment of the compounds of the present invention include a fatty acid moiety conjugated, via a linker, to a K at position 17.

In an embodiment, compounds of the present invention include a fatty acid moiety conjugated directly, without a linker, to a natural or unnatural amino acid with a functional group available for conjugation. In certain preferred embodiments the conjugated amino acid is selected from the group consisting of K, C, E and D. In particularly preferred embodiments the conjugated amino acid is K. In such embodiments, the conjugation is to the epsilon-amino group of the K side-chain.

In an embodiment, the linker comprises one to four amino acids, an amino polyethylene glycol carboxylate, or mixtures thereof. In an embodiment, the amino polyethylene glycol carboxylate has the following formula:
H-{NH-CH2-CH2-[O-CH2-CH2]ₚ-O-(CH2)_{z}-CO}ᵣ-OH, wherein p is any integer from 1 to 12, z is any integer from 1 to 20, and r is 1 or 2.

In an embodiment is a compound of Formula I which comprises an amino acid conjugated to a fatty acid via a linker, wherein the linker is one to two amino acids selected from the group consisting of Glu and γ-Glu. In an embodiment the linker is one to two (2-[2-(2-Amino-ethoxy)-ethoxy]-acetyl) moieties. The compounds of the present invention utilize a C₁₆-C₂₂ fatty acid chemically conjugated to the functional group of an amino acid either by a direct bond or by a linker. In an embodiment, the fatty acid moiety is conjugated to a lysine at position 17 via a linker between the lysine and the fatty acid. In an embodiment, the fatty acid moiety is conjugated to a lysine at position 20 via a linker between the lysine and fatty acid. In an embodiment, the fatty acid chain is any single chain C₁₆-C₂₂ fatty acid.

In an embodiment is a compound of Formula I, or a pharmaceutically acceptable salt thereof, wherein the fatty acid is conjugated with a linker, and the linker comprises one or more (2-[2-(2-Amino-ethoxy)-ethoxy]-acetyl) moieties, in combination with zero or one to four amino acids. In an embodiment, the linker may comprise one to four Glu or γ-Glu amino acid residues. In an embodiment, the linker may comprise 1 or 2 Glu or γ-Glu amino acid residues. In an embodiment is a compound of Formula I, or a pharmaceutically acceptable salt thereof, comprises a fatty acid conjugated via a linker wherein, the linker comprises either 1 or 2 γ-Glu amino acid residues. In an embodiment is a compound of Formula I, or a pharmaceutically acceptable salt thereof, comprises a fatty acid conjugated via a linker wherein the linker may comprise one to four amino acid residues (such as, for example Glu and γ-Glu amino acids) used in combination with up to 36 (2-[2-(2-Amino-ethoxy)-ethoxy]-acetyl) moieties. Specifically, in an embodiment is a Formula I compound, or a pharmaceutically acceptable salt thereof, which comprises a fatty acid conjugated via a linker wherein, the linker constitutes combinations of one to four Glu and γ-Glu amino acids and one to four (2-[2-(2-Amino-ethoxy)-ethoxy]-acetyl) moieties. In an embodiment is a Formula I compound, or a pharmaceutically acceptable salt thereof, which comprises a fatty acid conjugated via a linker wherein the linker is comprised of combinations of one or two γ-Glu amino acids and one or two (2-[2-(2-Amino-ethoxy)-ethoxy]-acetyl) moieties. In an embodiment is a Formula I compound, or a pharmaceutically acceptable salt thereof, which comprises a fatty acid conjugated via a linker wherein the linker and fatty acid components have the following formula:
(2-[2-(2-Amino-ethoxy)-ethoxy]-acetyl)ₐ-(γ-Glu)_{b}-CO-(CH₂)_{q}-CO₂H, wherein a is 1 or 2, b is 1 or 2 and q is 16 or 18. In an embodiment, a is 2, b is 1 and q is 18; and the structure is:

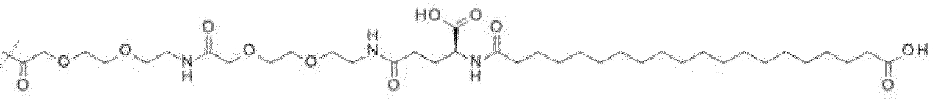

In an embodiment, a is 1, b is 2 and q is 18; and the structure is:

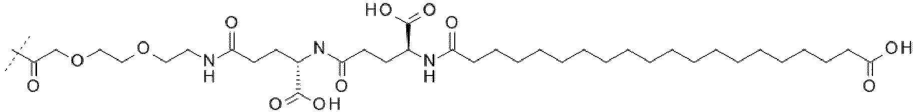

In an embodiment a is 1, b is 1, and q is 18; and the structure is:

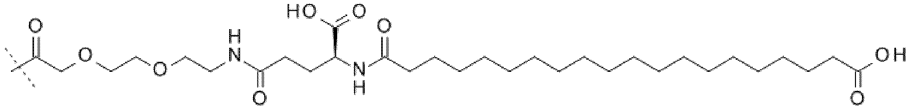

The term "C₁₆-C₂₂ fatty acid" as used herein means a carboxylic acid with between 16 and 22 carbon atoms. In an embodiment, the C₁₆-C₂₂ fatty acid suitable for use herein can be a saturated diacid. In an embodiment, the fatty acid is C₂₀-C₂₂. In an embodiment q is selected from the group consisting of 14, 16, 18, and 20. In an embodiment q is selected from 18 and 20. In an embodiment q is 18. In an embodiment q is 20.

In an embodiment, specific saturated C₁₆-C₂₂ fatty acids that are suitable for the compounds and uses thereof disclosed herein include, but are not limited to, hexadecanedioic acid (C₁₆ diacid), heptadecanedioic acid (C₁₇ diacid), octadecanedioic acid (C₁₈ diacid), nonadecanedioic acid (C₁₉ diacid), eicosanedioic acid (C₂₀ diacid), heneicosanedioic acid (C₂₁ diacid), docosanedioic acid (C₂₂ diacid), including branched and substituted derivatives thereof.

In an embodiment, the C₁₆-C₂₂ fatty acid is selected from the group consisting of a saturated C₁₈ diacid, a saturated C₁₉ diacid, a saturated C₂₀ diacid, and branched and substituted derivatives thereof. In an embodiment, the C₁₆-C₂₂ fatty acid is selected from the group consisting of stearic acid, arachadic acid and eicosanedioic acid. In an embodiment, the C₁₆-C₂₂ fatty acid is arachadic acid.

As shown in the chemical structures of Examples 1-5 below, in an embodiment the linker-fatty acid moieties described above link to the epsilon-amino group of the lysine side-chain.

In an embodiment, is a compound of Formula I, or a pharmaceutically acceptable salt thereof, wherein none of X_{30,} X_{31,} X_{32,} X_{33,} X_{34,} X_{35,} X_{36,} X_{37,} X_{38,} X_{39,} and X₄₀ is C or is a substituent that contains a fatty acid. In an embodiment, is a compound of Formula I, or a pharmaceutically acceptable salt thereof, wherein none of X₁₀, X₁₂, X₁₃, X₁₄, X₁₆, X₁₇, X₁₉, X₂₀, X₂₁, X₂₃, X₂₄, X₂₆, X₂₇, X₂₈, X₂₉, X_{30,} X_{31,} X_{32,} X_{33,} X_{34,} X_{35,} X_{36,} X_{37,} X_{38,} X_{39,} and X₄₀ is a substituent that contains a fatty acid; and none of X_{30,} X_{34,} X_{39,} and X₄₀ is C. In an embodiment is a compound of Formula I, or a pharmaceutically acceptable salt thereof, wherein none of X₁₀, X₁₂, X₁₃, X₁₄, X₁₆, X₁₇, X₁₉, X₂₀, X₂₁, X₂₃, X₂₄, X₂₆, X₂₇, X₂₈, X₂₉, X₃₀, X_{31,} X_{32,} X_{33,} X_{34,} X_{35,} X_{36,} X_{37,} X_{38,} X_{39,} and X₄₀ is a substituent that contains a fatty acid.

As used herein "time-extension technology" means a peptide time-extension technology for example, recombinant human serum albumin ("rHSA"), peptide conjugation to a pharmaceutically acceptable polymer, such as polymeric sequence of amino acids ("XTEN"), unsulfated heparin-like carbohydrate polymer ("HEP"), hydroxyl ethyl starch ("HES"), llama heavy-chain antibody fragments ("VHH"), pegylation, Fc conjugation, bovine serum albumin ("BSA") (Sleep, D. Epert Opin Drug Del (2015) 12, 793-812; Podust VN et.al. J Control. Release, 2015; ePUB; Hey, T. et. al. in: R. Kontermann (Ed.), Therapeutic Proteins: Strategies to Modulate their Plasma Half-Lives, Wiley-VCH Verlag Gmbh & Co. KGaA, Weinheim, Germany, 2012, pp117-140; DeAngelis, PL, Drug Dev Delivery (2013) January, 12/31/2012. In an embodiment time-extension technology is applied using a linker group. In an embodiment, the time-extension technology is applied using 0, 1, 2, or 3 amino acids as linker.

In an embodiment is a compound of Formula I, or a pharmaceutically acceptable salt thereof, without a fatty acid (*i.e*., a compound where none of X₁₀, X₁₂, X₁₃, X₁₄, X₁₆, X₁₇, X₁₉, X₂₀, X₂₁, X₂₃, X₂₄, X₂₆, X₂₇, X₂₈, X₂₉, X_{30,} X_{31,} X_{32,} X_{33,} X_{34,} X_{35,} X_{36,} X_{37,} X_{38,} X_{39,} and X₄₀ is a substituent that contains a fatty acid) or time-extension technology may be administered to a patient in need thereof via transdermal or infusion methods of administration. Further, a compound of Formula I, or a pharmaceutically acceptable salt thereof, without a fatty acid may be further modified using a peptide time-extension technology for example, recombinant human serum albumin ("rHSA"), peptide conjugation to a pharmaceutically acceptable polymer, such as polymeric sequence of amino acids ("XTEN"), unsulfated heparin-like carbohydrate polymer ("HEP"), and hydroxyl ethyl starch ("HES"). In an embodiment, a time-extension technology is applied using a cysteine amino acid in a Formula I compound, or a pharmaceutically acceptable salt thereof, without a fatty acid, using procedures known to the skilled artisan. In an embodiment, a time-extension technology is applied to one amino acid in a Formula I compound, or a pharmaceutically acceptable salt thereof, without a fatty acid. In an embodiment, wherein a time-extension technology is applied to a Formula I compound, or a pharmaceutically acceptable salt thereof, without a fatty acid, X₁₇ is selected from the group consisting of I, K and Q. In an embodiment wherein a time-extension technology is applied to a Formula I compound, or a pharmaceutically acceptable salt thereof, without a fatty acid, X₃₀ is C. In an embodiment wherein a time-extension technology is applied to a Formula I compound, or a pharmaceutically acceptable salt thereof, without a fatty acid, X₃₄ is C. In an embodiment wherein a time-extension technology is applied to a Formula I compound, or a pharmaceutically acceptable salt thereof, without a fatty acid, X₃₉ is C. In an embodiment wherein a time-extension technology is applied to a Formula I compound, or a pharmaceutically acceptable salt thereof, without a fatty acid, X₄₀ is C.

When used herein in reference to one or more of the GIP or GLP-1 receptors, the terms "activity," "activate[s]" "activat[ing]" and the like refers to the capacity of a compound, or a pharmaceutically acceptable salt thereof, to bind to and induce a response at the receptor(s), as measured using assays known in the art, such as the in vitro assays described below.

The affinity of compounds, or a pharmaceutically acceptable salt thereof, of the present invention for each of the GIP and GLP-1 receptors may be measured using techniques known for measuring receptor binding levels in the art, including, for example those described in the examples below, and is commonly expressed as a Ki value. The activity of the compounds of the present invention at each of the receptors may also be measured using techniques known in the art, including for example the in vitro activity assays described below, and is commonly expressed as an EC₅₀ value, which is the concentration of compound causing half-maximal simulation in a dose response curve.

In an embodiment, a pharmaceutical composition of a compound of Formula I is suitable for administration by a parenteral route (*e.g.*, subcutaneous, intravenous, intraperitoneal, intramuscular, or transdermal). In an embodiment, a pharmaceutical composition of a compound of Formula I is suitable for oral administration (e.g., tablet, capsule). Some pharmaceutical compositions and processes for preparing same are well known in the art. (*See, e.g.,* Remington: The Science and Practice of Pharmacy (D.B. Troy, Editor, 21st Edition, Lippincott, Williams & Wilkins, 2006). Physiochemical properties of a peptide in addition to anatomical and physiological features of the gastrointestinal tract may provide challenges to efficient oral delivery of a peptide. In an embodiment a pharmaceutical composition for oral administration comprises of a compound of this invention, and a permeation enhancer. In an embodiment, a pharmaceutical composition for oral administration comprises a compound of Formula I or a pharmaceutically acceptable salt thereof, a permeation enharncer, and a protease inhibitor. In an embodiment, a pharmaceutical composition for oral administration comprises a compound of Formula I, or a pharmaceutically acceptable salt thereof, and a permeation enharncer,

Monolithic and multi-particulate dosage forms for compounds of the present invention are contemplated. In an embodiment, a compound of Formula I is provided as a monolithic composition. A monolithic composition is intended for release of all components in a single location. A multi-particuate composition is intended to achieve fast transit from the stomach to the intestine and allow for distribution of composition components over large surface of small intestine. Concurrent release of a compound and functional excipients is desired for monolithic and multi-particulate dosage compositions. In an embodiment a monolithic composition of a compound of Formula I, or a pharmaceutically acceptable salt thereof, is formulated as an enteric capsule, enteric coated capsule or an enteric coated tablet. Such multi-particulate composition may be formulated as an enteric coated minitablets, or enteric coated granules where the coating is generally intact in the stomach at low pH and dissolves at the higher pH of the intestine. Two types of coated minitablets or coated granules may be formulated for either delivery to proximal small intestine by dissolution above pH 5.5 or to distal small intestine by dissolution above pH 7-7.2. A coating system for distal small intestinal release can also be applied to monolithic capsules or tablets if distal small intestinal delivery is desired. Minitablets may be filled into a standard uncoated capsule.

As used herein the term "permeation enhancer" means permeation enhancer that enhances oral absorption of a compound of this invention. As used herein, permeation enhancer means permeation enhancers, such as sodium decanoate, sodium taurodeoxycholate, lauroyl carnitine, dodecyl maltoside, dodecyl phosphatidylcholine, SNAC, a Rhamnolipid, and permeation enhancers reported in the literature, such as for example, Permeant inhibitor of phosphatase, PIP-250 and PIP-640. *See,* Pharmaceutics. 2019 Jan; 11(1): 41, (See Biomaterials. 2012; 33: 3464-3474), ZOT (zonula occludens toxin), ΔG (fragment of ZOT) (See Int. J. Pharm. 2009; 365, 121-130). In an embodiment, a permeation enhancer is selected from the group consisting of sodium decanoate, sodium taurodeoxycholate, and lauroyl carnitine. In an embodiment, a permeation enhancer is selected from the group consisting of C10, LC, and NaTDC. In an embodiment a permeation enhancer is C10.

As used herein the term "protease inhibitor" means a protease inhibitor that may be selected from the group consisting of protein based, peptide based, and small molecule based. Protease inhibitors are well known and may include, for example, soybean trypsin inhibitor ("SBTI"), soybean trypsin-chymotrypsin inhibitor ("SBTCI"), ecotin, sunflower trypsin inhibitor ("SFTI"), leupeptin, citric acid, ethylenediaminetetraacetic acid ("EDTA"), sodium glycocholate and 4-(2-aminoethyl) benzenesulfonyl fluoride hydrochloride ("AEBSF"). In an embodiment a protease inhibitor is selected from the group consisting of SBTI, SBTCI and SFTI. In an embodiment, a protease inhibitor is SBTI.

In an embodiment is a compound of Formula I, or a pharmaceutically acceptable salt thereof, wherein the compound is a potent GIPR/GLP-1R dual agonist that is a partial agonist on the GLP-1R as demonstrated by a Cell Membrane Guanosine 5'-(gamma-thio) Triphosphate-[³⁵S] (GTPγS) Binding Assay, and a partial agonist on the GLP-1R as demonstarted by a β-arrestin-2 recruitment assay. In an embodiment is a compound of Formula I, or pharmaceutically acceptable salt thereof, wherein the compound stimulates GLP-1R induced activation of Gαₛ in the GLP-1R HEK293 Cell Membrane Guanosine 5'-(gamma-thio) Triphosphate-[³⁵S] (GTPγS) Binding Assay. In an embodiment, is a compound showing partial agonism of 75% or less in the GLP-1R HEK293 Cell Membrane Guanosine 5'-(gamma-thio) Triphosphate-[³⁵S] (GTPγS) Binding Assay, and 35% or less in the GLP-CHO Cell β-Arrestin.Recruitment Assay.

In an embodiment is a method for treating diabetes comprising administering an effective amount of a compound showing partial agonism of 75% or less in the GLP-1R HEK293 Cell Membrane Guanosine 5'-(gamma-thio) Triphosphate-[³⁵S] (GTPγS) Binding Assay, and an effective amount of a compound that is a GIP agonist. In an embodiment, the compound showing partial agonism in the GLP-1R HEK293 Cell Membrane Guanosine 5'-(gamma-thio) Triphosphate-[³⁵S] (GTPγS) Binding Assay is co-administered with a compound having GIP agonist activity. In an embodiment, the compound showing partial agonism in the GLP-1R HEK293 Cell Membrane Guanosine 5'-(gamma-thio) Triphosphate-[³⁵S] (GTPγS) Binding Assay is administered as an active agent within one week before or after a compound having GIP agonist activity. In an embodiment, a method for treating diabetes comprises administering an effective amount of a compound showing 35% or less in the GLP-CHO Cell β-Arrestin.Recruitment Assay and administering an effective amount of a compound showing partial agonism of 75% or less in the GLP-1R HEK293 Cell Membrane Guanosine 5'-(gamma-thio) Triphosphate-[³⁵S] (GTPγS) Binding Assay.

Compounds of the present invention may react with any of a number of inorganic and organic acids/bases to form pharmaceutically acceptable acid/base addition salts. Pharmaceutically acceptable salts and common methodology for preparing them are well known in the art. (*See, e.g.*, P. Stahl, et al. Handbook of Pharmaceutical Salts: Properties, Selection and Use, 2nd Revised Edition (Wiley-VCH, 2011)). Pharmaceutically acceptable salts of the present invention include, but are not limited to, sodium, trifluoroacetate, hydrochloride, ammonium, and acetate salts. In an embodiment, a pharmaceutically acceptable salt of is selected from the group consisting of sodium, hydrochloride, and acetate salts.

The present invention also encompasses novel intermediates and processes useful for the synthesis of compounds of the present invention, or a pharmaceutically acceptable salt thereof. The intermediates and compounds of the present invention may be prepared by a variety of procedures known in the art. In particular, the Examples below describe a process using chemical synthesis. The specific synthetic steps for each of the routes described may be combined in different ways to prepare compounds of the present invention. The reagents and starting materials are readily available to one of ordinary skill in the art.

When used herein, the term "effective amount" refers to the amount or dose of a compound of the present invention, or a pharmaceutically acceptable salt thereof, which, upon single or multiple dose administration to the patient, provides the desired effect in the patient under diagnosis or treatment. An effective amount can be determined by a person of skill in the art using known techniques and by observing results obtained under analogous circumstances. In determining the effective amount for a subject, a number of factors are considered, including, but not limited to: the species of mammal; its size, age, and general health; the specific disease or disorder involved; the degree of or involvement or the severity of the disease or disorder; the response of the individual patient; the particular compound administered; the mode of administration; the bioavailability characteristics of the preparation administered; the dose regimen selected; the use of concomitant medication; and other relevant circumstances.

When used herein, the term "subject in need thereof" refers to a mammal, preferably a human, with a disease or condition requiring treatment or therapy, including for example those listed in the preceding paragraphs. As used herein "EDTA" means ethylenediaminetetraacetic acid. As used herein "DMSO" means dimethyl sulfoxide. As used herein "CPM" means counts per minute. As used herein "IBMX" means 3-isobutyl-1-methylxanthine. As used herein "LC/MS" means liquid chromatography/mass spectrometry. As used herein "HTRF" means homogeneous time-resolved fluorescence. As used herein "BSA" mean bovine serum albumin.

The invention is further illustrated by the following examples, which are not to be construed as limiting.

### PEPTIDE SYNTHESIS

### Example 1

### Y-Aib-EGT-αMeF(2F)-TSDYSI-αMeL-LDEK((2-[2-(2-Amino-ethoxy)-ethoxy]-acetyl)₂-(γ-Glu)-CO-(CH₂)₁₈-CO₂H)AQ-Aib-EFI-(D-Glu)-YLIEGGPSSGAPPPS-NH₂ (SEQ ID NO:10).

The structure of SEQ ID NO:10 is depicted below using the standard single letter amino acid codes with the exception of residues Aib2, αMeF(2F)6, αMeL13, K17, Aib20, D-Glu24, and Ser39 where the structures of these amino acid residues have been expanded:

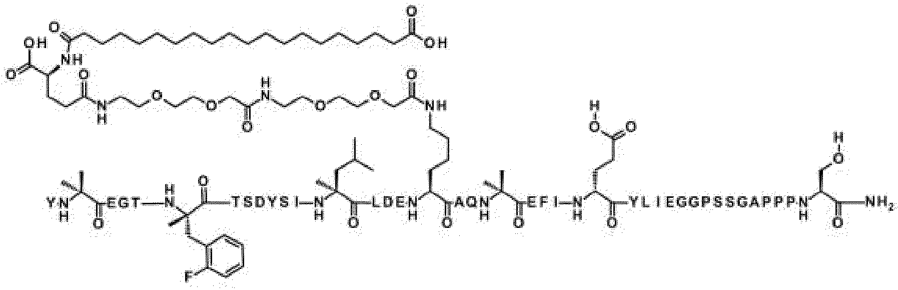

The peptide backbone of Example 1 is synthesized using Fluorenylmethyloxycarbonyl (Fmoc)/tert-Butyl (t-Bu) chemistry on a Symphony X peptide synthesizer (Gyros Protein Technologies. Tucson, AZ).

The resin consists of 1% DVB cross-linked polystyrene (Fmoc-Rink-MBHA Low Loading resin, 100-200 mesh, EMD Millipore) at a substitution of 0.3-0.4 meq/g. Standard side-chain protecting groups were used. Fmoc-Lys(Mtt)-OH is used for the lysine at position 17 and Boc-Tyr(tBu)-OH) was used for the tyrosine at position 1. Fmoc groups are removed prior to each coupling step (2 x 7 minutes) using 20% piperidine in DMF. All standard amino acid couplings are performed for 1 hour to a primary amine and 3 hour to a secondary amine, using an equal molar ratio of Fmoc amino acid (0.3 mM), diisopropylcarbodiimide (0.9 mM) and Oxyma (0.9 mM), at a 9-fold molar excess over the theoretical peptide loading. Exceptions are couplings to C*α*-methylated amino acids, which are coupled for 3 hours. After completion of the synthesis of the peptide backbone, the resin is thoroughly washed with DCM for 6 times to remove residual DMF. The Mtt protecting group on the lysine at position 17 is selectively removed from the peptide resin using two treatments of 30% hexafluoroisopropanol (Oakwood Chemicals) in DCM (2 x 40-minute treatment).

Subsequent attachment of the fatty acid-linker moiety is accomplished by coupling of 2-[2-(2-Fmoc-amino-ethoxy)-ethoxy]-acetic acid (Fmoc-AEEA-OH, ChemPep, Inc.), Fmoc-glutamic acid *α*-t-butyl ester (Fmoc-Glu-OtBu, Ark Pharm, Inc.), mono-OtBu-eicosanedioic acid (WuXi AppTec, Shanghai, China). 3-Fold excess of reagents (AA: PyAOP: DIPEA=1: 1 :1 mol/mol) are used for each coupling that is 1-hour long.

After the synthesis is complete, the peptide resin is washed with DCM, and then thoroughly air-dried. The dry resin is treated with 10 mL of cleavage cocktail (trifluoroacetic acid: water: triisopropylsilane, 95:2.5:2.5 v/v) for 2 hours at room temperature. The resin is filtered off, washed twice each with 2 mL of neat T^{'}FA, and the combined filtrates are treated with 5-fold excess volume of cold diethyl ether (-20°C) to precipitate the crude peptide. The peptide/ether suspension is then centrifuged at 3500 rpm for 2 min to form a solid pellet, the supernatant is decanted, and the solid pellet is triturated with ether two additional times and dried in vacuo. The crude peptide is solubilized in 20% acetonitrile/20%Acetic acid/60%water and purified by RP- HPLC on a Luna 5 *µ*m Phenyl-Hexyl preparative column (21 x 250 mm, Phenomenex) with linear gradients of 100% acetonitrile and 0.1% TFA/water buffer system (30-50% acetonitrile in 60 min). The purity of peptide is assessed using analytical RP-HPLC and pooling criteria is >95%. The main pool purity of compound 1 is found to be 98.0%. Subsequent lyophilization of the final main product pool yielded the lyophilized peptide TFA salt. The molecular weight is determined by LC- MS (obsd: M+3 =1657.2; Calc M+3 =1657.0).

### Example 2

### Y-Aib-EGT-αMeF(2F)-TSDYSI-αMeL-LD-Orn-K((2-[2-(2-Amino-ethoxy)-ethoxy]-acetyl)₂-(γ-Glu)-CO-(CH₂)₁₆-CO₂H)AQ-Aib-EFI-(D-Glu)-YLIEGGPSSGAPPPS-NH₂ (SEQ ID NO:11)

The structure of SEQ ID NO:11 is depicted below using the standard single letter amino acid codes with the exception of residues Aib2, αMeF(2F)6, αMeL13, Orn16, K17, Aib20 D-Glu24, and Ser39 where the structures of these amino acid residues have been expanded: 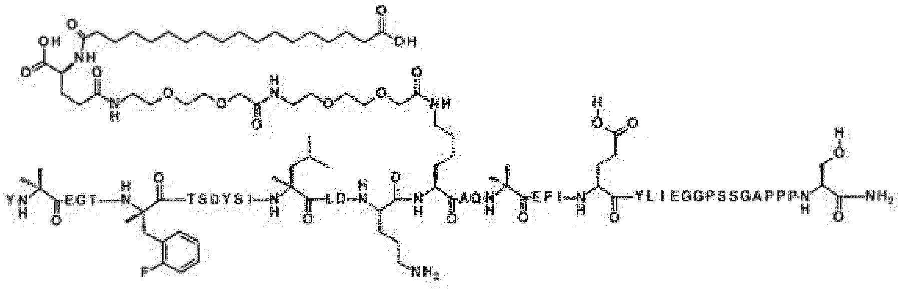 The compound according to SEQ ID NO:11 is prepared substantially as described by the procedures of Example 1. The molecular weight is determined by LC- MS (obsd: M+3 =1642.6; Calc M+3 =1642.8).

### Example 3

Example 3 is a compound represented by the following description:

Y-Aib-EGT-αMeF(2F)-TSDYSI-αMeL-LD-Orn-K((2-[2-(2-Amino-ethoxy)-ethoxy]-
acetyl)2-(γ-Glu)-CO-(CH2)18-CO2H)AQ-Aib-EFI-(D-Glu)-YLIEGGPSSGAPPPS-NH2
(SEQ ID NO:12)

The structure of SEQ ID NO:12 is depicted below using the standard single letter amino acid codes with the exception of residues Aib2, αMeF(2F)6, αMeL13, Orn16, K17, Aib20, D-Glu24, and Ser39, where the structures of these amino acid residues have been expanded: 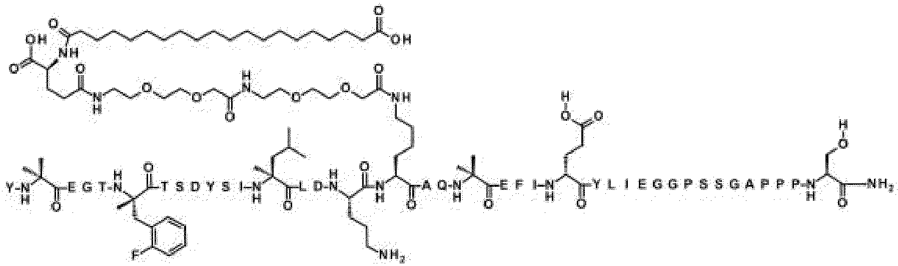 The compound according to SEQ ID NO:12 is prepared substantially as described by the procedures of Example 1. The molecular weight is determined by LC- MS (obsd: M+3 =1651.8; Calc M+3 =1652.2).

### Example 4

Y-Aib-EGT-αMeF(2F)-TSD-4Pal-SI-αMeL-LD-Orn-K((2-[2-(2-Amino-ethoxy)-ethoxy]-
acetyl)₂-(γ-Glu)-CO-(CH₂)₁₆-CO₂H)AQ-Aib-EFI-(D-Glu)-αMeY-LIEGGPSSGAPPPS-
NH₂ (SEQ ID NO:13)

The structure of SEQ ID NO:13 is depicted below using the standard single letter amino acid codes with the exception of residues Aib2, αMeF(2F)6, 4Pal10, αMeL13, Orn16, K17, Aib20, D-Glu24 αMeY25, and Ser39, where the structures of these amino acid residues have been expanded: 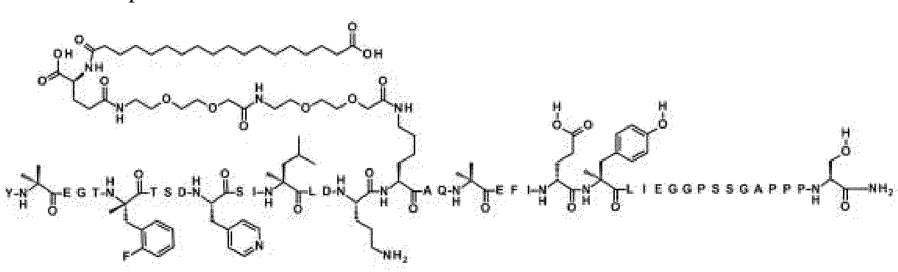 The compound according to SEQ ID NO:13 is prepared substantially as described by the procedures of Example 1. The molecular weight is determined by LC- MS (obsd: M+3 =1642.5; Calc M+3 =1642.1).

### Example 5

### Y-Aib-EGT-αMeF(2F)-TSDVSI-αMeL-LD-Orn-K((2-[2-(2-Amino-ethoxy)-ethoxy]-acetyl)₂-(γ-Glu)-CO-(CH₂)₁₆-CO₂H)AQ-Aib-EFI-(D-Glu)-αMeY-LIEGGPSSGAPPPS-NH₂ (SEQ ID NO:14)

Y-Aib-EGT-αMeF(2F)-TSDVSI-αMeL-LD-Orn-K((2-[2-(2-Amino-ethoxy)-ethoxy]-
acetyl)2-(γ-Glu)-CO-(CH2)16-CO2H)AQ-Aib-EFI-(D-Glu)-αMeY-LIEGGPSSGAPPPS-
NH2 (SEQ ID NO:14)

The structure of SEQ ID NO:14 is depicted below using the standard single letter amino acid codes with the exception of residues Aib2, αMeF(2F)6, αMeL13, Orn16, K17, Aib20, D-Glu24,αMeY25, and Ser39, where the structures of these amino acid residues have been expanded: 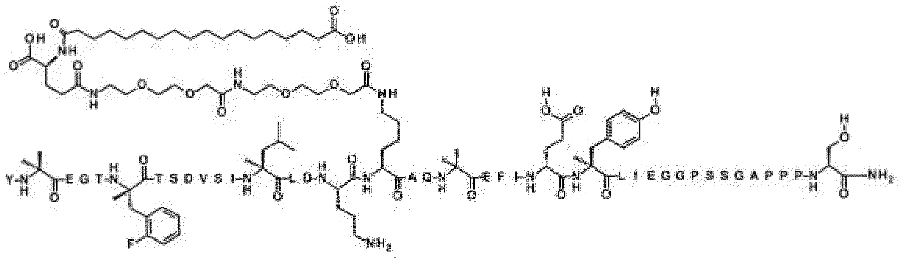 The compound according to SEQ ID NO:14 is prepared substantially as described by the procedures of Example 1. The molecular weight is determined by LC- MS (obsd: M+3 =1626.1; Calc M+3 =1626.1).

### Example 6 through Example 287

The compounds according to Examples 6 (SEQ ID NO:15) through Example 287 (SEQ ID NO:296) are prepared substantially as described by the procedures of Example 1.

| Example | Compound Name | SEQ ID NO | Calculated MW (average) | Found MW (average) |
|---|---|---|---|---|
| 6 | Y-Aib-EGTFTSDYSILLDKK((2-[2-(2- Amino-ethoxy)-ethoxy]-acetyl)2-(γ-Glu)-CO- (CH2)18-CO2H)AQ-Aib- AFIEYLLEGGPSSGAPPPS-NH2 | 15 | 4863.5 | 4862.1 |
| 7 | Y-Aib-EGTFTSDYSILLDSK((2-[2-(2- Amino-ethoxy)-ethoxy]-acetyl)2-(γ-Glu)-CO- (CH2)18-CO2H)AQ-Aib- AFIEYLLEGGPSSGAPPPS-NH2 | 16 | 4822.4 | 4821.3 |
| 8 | Y-Aib-EGTFTSDYSILLDKK((2-[2-(2- Amino-ethoxy)-ethoxy]-acetyl)2-(γ-Glu)-CO- (CH2)18-CO2H)AQ-Aib- AFIEYLIEGGPSSGAPPPS-NH2 | 17 | 4863.5 | 4863.2 |
| 9 | Y-Aib-EGTFTSDYSILLDSK((2-[2-(2- Amino-ethoxy)-ethoxy]-acetyl)2-(γ-Glu)-CO- (CH2)18-CO2H)AQ-Aib- AFIEYLIEGGPSSGAPPPS-NH2 | 18 | 4822.4 | 4820.7 |
| 10 | Y-Aib-EGTFTSDYSILLDSIAQ-Aib- AFIK((2-[2-(2-Amino-ethoxy)-ethoxy]- acetyl)2-(γ-Glu)-CO-(CH2)18-CO2H)YLLA- Aib-GPSSGAPPPS- NH2 | 19 | 4776.5 | 4775.4 |
| 11 | Y-Aib-EGTFTSDYSILLDSIAQ-Aib- AFIEYLLK((2-[2-(2-Amino-ethoxy)- ethoxy]-acetyl)2-(γ-Glu)-CO-(CH2)18-CO2H)- Aib-GPSSGAPPPS-NH2 | 20 | 4834.5 | 4834.8 |
| 12 | Y-Aib-EGTFTSDYSILLDKIAQK((2-[2-(2- Amino-ethoxy)-ethoxy]-acetyl)2-(γ-Glu)-CO- (CH2)18-CO2H)-AFIEYLIEGGPSSGAPPPS- NH2 | 21 | 4891.6 | 4890.0 |
| 13 | Y-Aib-EGTFTSDYSILLD-Aib-IAQK((2-[2- (2-Amino-ethoxy)-ethoxy]-acetyl)2-(γ-Glu)- CO-(CH2)18-CO2H)- AFIEYLIEGGPSSGAPPPS-NH2 | 22 | 4848.5 | 4846.8 |
| 14 | Y-Aib-EGTFTSDYSILLDKIAQK((2-[2-(2- Amino-ethoxy)-ethoxy]-acetyl)2-(γ-Glu)-CO- (CH2)18-CO2H)EFIQYLLE-Aib- GPSSGAPPPS-NH2 | 23 | 4976.7 | 4975.5 |
| 15 | H-Aib-EGTFTSDYSILLDKK((2-[2-(2- Amino-ethoxy)-ethoxy]-acetyl)2-(γ-Glu)-CO- (CH2)18-CO2H)AQ-Aib-AFIEYLLE-Aib- GPSSGAPPPS-NH2 | 24 | 4865.5 | 4863.9 |
| 16 | H-Aib-EGTFTSDYSILLDKK((2-[2-(2- Amino-ethoxy)-ethoxy]-acetyl)₂-(γ-Glu)-CO- (CH₂)₁₈-CO₂H)AQ-Aib-AFIEYLIE-Aib- GPSSGAPPPS-NH₂ | 25 | 4865.5 | 4863.9 |
| 17 | H-Aib-EGTFTSDYSILLDKIAQK((2-[2-(2- Amino-ethoxy)-ethoxy]-acetyl)₂-(γ-Glu)-CO- (CH₂)₁₈-CO₂H)AFIEYLLE-Aib-GPSSG-NH₂ | 26 | 4444.1 | 4442.7 |
| 18 | H-Aib-EGTFTSDYSI-αMeL-LDKK(Dab-(2- [2-(2-Amino-ethoxy)-ethoxy]-acetyl)-Dab-(2- [2-(2-Amino-ethoxy)-ethoxy]-acetyl)-CO- (CH2)18-CO2H)AQ-αMeK-AFIQYLLA-Aib- GPSSGAPKPS-NH2 | 27 | 4979.8 | 4978.8 |
| 19 | H-Aib-EGTFTSDYSI-αMeL-LDKK(Dab-(2- [2-(2-Amino-ethoxy)-ethoxy]-acetyl)-Dab-(2- [2-(2-Amino-ethoxy)-ethoxy]-acetyl)-CO- (CH2)18-CO2H)AQ-αMeK-AFIQYLLA-Aib- GPSSGAPPPS-NH2 | 28 | 4948.8 | 4947.2 |
| 20 | Y-Aib-EGTFTSDYSI-αMeL-LDKK((2-[2- (2-Amino-ethoxy)-ethoxy]-acetyl)2-(γ-Glu)- CO-(CH2)18-CO2H)AQ-Aib- AFIEYLIEGGPSSGAPPPS-NH2 | 29 | 4877.5 | 4875.9 |
| 21 | Y-Aib-EGTFTSDYSI-αMeL-LDEK((2-[2- (2-Amino-ethoxy)-ethoxy]-acetyl)2-(γ-Glu)- CO-(CH2)18-CO2H)AQ-αMeK- AFIEYLLEGGPSSGAPPPS-NH2 | 30 | 4935.6 | 4934.1 |
| 22 | Y-Aib-EGTFTSDYSI-αMeL-LDEK((2-[2- (2-Amino-ethoxy)-ethoxy]-acetyl)2-(γ-Glu)- CO-(CH2)18-CO2H)AQ-αMeK-AFIEYLLE- Aib-GPSSGAPPPS-NH2 | 31 | 4963.6 | 4962.0 |
| 23 | Y-Aib-EGTFTSDK((2-[2-(2-Amino-ethoxy)- ethoxy]-acetyl)2-(γ-Glu)-CO-(CH2)18- CO2H)SILLDKIAQ-Aib- AFIEYLIEGGPSSGAPPPS-NH2 | 32 | 4813.5 | 4812.9 |
| 24 | Y-Aib-EGTFTSDYK((2-[2-(2-Amino- ethoxy)-ethoxy]-acetyl)2-(γ-Glu)-CO-(CH2)18- CO2H)ILLDKIAQ-Aib- AFIEYLIEGGPSSGAPPPS-NH2 | 33 | 4889.6 | 4888.6 |
| 25 | Y-Aib-EGTFTSDYSK((2-[2-(2-Amino- ethoxy)-ethoxy]-acetyl)2-(γ-Glu)-CO-(CH2)18- CO2H)LLDKIAQ-Aib- AFIEYLIEGGPSSGAPPPS-NH2 | 34 | 4863.5 | 4862.5 |
| 26 | Y-Aib-EGTFTSDYSIK((2-[2-(2-Amino- ethoxy)-ethoxy]-acetyl)2-(γ-Glu)-CO-(CH2)18- CO2H)LDKIAQ-Aib- AFIEYLIEGGPSSGAPPPS-NH2 | 35 | 4863.5 | N/I |
| 27 | Y-Aib-EGTFTSDYSILK((2-[2-(2-Amino- ethoxy)-ethoxy]-acetyl)2-(γ-Glu)-CO-(CH2)18- CO2H)DKIAQ-Aib- AFIEYLIEGGPSSGAPPPS-NH2 | 36 | 4863.5 | N/I |
| 28 | Y-Aib-EGTFTSDYSILLK((2-[2-(2-Amino- ethoxy)-ethoxy]-acetyl)2-(γ-Glu)-CO-(CH2)18- CO2H)KIAQ-Aib- AFIEYLIEGGPSSGAPPPS-NH2 | 37 | 4861.6 | N/I |
| 29 | Y-Aib-EGTFTSDYSILLDK((2-[2-(2-Amino- ethoxy)-ethoxy]-acetyl)₂-(γ-Glu)-CO-(CH₂)₁₈- CO₂H)IAQ-Aib-AFIEYLIEGGPSSGAPPPS- NH₂ | 38 | 4848.5 | N/I |
| 30 | Y-Aib-EGTFTSDYSILLDKIK((2-[2-(2- Amino-ethoxy)-ethoxy]-acetyl)2-(γ-Glu)-CO- (CH2)18-CO2H)Q-Aib- AFIEYLIEGGPSSGAPPPS-NH2 | 39 | 4905.6 | N/I |
| 31 | Y-Aib-EGTFTSDYSILLDKIAK((2-[2-(2- Amino-ethoxy)-ethoxy]-acetyl)2-(γ-Glu)-CO- (CH2)18-CO2H)-Aib- AFIEYLIEGGPSSGAPPPS-NH2 | 40 | 4848.5 | N/I |
| 32 | Y-Aib-EGTFTSDYSILLDKIAQ-Aib-K((2- [2-(2-Amino-ethoxy)-ethoxy]-acetyl)2-(γ- Glu)-CO-(CH2)18- CO2H)FIEYLIEGGPSSGAPPPS-NH2 | 41 | 4905.6 | N/I |
| 33 | Y-Aib-EGTFTSDYSILLDKIAQ-Aib- AFK((2-[2-(2-Amino-ethoxy)-ethoxy]- acetyl)2-(γ-Glu)-CO-(CH2)18- CO2H)EYLIEGGPSSGAPPPS-NH2 | 42 | 4863.5 | N/I |
| 34 | Y-Aib-EGTFTSDYSILLDKIAQ-Aib- AFIK((2-[2-(2-Amino-ethoxy)-ethoxy]- acetyl)2-(γ-Glu)-CO-(CH2)18- CO2H)YLIEGGPSSGAPPPS-NH2 | 43 | 4847.6 | N/I |
| 35 | Y-Aib-EGTFTSDYSILLDKIAQ-Aib- AFIEYK((2-[2-(2-Amino-ethoxy)-ethoxy]- acetyl)2-(γ-Glu)-CO-(CH2)18- CO2H)IEGGPSSGAPPPS-NH2 | 44 | 4863.5 | N/I |
| 36 | Y-Aib-EGTFTSDYSILLDKIAQ-Aib- AFIEYLK((2-[2-(2-Amino-ethoxy)-ethoxy]- acetyl)2-(γ-Glu)-CO-(CH2)18- CO2H)EGGPSSGAPPPS-NH2 | 45 | 4863.5 | N/I |
| 37 | Y-Aib-EGTFTSDYSILLDKIAQ-Aib- AFIEYLIK((2-[2-(2-Amino-ethoxy)-ethoxy]- acetyl)2-(γ-Glu)-CO-(CH2)18- CO2H)GGPSSGAPPPS-NH2 | 46 | 4847.6 | N/I |
| 38 | Y-Aib-EGTFTSDYSILLDKIAQ-Aib- AFIEYLIEK((2-[2-(2-Amino-ethoxy)- ethoxy]-acetyl)2-(γ-Glu)-CO-(CH2)18- CO2H)GPSSGAPPPS-NH2 | 47 | 4919.6 | N/I |
| 39 | Y-Aib-EGTFTSDYSILLDKIAQ-Aib- AFIEYLIEGK((2-[2-(2-Amino-ethoxy)- ethoxy]-acetyl)2-(γ-Glu)-CO-(CH2)18- CO2H)PSSGAPPPS-NH2 | 48 | 4919.6 | N/I |
| 40 | Y-Aib-EGTFTSDYSILLDKIAQ-Aib- AFIEYLIEGGK((2-[2-(2-Amino-ethoxy)- ethoxy]-acetyl)2-(γ-Glu)-CO-(CH2)18- CO2H)SSGAPPPS-NH2 | 49 | 4879.5 | N/I |
| 41 | Y-Aib-EGTFTSDYSILLDKIAQ-Aib- AFIEYLIEGGPK((2-[2-(2-Amino-ethoxy)- ethoxy]-acetyl)2-(γ-Glu)-CO-(CH2)18- CO2H)SGAPPPS-NH2 | 50 | 4889.6 | N/I |
| 42 | Y-Aib-EGTFTSDYSILLDKIAQ-Aib- AFIEYLIEGGPSK((2-[2-(2-Amino-ethoxy)- ethoxy]-acetyl)2-(γ-Glu)-CO-(CH2)18- CO2H)GAPPPS-NH2 | 51 | 4889.6 | N/I |
| 43 | Y-Aib-EGTFTSDYSILLDKIAQ-Aib- AFIEYLIEGGPSSK((2-[2-(2-Amino- ethoxy)-ethoxy]-acetyl)2-(γ-Glu)-CO-(CH2)18- CO2H)APPPS-NH2 | 52 | 4919.6 | N/I |
| 44 | Y-Aib-EGTFTSDYSILLDKIAQ-Aib- AFIEYLIEGGPSSGK((2-[2-(2-Amino- ethoxy)-ethoxy]-acetyl)2-(γ-Glu)-CO-(CH2)18- CO2H)PPPS-NH2 | 53 | 4905.6 | N/I |
| 45 | Y-Aib-EGTFTSDYSILLDKIAQ-Aib- AFIEYLIEGGPSSGAK((2-[2-(2-Amino- ethoxy)-ethoxy]-acetyl)2-(γ-Glu)-CO-(CH2)18- CO2H)PPS-NH2 | 54 | 4879.5 | N/I |
| 46 | Y-Aib-EGTFTSDYSILLDKIAQ-Aib- AFIEYLIEGGPSSGAPK((2-[2-(2-Amino- ethoxy)-ethoxy]-acetyl)2-(γ-Glu)-CO-(CH2)18- CO2H)PS-NH2 | 55 | 4879.5 | N/I |
| 47 | Y-Aib-EGTFTSDYSILLDKIAQ-Aib- AFIEYLIEGGPSSGAPPK((2-[2-(2-Amino- ethoxy)-ethoxy]-acetyl)2-(γ-Glu)-CO-(CH2)18- CO2H)S-NH2 | 56 | 4879.5 | N/I |
| 48 | Y-Aib-EGTFTSDYSILLDKIAQ-Aib- AFIEYLIEGGPSSGAPPPK((2-[2-(2-Amino- ethoxy)-ethoxy]-acetyl)2-(γ-Glu)-CO-(CH2)18- CO2H)-NH2 | 57 | 4889.6 | N/I |
| 49 | Y-Aib-EGTFTSDYSILLDKIAQ-Aib- AFIEYLIEGGPSSGAPPPSK((2-[2-(2- Amino-ethoxy)-ethoxy]-acetyl)2-(γ-Glu)-CO- (CH2)18-CO2H)-NH2 | 58 | 4976.7 | N/I |
| 50 | Y-Aib-EGTFTSDYSILLDKK((2-[2-(2- Amino-ethoxy)-ethoxy]-acetyl)2-(γ-Glu)-CO- (CH2)18-CO2H)AQ-Aib-AFIEYLIEGGPSSG- NH2 | 59 | 4414.0 | N/I |
| 51 | Y-Aib-EGTFTSDYSILLDKK((2-[2-(2- Amino-ethoxy)-ethoxy]-acetyl)2-(γ-Glu)-CO- (CH2)18-CO2H)AQ-Aib-AFIEYLIEGG-NH2 | 60 | 4085.7 | N/I |
| 52 | Y-Aib-EGTFTSDYSI-αMeL-LDSK((2-[2-(2- Amino-ethoxy)-ethoxy]-acetyl)2-(γ-Glu)-CO- (CH2)18-CO2H)AQ-Aib- AFIEYLIEGGPSSGAPPPS-NH2 | 61 | 4836.4 | N/I |
| 53 | H-Aib-EGTFTSDYSI-αMeL-LDKK((2-[2- (2-Amino-ethoxy)-ethoxy]-acetyl)₂-(γ-Glu)- CO-(CH₂)₁₈-CO₂H)AQ-Aib- AFIEYLIEGGPSSGAPPPS-NH₂ | 62 | 4851.5 | N/I |
| 54 | H-Aib-EGTFTSDYSI-αMeL-LDKK((2-[2- (2-Amino-ethoxy)-ethoxy]-acetyl)2-(γ-Glu)- CO-(CH2)18- CO2H)AQHAFIEYLIEGGPSSGAPPPS-NH2 | 63 | 4903.5 | N/I |
| 55 | H-Aib-EGTFTSDYSI-αMeL-LDEK((2-[2- (2-Amino-ethoxy)-ethoxy]-acetyl)2-(γ-Glu)- CO-(CH2)18- CO2H)AQHAFIEYLIEGGPSSGAPPPS-NH2 | 64 | 4904.5 | N/I |
| 56 | Y-Aib-EGTFTSDYSI-αMeL-LDEK((2-[2- (2-Amino-ethoxy)-ethoxy]-acetyl)2-(γ-Glu)- CO-(CH2)18- CO2H)AQHAFIEYLIEGGPSSGAPPPS-NH2 | 65 | 4930.5 | N/I |
| 57 | Y-Aib-EGT-αMeF-TSDYSILLDKK((2-[2- (2-Amino-ethoxy)-ethoxy]-acetyl)2-(γ-Glu)- CO-(CH2)18-CO2H)AQ-Aib- AFIEYLIEGGPSSGAPPPS-NH2 | 66 | 4877.5 | N/I |
| 58 | Y-Aib-EGTFTSDYSSLLDKK((2-[2-(2- Amino-ethoxy)-ethoxy]-acetyl)2-(γ-Glu)-CO- (CH2)18-CO2H)AQ-Aib- AFIEYLIEGGPSSGAPPPS-NH2 | 67 | 4837.4 | N/I |
| 59 | Y-Aib-EGTFTSDYSI-αMeL-LDEK((2-[2- (2-Amino-ethoxy)-ethoxy]-acetyl)2-(γ-Glu)- CO-(CH2)18-CO2H)AQ-Aib- AFIEYLIEGGPSSGAPPPS-NH2 | 68 | 4878.5 | N/I |
| 60 | Y-Aib-EGTFTSDYSI-αMeL-LD-Aib-K((2- [2-(2-Amino-ethoxy)-ethoxy]-acetyl)2-(γ- Glu)-CO-(CH2)18-CO2H)AQ-Aib- AFIEYLIEGGPSSGAPPPS-NH2 | 69 | 4834.5 | N/I |
| 61 | Y-Aib-EGTFTSDYSI-αMeL-LDSK((2-[2-(2- Amino-ethoxy)-ethoxy]-acetyl)2-(γ-Glu)-CO- (CH2)18-CO2H)AQ-Aib- AFIEYLLEGGPSSGAPPPS-NH2 | 70 | 4836.4 | N/I |
| 62 | Y-Aib-EGTFTSDYSI-αMeL-LDKK((2-[2- (2-Amino-ethoxy)-ethoxy]-acetyl)2-(γ-Glu)- CO-(CH2)18-CO2H)AQ-Aib-AFIEYLIEGG- NH2 | 71 | 4099.7 | N/I |
| 63 | Y-Aib-EGTFTSDYSI-αMeL-LDEK((2-[2- (2-Amino-ethoxy)-ethoxy]-acetyl)2-(γ-Glu)- CO-(CH2)18-CO2H)AQ-Aib-AFIEYLIEGG- NH2 | 72 | 4100.6 | N/I |
| 64 | Y-Aib-EGTFTSDYSI-αMeL-LDSK((2-[2-(2- Amino-ethoxy)-ethoxy]-acetyl)2-(γ-Glu)-CO- (CH2)18-CO2H)AQ-Aib-AFIEYLIEGG-NH2 | 73 | 4058.6 | N/I |
| 65 | Y-Aib-EGTFTSDYSI-αMeL-LDTK((2-[2- (2-Amino-ethoxy)-ethoxy]-acetyl)2-(γ-Glu)- CO-(CH2)18-CO2H)AQ-Aib-AFIEYLIEGG- NH2 | 74 | 4072.6 | N/I |
| 66 | Y-Aib-EGTFTSDYSI-αMeL-LDEK((2-[2- (2-Amino-ethoxy)-ethoxy]-acetyl)2-(γ-Glu)- CO-(CH2)18-CO2H)AQ-Aib- AFIEYLLEGGPSSGAPPPS-NH2 | 75 | 4878.5 | N/I |
| 67 | Y-Aib-EGTFTSDYSILLDKK((2-[2-(2- Amino-ethoxy)-ethoxy]-acetyl)₂-(γ-Glu)-CO- (CH₂)₁₈-CO₂H)AQ-Aib-A-αMeF- IEYLIEGGPSSGAPPPS-NH₂ | 76 | 4877.5 | N/I |
| 68 | Y-Aib-EGTFTSDY-αMeS-ILLDKK((2-[2- (2-Amino-ethoxy)-ethoxy]-acetyl)2-(γ-Glu)- CO-(CH2)18-CO2H)AQ-Aib- AFIEYLIEGGPSSGAPPPS-NH2 | 77 | 4877.5 | N/I |
| 69 | Y-Aib-EGT-αMeF-TSDYSI-αMeL- LDKK((2-[2-(2-Amino-ethoxy)-ethoxy]- acetyl)2-(γ-Glu)-CO-(CH2)18-CO2H)AQ-Aib- AFIEYLIEGGPSSGAPPPS-NH2 | 78 | 4891.6 | N/I |
| 70 | Y-Aib-EGTFTSDK((2-[2-(2-Amino-ethoxy)- ethoxy]-acetyl)₂-(γ-Glu)-CO-(CH₂)₁₈- CO₂H)SILLDKIAQ-Aib-AFIEYLIEGG-NH₂ | 79 | 4035.7 | N/I |
| 71 | Y-Aib-EGTFTSDYSILK((2-[2-(2-Amino- ethoxy)-ethoxy]-acetyl)2-(γ-Glu)-CO-(CH2)18- CO2H)DKIAQ-Aib-AFIEYLIEGG-NH2 | 80 | 4085.7 | N/I |
| 72 | Y-Aib-EGTFTSDYSILLDKIAQ-Aib-K((2- [2-(2-Amino-ethoxy)-ethoxy]-acetyl)2-(γ- Glu)-CO-(CH2)18-CO2H)FIEYLIEGG-NH2 | 81 | 4127.8 | N/I |
| 73 | Y-Aib-EGTFTSDYSILLDKIAQ-Aib- AFIEYLIK((2-[2-(2-Amino-ethoxy)-ethoxy]- acetyl)2-(γ-Glu)-CO-(CH2)18-CO2H)GG-NH2 | 82 | 4069.7 | N/I |
| 74 | Y-Aib-EGTFTSDYSI-αMeL-LDKK((2-[2- (2-Amino-ethoxy)-ethoxy]-acetyl)2-(γ-Glu)- CO-(CH2)18-CO2H)AQ-Aib-A-αMeF- IEYLIEGGPSSGAPPPS-NH2 | 83 | 4891.6 | N/I |
| 75 | Y-Aib-EGTFTSDYSI-αMeL-LDKK((2-[2- (2-Amino-ethoxy)-ethoxy]-acetyl)2-(γ-Glu)- CO-(CH2)18-CO2H)AQ-Aib-AFIEY-αMeL- IEGGPSSGAPPPS-NH2 | 84 | 4891.6 | N/I |
| 76 | Y-Aib-EGT-aMeF-TSDYSI-αMeL- LDKK((2-[2-(2-Amino-ethoxy)-ethoxy]- acetyl)2-(γ-Glu)-CO-(CH2)18-CO2H)AQ-Aib- A-αMeF-IEYLIEGGPSSGAPPPS-NH2 | 85 | 4905.6 | N/I |
| 77 | Y-Aib-EGTFTSDYSILLKIAQ-Aib- AFIEYLIEGGPSSGAPPK((2-[2-(2-Amino- ethoxy)-ethoxy]-acetyl)2-(γ-Glu)-CO-(CH2)18- CO2H)S-NH2 | 86 | 4764.5 | N/I |
| 78 | (D-Tyr)-Aib-EGTFTSDYSILLDKK((2-[2-(2- Amino-ethoxy)-ethoxy]-acetyl)2-(γ-Glu)-CO- (CH2)18-CO2H)AQ-Aib- AFIEYLIEGGPSSGAPPPS-NH2 | 87 | 4863.5 | N/I |
| 79 | Ac-(D-Tyr)-AEGTFTSDYSILLDKK((2-[2- (2-Amino-ethoxy)-ethoxy]-acetyl)2-(γ-Glu)- CO-(CH2)18-CO2H)AQ-Aib- AFIEYLIEGGPSSGAPPPS-NH2 | 88 | 4891.5 | N/I |
| 80 | Y-(D-Ala)-EGTFTSDYSILLDKK((2-[2-(2- Amino-ethoxy)-ethoxy]-acetyl)2-(γ-Glu)-CO- (CH2)18-CO2H)AQ-Aib- AFIEYLIEGGPSSGAPPPS-NH2 | 89 | 4849.5 | N/I |
| 81 | Y-Aib-EGTFTSDY-(D-Ser)-ILLDKK((2-[2- (2-Amino-ethoxy)-ethoxy]-acetyl)2-(γ-Glu)- CO-(CH2)18-CO2H)AQ-Aib- AFIEYLIEGGPSSGAPPPS-NH2 | 90 | 4863.5 | N/I |
| 82 | Y-Aib-EGTFTSDYS-(D-Ile)-LLDKK((2-[2- (2-Amino-ethoxy)-ethoxy]-acetyl)2-(γ-Glu)- CO-(CH2)18-CO2H)AQ-Aib- AFIEYLIEGGPSSGAPPPS-NH2 | 91 | 4863.5 | N/I |
| 83 | Y-Aib-EGTFTSDYSILLDKK((2-[2-(2- Amino-ethoxy)-ethoxy]-acetyl)2-(γ-Glu)-CO- (CH2)18-CO2H)AQ-Aib-AFIEYLI-(D-Glu)- GGPSSGAPPPS-NH2 | 92 | 4863.5 | N/I |
| 84 | Y-Aib-EGTFTSDYSILLDKK((2-[2-(2- Amino-ethoxy)-ethoxy]-acetyl)2-(γ-Glu)-CO- (CH2)18-CO2H)AQ-Aib-AFI-(D-Glu)- YLIEGGPSSGAPPPS-NH2 | 93 | 4863.5 | N/I |
| 85 | Y-Aib-EGTFTSDASILLDKK((2-[2-(2- Amino-ethoxy)-ethoxy]-acetyl)2-(γ-Glu)-CO- (CH2)18-CO2H)AQ-Aib- AFIEYLIEGGPSSGAPPPS-NH2 | 94 | 4771.4 | N/I |
| 86 | Y-Aib-EGTFTSDYSILLDKK((2-[2-(2- Amino-ethoxy)-ethoxy]-acetyl)2-(γ-Glu)-CO- (CH2)18-CO2H)AQ-Aib- AFIEYLIEAGPSSGAPPPS-NH2 | 95 | 4877.5 | N/I |
| 87 | Y-αMePro-EGTFTSDYSILLDKK((2-[2-(2- Amino-ethoxy)-ethoxy]-acetyl)2-(γ-Glu)-CO- (CH2)18-CO2H)AQ-Aib- AFIEYLIEGGPSSGAPPPS-NH2 | 96 | 4889.5 | N/I |
| 88 | Y-Pro-EGTFTSDYSILLDKK((2-[2-(2- Amino-ethoxy)-ethoxy]-acetyl)2-(γ-Glu)-CO- (CH2)18-CO2H)AQ-Aib- AFIEYLIEGGPSSGAPPPS-NH2 | 97 | 4875.5 | N/I |
| 89 | Y-Aib-Aad-GTFTSDYSILLDKK((2-[2-(2- Amino-ethoxy)-ethoxy]-acetyl)2-(γ-Glu)-CO- (CH2)18-CO2H)AQ-Aib- AFIEYLIEGGPSSGAPPPS-NH2 | 98 | 4877.5 | N/I |
| 90 | Y-Aib-NGTFTSDYSILLDKK((2-[2-(2- Amino-ethoxy)-ethoxy]-acetyl)2-(γ-Glu)-CO- (CH2)18-CO2H)AQ-Aib- AFIEYLIEGGPSSGAPPPS-NH2 | 99 | 4848.5 | N/I |
| 91 | Y-Aib-(γ-Glu)-GTFTSDYSILLDKK((2-[2- (2-Amino-ethoxy)-ethoxy]-acetyl)₂-(γ-Glu)- CO-(CH₂)₁₈-CO₂H)AQ-Aib- AFIEYLIEGGPSSGAPPPS-NH₂ | 100 | 4863.5 | N/I |
| 92 | Y-Aib-EGT-αMeF-TSDK((2-[2-(2-Amino- ethoxy)-ethoxy]-acetyl)2-(γ-Glu)-CO-(CH2)18- CO2H)SILLDKIAQ-Aib-AFIEYLIEGG-NH2 | 101 | 4049.7 | N/I |
| 93 | Y-Aib-EGT-αMeF-TSDYSILK((2-[2-(2- Amino-ethoxy)-ethoxy]-acetyl)2-(γ-Glu)-CO- (CH2)18-CO2H)DKIAQ-Aib-AFIEYLIEGG- NH2 | 102 | 4099.7 | N/I |
| 94 | Y-Aib-EGT-αMeF-TSDYSILLDKIAQ-Aib- K((2-[2-(2-Amino-ethoxy)-ethoxy]-acetyl)2- (γ-Glu)-CO-(CH2)18-CO2H)FIEYLIEGG-NH2 | 103 | 4141.8 | N/I |
| 95 | Y-Aib-EGT-αMeF-TSDYSILLDKIAQ-Aib- AFIEYLIK((2-[2-(2-Amino-ethoxy)-ethoxy]- acetyl)2-(γ-Glu)-CO-(CH2)18-CO2H)GG-NH2 | 104 | 4083.7 | N/I |
| 96 | Y-Aib-EGTFTSDK((2-[2-(2-Amino-ethoxy)- ethoxy]-acetyl)2-(γ-Glu)-CO-(CH2)18- CO2H)SI-αMeL-LDKIAQ-Aib- AFIEYLIEGG-NH2 | 105 | 4049.7 | N/I |
| 97 | Y-Aib-EGTFTSDYSI-αMeL-LDKIAQ-Aib- AFIEYLIK((2-[2-(2-Amino-ethoxy)-ethoxy]- acetyl)2-(γ-Glu)-CO-(CH2)18-CO2H)GG-NH2 | 106 | 4083.7 | N/I |
| 98 | Y-Aib-EGT-αMeF-TSDYSILLDKK((2-[2- (2-Amino-ethoxy)-ethoxy]-acetyl)2-(γ-Glu)- CO-(CH2)18-CO2H)AQ-Aib-AFIEYLIEGG- NH2 | 107 | 4099.7 | N/I |
| 99 | Y-Aib-EGT-αMeF-TSDYSI-αMeL- LDKK((2-[2-(2-Amino-ethoxy)-ethoxy]- acetyl)2-(γ-Glu)-CO-(CH2)18-CO2H)AQ-Aib- AFIEYLIEGG-NH2 | 108 | 4113.7 | N/I |
| 100 | Y-Aib-EGT-αMeF-TSDYSI-αMeL- LDEK((2-[2-(2-Amino-ethoxy)-ethoxy]- acetyl)2-(γ-Glu)-CO-(CH2)18-CO2H)AQ-Aib- AFIEYLIEGG-NH2 | 109 | 4114.7 | N/I |
| 101 | Y-Aib-EGT-αMeF(2F)-TSDYSI-Aib- LDEK((2-[2-(2-Amino-ethoxy)-ethoxy]- acetyl)2-(γ-Glu)-CO-(CH2)18-CO2H)AQ-Aib- AFIEYLIEGG-NH2 | 110 | 4090.6 | N/I |
| 102 | Y-Aib-EGT-αMeF-TSDYSI-Aib-LDEK((2- [2-(2-Amino-ethoxy)-ethoxy]-acetyl)2-(γ- Glu)-CO-(CH2)18-CO2H)AQ-Aib- AFIEYLIEGG-NH2 | 111 | 4072.6 | N/I |
| 103 | Y-Aib-EGT-αMeF(2F)-TSDYSI-αMeL- LDEK((2-[2-(2-Amino-ethoxy)-ethoxy]- acetyl)2-(γ-Glu)-CO-(CH2)18-CO2H)AQ-Aib- EFIEYLIEGG-NH2 | 112 | 4190.7 | N/I |
| 104 | Y-Aib-EGT-αMeF(2F)-TSDYSI-αMeL- LDEK((2-[2-(2-Amino-ethoxy)-ethoxy]- acetyl)₂-(γ-Glu)-CO-(CH₂)₁₆-CO₂H)AQ-Aib- EFIEYLIEGG-NH₂ | 113 | 4162.6 | N/I |
| 105 | DesHis-ψ[NHCO]-Aib- EGTFTSDYSILLDKK((2-[2-(2-Amino- ethoxy)-ethoxy]-acetyl)2-(γ-Glu)-CO-(CH2)18- CO2H)AQ-Aib-AFIEYLIEGGPSSGAPPPS- NH2 | 114 | 4822.5 | N/I |
| 106 | DesHis-Aib-EGTFTSDYSILLDKK((2-[2-(2- Amino-ethoxy)-ethoxy]-acetyl)2-(γ-Glu)-CO- (CH2)18-CO2H)AQ-Aib- AFIEYLIEGGPSSGAPPPS-NH2 | 115 | 4822.5 | N/I |
| 107 | DesTyr-Aib-EGTFTSDYSILLDKK((2-[2-(2- Amino-ethoxy)-ethoxy]-acetyl)₂-(γ-Glu)-CO- (CH₂)₁₈-CO₂H)AQ-Aib- AFIEYLIEGGPSSGAPPPS-NH₂ | 116 | 4848.5 | N/I |
| 108 | Y-Aib-EGTFTSDYSILLDKK((2-[2-(2- Amino-ethoxy)-ethoxy]-acetyl)-AOC-(γ- Glu)-CO-(CH2)18-CO2H)AQ-Aib- AFIEYLIEGGPSSGAPPPS-NH2 | 117 | 4859.6 | N/I |
| 109 | Y-Aib-EGTFTSDYSILLDKK(AOC-(2-[2-(2- Amino-ethoxy)-ethoxy]-acetyl)-(γ-Glu)-CO- (CH2)18-CO2H)AQ-Aib- AFIEYLIEGGPSSGAPPPS-NH2 | 118 | 4859.6 | N/I |
| 110 | Y-Aib-EGTFTSDYSILLDKK((2-[2-(2- Amino-ethoxy)-ethoxy]-acetyl)-(γ-Glu)- (Trx)-CO-(CH2)18-CO2H)AQ-Aib- AFIEYLIEGGPSSGAPPPS-NH2 | 119 | | N/I |
| 111 | Y-Aib-EGTFTSDYSILLDKK((2-[2-(2- Amino-ethoxy)-ethoxy]-acetyl)-(Trx)-(γ- Glu)-CO-(CH2)18-CO2H)AQ-Aib- AFIEYLIEGGPSSGAPPPS-NH2 | 120 | | N/I |
| 112 | Y-Aib-EGTFTSDYSILLDKK((2-[2-(2- Amino-ethoxy)-ethoxy]-acetyl)-(εK)-(γ-Glu)- CO-(CH2)18-CO2H)AQ-Aib- AFIEYLIEGGPSSGAPPPS-NH2 | 121 | 4846.5 | N/I |
| 113 | Y-Aib-EGTFTSDYSILLDKK((2-[2-(2- Amino-ethoxy)-ethoxy]-acetyl)2-(εK)-CO- (CH2)18-CO2H)AQ-Aib- AFIEYLIEGGPSSGAPPPS-NH2 | 122 | 4862.6 | N/I |
| 114 | Y-Aib-EGTFTSDYSILLDKK((2-[2-(2- Amino-ethoxy)-ethoxy]-acetyl)-(εK)-(εK)- CO-(CH2)18-CO2H)AQ-Aib- AFIEYLIEGGPSSGAPPPS-NH2 | 123 | 4845.6 | N/I |
| 115 | Y-Aib-EGT-αMeF-TSDYSI-αMeL- LDEK((2-[2-(2-Amino-ethoxy)-ethoxy]- acetyl)2-(γ-Glu)-CO-(CH2)18-CO2H)AQ-Aib- AFIEYLIEGGPSSGAPPPS-NH2 | 124 | 4892.5 | N/I |
| 116 | Y-Aib-EGT-αMeF-TSDYSI-αMeL- LDEK((2-[2-(2-Amino-ethoxy)-ethoxy]- acetyl)2-(γ-Glu)-CO-(CH2)18-CO2H)AQ-Aib- EFIEYLIEGGPSSGAPPPS-NH2 | 125 | 4950.5 | N/I |
| 117 | Y-Aib-EGT-αMeF-TSDYSI-Aib-LDEK((2- [2-(2-Amino-ethoxy)-ethoxy]-acetyl)2-(γ- Glu)-CO-(CH2)18-CO2H)AQ-Aib- AFIEYLIEGGPSSGAPPPS-NH2 | 126 | 4850.4 | N/I |
| 118 | Y-Aib-EGT-αMeF(2F)-TSDYSI-αMeL- LDEK((2-[2-(2-Amino-ethoxy)-ethoxy]- acetyl)2-(γ-Glu)-CO-(CH2)18-CO2H)AQ-Aib- EFIEYLIEGGPSSGAPPPS-NH2 | 127 | 4968.5 | N/I |
| 119 | F-Aib-EGT-αMeF-TSDYSI-αMeL- LDEK((2-[2-(2-Amino-ethoxy)-ethoxy]- acetyl)2-(γ-Glu)-CO-(CH2)18-CO2H)AQ-Aib- AFIEYLIEGGPSSGAPPPS-NH2 | 128 | 4876.5 | N/I |
| 120 | Y-Aib-cTA-GT-αMeF-TSDYSI-αMeL- LDEK((2-[2-(2-Amino-ethoxy)-ethoxy]- acetyl)2-(γ-Glu)-CO-(CH2)18-CO2H)AQ-Aib- AFIEYLIEGGPSSGAPPPS-NH2 | 129 | 4902.5 | N/I |
| 121 | Y-Aib-EGT-αMeF-TSDYSI-αMeL- LDEK((2-[2-(2-Amino-ethoxy)-ethoxy]- acetyl)2-(γ-Glu)-CO-(CH2)18- CO2H)AQQAFIEYLIEGGPSSGAPPPS-NH2 | 130 | 4935.5 | N/I |
| 122 | Y-Aib-EGT-αMeF-TSDYSI-αMeL- LDEK((2-[2-(2-Amino-ethoxy)-ethoxy]- acetyl)2-(γ-Glu)-CO-(CH2)18- CO2H)AQQAFIEYLIE-Aib-GPSSGAPPPS- NH2 | 131 | 4963.6 | N/I |
| 123 | Y-Aib-EGT-αMeF-TSDYSI-αMeL- LDKK((2-[2-(2-Amino-ethoxy)-ethoxy]- acetyl)2-(γ-Glu)-CO-(CH2)18-CO2H)AQ-Aib- EFIEYLIEGGPSSG-NH2 | 132 | 4500.1 | N/I |
| 124 | Y-Aib-EGT-αMeF-TSDYSI-αMeL- LDEK((2-[2-(2-Amino-ethoxy)-ethoxy]- acetyl)2-(γ-Glu)-CO-(CH2)18-CO2H)AQ-Aib- EFIEYLIEGGPSSG-NH2 | 133 | 4501.0 | N/I |
| 125 | Y-Aib-EGT-αMeF-TSDYSI-αMeL- LDKK((2-[2-(2-Amino-ethoxy)-ethoxy]- acetyl)2-(γ-Glu)2-CO-(CH2)18-CO2H)AQ-Aib- AFIEYLIEGGPSSGAPPPS-NH2 | 134 | 5020.7 | N/I |
| 126 | Y-Aib-EGT-αMeF-TSDYSI-αMeL- LDKK((2-[2-(2-Amino-ethoxy)-ethoxy]- acetyl)2-(γ-Glu)-CO-(CH2)18-CO2H)AQ-Aib- AFIEYLIEAGPSSGAPPPS-NH2 | 135 | 4905.6 | N/I |
| 127 | Y-Aib-EGT-αMeF-TSDISILLDKK((2-[2-(2- Amino-ethoxy)-ethoxy]-acetyl)₂-(γ-Glu)-CO- (CH₂)₁₈-CO₂H)AQ-Aib- AFIEYLIEGGPSSGAPPPS-NH₂ | 136 | 4827.5 | N/I |
| 128 | Y-Aib-EGT-αMeF-TSDHSILLDKK((2-[2- (2-Amino-ethoxy)-ethoxy]-acetyl)2-(γ-Glu)- CO-(CH2)18-CO2H)AQ-Aib- AFIEYLIEGGPSSGAPPPS-NH2 | 137 | 4851.5 | N/I |
| 129 | Y-Aib-EGT-αMeF-TSDLSILLDKK((2-[2- (2-Amino-ethoxy)-ethoxy]-acetyl)2-(γ-Glu)- CO-(CH2)18-CO2H)AQ-Aib- AFIEYLIEGGPSSGAPPPS-NH2 | 138 | 4827.5 | N/I |
| 130 | Y-Aib-EGT-αMeF-TSDESILLDKK((2-[2- (2-Amino-ethoxy)-ethoxy]-acetyl)2-(γ-Glu)- CO-(CH2)18-CO2H)AQ-Aib- AFIEYLIEGGPSSGAPPPS-NH2 | 139 | 4843.5 | N/I |
| 131 | Y-Aib-EGT-αMeF-TSD-αMeF- SILLDKK((2-[2-(2-Amino-ethoxy)-ethoxy]- acetyl)2-(γ-Glu)-CO-(CH2)18-CO2H)AQ-Aib- AFIEYLIEGGPSSGAPPPS-NH2 | 140 | 4875.6 | N/I |
| 132 | Y-Aib-EGT-αMeF-TSD-3Pal-SILLDKK((2- [2-(2-Amino-ethoxy)-ethoxy]-acetyl)2-(γ- Glu)-CO-(CH2)18-CO2H)AQ-Aib- AFIEYLIEGGPSSGAPPPS-NH2 | 141 | 4862.5 | N/I |
| 133 | DesTyr-Aib-EGT-αMeF-TSDYSI-Aib- LDEK((2-[2-(2-Amino-ethoxy)-ethoxy]- acetyl)2-(γ-Glu)-CO-(CH2)18-CO2H)AQ-Aib- AFIEYLIEGGPSSGAPPPS-NH2 | 142 | 4835.4 | N/I |
| 134 | DesTyr-Aib-EGT-αMeF(2F)-TSDYSI- αMeL-LDEK((2-[2-(2-Amino-ethoxy)- ethoxy]-acetyl)2-(γ-Glu)-CO-(CH2)18- CO2H)AQ-Aib-EFIEYLIEGGPSSGAPPPS- NH2 | 143 | 4953.5 | N/I |
| 135 | H-Aib-NGTFTSDYSILLDKK((2-[2-(2- Amino-ethoxy)-ethoxy]-acetyl)₂-(γ-Glu)-CO- (CH₂)₁₈-CO₂H)AQ-Aib- AFIEYLIEGGPSSGAPPPS-NH₂ | 144 | 4822.5 | N/I |
| 136 | Y-Aib-EGTFTSDASILLDKK((2-[2-(2- Amino-ethoxy)-ethoxy]-acetyl)2-(γ-Glu)-CO- (CH2)18-CO2H)AQ-Aib- AFIEYLIEAGPSSGAPPPS-NH2 | 145 | 4785.4 | N/I |
| 137 | Y-Aib-EGT-αMeF-TSDYSI-αMeL- LDKK((2-[2-(2-Amino-ethoxy)-ethoxy]- acetyl)2-(γ-Glu)-CO-(CH2)18-CO2H)AQ-Aib- Aad-FIEYLIEGGPSSGAPPPS-NH2 | 146 | 4963.6 | N/I |
| 138 | Y-Aib-EGT-αMeF-TSDYSI-αMeL- LDKK((2-[2-(2-Amino-ethoxy)-ethoxy]- acetyl)2-(γ-Glu)-CO-(CH2)18-CO2H)AQ-Aib- SFIEYLIEGGPSSGAPPPS-NH2 | 147 | 4907.6 | N/I |
| 139 | Y-Aib-EGT-αMeF-TSDYSI-αMeL- LDKK((2-[2-(2-Amino-ethoxy)-ethoxy]- acetyl)2-(γ-Glu)-CO-(CH2)18-CO2H)AQ-Aib- TFIEYLIEGGPSSGAPPPS-NH2 | 148 | 4921.6 | N/I |
| 140 | Y-Aib-EGT-αMeF-TSDYSI-αMeL- LDKK((2-[2-(2-Amino-ethoxy)-ethoxy]- acetyl)2-(γ-Glu)-CO-(CH2)18-CO2H)AQ-Aib- DFIEYLIEGGPSSGAPPPS-NH2 | 149 | 4935.6 | N/I |
| 141 | Y-Aib-EGT-αMeF-TSDYSI-αMeL- LDKK((2-[2-(2-Amino-ethoxy)-ethoxy]- acetyl)2-(γ-Glu)-CO-(CH2)18-CO2H)AQ-Aib- IFIEYLIEGGPSSGAPPPS-NH2 | 150 | 4933.6 | N/I |
| 142 | Y-Aib-EGT-αMeF-TSDYSI-αMeL- LDKK((2-[2-(2-Amino-ethoxy)-ethoxy]- acetyl)2-(γ-Glu)-CO-(CH2)18-CO2H)AQ-Aib- HFIEYLIEGGPSSGAPPPS-NH2 | 151 | 4957.6 | N/I |
| 143 | Y-Aib-EGT-αMeF-TSDYSI-αMeL- LDKK((2-[2-(2-Amino-ethoxy)-ethoxy]- acetyl)2-(γ-Glu)-CO-(CH2)18-CO2H)AQ-Aib- Aib-FIEYLIEGGPSSGAPPPS-NH2 | 152 | 4905.6 | N/I |
| 144 | Y-Aib-EGT-αMeF-TSDYSI-αMeL- LDKK((2-[2-(2-Amino-ethoxy)-ethoxy]- acetyl)2-(γ-Glu)-CO-(CH2)18-CO2H)AQH- Aib-FIEYLIEGGPSSGAPPPS-NH2 | 153 | 4957.6 | N/I |
| 145 | Y-Aib-EGT-αMeF-TSDASI-αMeL- LDKK((2-[2-(2-Amino-ethoxy)-ethoxy]- acetyl)2-(γ-Glu)-CO-(CH2)18-CO2H)AQ-Aib- AFIEYLIEGGPSSGAPPPS-NH2 | 154 | 4799.5 | N/I |
| 146 | Y-Aib-EGT-αMeF(2F)-TSDYSI-αMeL- LDEK((2-[2-(2-Amino-ethoxy)-ethoxy]- acetyl)2-(γ-Glu)-CO-(CH2)18-CO2H)AQ-Aib- EFIQYLIEGGPSSGAPPPS-NH2 | 155 | 4967.5 | N/I |
| 147 | Y-Aib-EGT-αMeF(2F)-TSDYSI-αMeL- LDEK((2-[2-(2-Amino-ethoxy)-ethoxy]- acetyl)2-(γ-Glu)-CO-(CH2)18-CO2H)AQ-Aib- EFI-Aad-YLIEGGPSSGAPPPS-NH2 | 156 | 4982.6 | N/I |
| 148 | Y-Aib-EGT-αMeF(2F)-TSDYSI-αMeL- LDEK((2-[2-(2-Amino-ethoxy)-ethoxy]- acetyl)2-(γ-Glu)-CO-(CH2)18-CO2H)AQ-Aib- EFIAYLIEGGPSSGAPPPS-NH2 | 157 | 4910.5 | N/I |
| 149 | Y-Aib-EGT-αMeF(2F)-TSDYSI-αMeL- LDEK((2-[2-(2-Amino-ethoxy)-ethoxy]- acetyl)2-(γ-Glu)-CO-(CH2)18-CO2H)AQ-Aib- EFIVYLIEGGPSSGAPPPS-NH2 | 158 | 4938.5 | N/I |
| 150 | Y-Aib-EGT-αMeF(2F)-TSDYSI-αMeL- LDEK((2-[2-(2-Amino-ethoxy)-ethoxy]- acetyl)2-(γ-Glu)-CO-(CH2)18-CO2H)AQ-Aib- EFISYLIEGGPSSGAPPPS-NH2 | 159 | 4926.5 | N/I |
| 151 | Y-Aib-EGT-αMeF(2F)-TSDYSI-αMeL- LDEK((2-[2-(2-Amino-ethoxy)-ethoxy]- acetyl)2-(γ-Glu)-CO-(CH2)18-CO2H)AQ-Aib- EFIPYLIEGGPSSGAPPPS-NH2 | 160 | 4936.5 | N/I |
| 152 | Y-Aib-EGT-αMeF(2F)-TSDYSI-αMeL- LDEK((2-[2-(2-Amino-ethoxy)-ethoxy]- acetyl)2-(γ-Glu)-CO-(CH2)18-CO2H)AQ-Aib- EFI-Aib-YLIEGGPSSGAPPPS-NH2 | 161 | 4924.5 | N/I |
| 153 | Y-Aib-EGT-αMeF(2F)-TSDYSI-αMeL- LDEK((2-[2-(2-Amino-ethoxy)-ethoxy]- acetyl)2-(γ-Glu)-CO-(CH2)18-CO2H)AQ-Aib- EFIHYLIEGGPSSGAPPPS-NH2 | 162 | 4976.6 | N/I |
| 154 | Y-Aib-EGT-αMeF(2F)-TSDHSI-αMeL- LDEK((2-[2-(2-Amino-ethoxy)-ethoxy]- acetyl)2-(γ-Glu)-CO-(CH2)18-CO2H)AQ-Aib- EFIEYLIEGGPSSGAPPPS-NH2 | 163 | 4942.5 | N/I |
| 155 | Y-Aib-EGT-αMeF(2F)-TSD-cTA-SI-αMeL- LDEK((2-[2-(2-Amino-ethoxy)-ethoxy]- acetyl)2-(γ-Glu)-CO-(CH2)18-CO2H)AQ-Aib- EFIEYLIEGGPSSGAPPPS-NH2 | 164 | 4944.5 | N/I |
| 156 | Y-Aib-EGT-αMeF(2F)-TSD-2Pal-SI-αMeL- LDEK((2-[2-(2-Amino-ethoxy)-ethoxy]- acetyl)2-(γ-Glu)-CO-(CH2)18-CO2H)AQ-Aib- EFIEYLIEGGPSSGAPPPS-NH2 | 165 | 4953.5 | N/I |
| 157 | Y-Aib-EGT-αMeF(2F)-TSD-3Pal-SI-αMeL- LDEK((2-[2-(2-Amino-ethoxy)-ethoxy]- acetyl)2-(γ-Glu)-CO-(CH2)18-CO2H)AQ-Aib- EFIEYLIEGGPSSGAPPPS-NH2 | 166 | 4953.5 | N/I |
| 158 | Y-Aib-EGT-αMeF(2F)-TSD-4Pal-SI-αMeL- LDEK((2-[2-(2-Amino-ethoxy)-ethoxy]- acetyl)2-(γ-Glu)-CO-(CH2)18-CO2H)AQ-Aib- EFIEYLIEGGPSSGAPPPS-NH2 | 167 | 4953.5 | N/I |
| 159 | Y-Aib-EGT-αMeF(2F)-TSD-αMeF-SI- αMeL-LDEK((2-[2-(2-Amino-ethoxy)- ethoxy]-acetyl)2-(γ-Glu)-CO-(CH2)18- CO2H)AQ-Aib-EFIEYLIEGGPSSGAPPPS- NH2 | 168 | 4938.5 | N/I |
| 160 | Y-Aib-EGT-αMeF(2F)-TSD-Aib-SI-αMeL- LDEK((2-[2-(2-Amino-ethoxy)-ethoxy]- acetyl)2-(γ-Glu)-CO-(CH2)18-CO2H)AQ-Aib- TFI-(D-Glu)-YLIEGGPSSGAPPPS-NH2 | 169 | 4862.4 | N/I |
| 161 | Y-Aib-EGT-αMeF(2F)-TSDHSI-αMeL- LDEK((2-[2-(2-Amino-ethoxy)-ethoxy]- acetyl)2-(γ-Glu)2-CO-(CH2)18-CO2H)AQ-Aib- TFI-(D-Glu)-YLIEGGPSSG-NH2 | 170 | 4594.1 | N/I |
| 162 | Y-Aib-EGT-αMeF(2F)-TSDHSI-αMeL- LDEK((2-[2-(2-Amino-ethoxy)-ethoxy]- acetyl)2-(γ-Glu)2-CO-(CH2)18-CO2H)AQ-Aib- TFI-(D-Glu)-HLIEGGPSSG-NH2 | 171 | 4568.1 | N/I |
| 163 | H-Aib-EGT-αMeF(2F)-TSDYSI-αMeL- LDEK((2-[2-(2-Amino-ethoxy)-ethoxy]- acetyl)2-(γ-Glu)-CO-(CH2)18-CO2H)AQ-Aib- EFIEYLIEGGPSSGAPPPS-NH2 | 172 | 4942.5 | N/I |
| 164 | Y-Aib-EGT-αMeF(2F)-TSDHSI-αMeL- LDEK((2-[2-(2-Amino-ethoxy)-ethoxy]- acetyl)2-(γ-Glu)-CO-(CH2)18-CO2H)AQ-Aib- TFI-(D-Glu)-YLI-(D-Glu)-GGPSSGAPPPS- NH2 | 173 | 4914.5 | N/I |
| 165 | Y-Aib-EGT-αMeF(2F)-TSDHSI-αMeL- LDEK((2-[2-(2-Amino-ethoxy)-ethoxy]- acetyl)2-(γ-Glu)-CO-(CH2)18-CO2H)AQ-Aib- TFI-(D-Glu)-αMeF-LI-(D-Glu)- GGPSSGAPPPS-NH2 | 174 | 4912.5 | N/I |
| 166 | Y-Aib-EGT-αMeF(2F)-TSDHSI-αMeL- LDEK((2-[2-(2-Amino-ethoxy)-ethoxy]- acetyl)2-(γ-Glu)-CO-(CH2)18-CO2H)AQ-Aib- TFI-(D-Glu)-YLIEGG-NH2 | 175 | 4136.7 | N/I |
| 167 | Y-Aib-EGT-αMeF(2F)-TSDHSI-αMeL- LDEK((2-[2-(2-Amino-ethoxy)-ethoxy]- acetyl)2-(γ-Glu)-CO-(CH2)18-CO2H)AQ-Aib- TFI-(D-Glu)-YLIEGGPSSG-NH2 | 176 | 4465.0 | N/I |
| 168 | Y-Aib-EGT-αMeF(2F)-TSDHSI-αMeL- LDEK((2-[2-(2-Amino-ethoxy)-ethoxy]- acetyl)2-(γ-Glu)-CO-(CH2)18-CO2H)AQ-Aib- TFI-(D-Glu)-YLIEGGPSSGAPPPS-NH2 | 177 | 4914.5 | N/I |
| 169 | Y-Aib-EGT-αMeF(2F)-TSDHSI-αMeL- LDEK((2-[2-(2-Amino-ethoxy)-ethoxy]- acetyl)2-(γ-Glu)-CO-(CH2)16-CO2H)AQ-Aib- TFI-(D-Glu)-YLIEGGPSSGAPPPS-NH2 | 178 | 4886.4 | N/I |
| 170 | Y-Aib-EGT-αMeF(2F)-TSDHSI-αMeL- LDEK((2-[2-(2-Amino-ethoxy)-ethoxy]- acetyl)2-(γ-Glu)-CO-(CH2)14-CO2H)AQ-Aib- TFI-(D-Glu)-YLIEGGPSSGAPPPS-NH2 | 179 | 4858.4 | N/I |
| 171 | Y-Aib-EGT-αMeF(2F)-TSDHSI-αMeL-LD- Orn-K((2-[2-(2-Amino-ethoxy)-ethoxy]- acetyl)2-(γ-Glu)-CO-(CH2)18-CO2H)AQ-Aib- TFI-(D-Glu)-YLIEGGPSSGAPPPS-NH2 | 180 | 4899.5 | N/I |
| 172 | Y-Aib-EGT-αMeF(2F)-TSDHSI-αMeL-LD- Dab-K((2-[2-(2-Amino-ethoxy)-ethoxy]- acetyl)2-(γ-Glu)-CO-(CH2)18-CO2H)AQ-Aib- TFI-(D-Glu)-YLIEGGPSSGAPPPS-NH2 | 181 | 4885.5 | N/I |
| 173 | Y-Aib-EGT-αMeF(2F)-TSDHSI-αMeL-LD- Dap-K((2-[2-(2-Amino-ethoxy)-ethoxy]- acetyl)₂-(γ-Glu)-CO-(CH₂)₁₈-CO₂H)AQ-Aib- TFI-(D-Glu)-YLIEGGPSSGAPPPS-NH₂ | 182 | 4871.5 | N/I |
| 174 | Y-Aib-EGT-αMeF(2F)-TSDHSI-αMeL- LDEK((2-[2-(2-Amino-ethoxy)-ethoxy]- acetyl)2-CO-(CH2)18-CO2H)AQ-Aib-TFI-(D- Glu)-YLIEGGPSSGAPPPS-NH2 | 183 | 4785.4 | N/I |
| 175 | Y-Aib-EGT-αMeF(2F)-TSDHSI-αMeL- LDEK((2-[2-(2-Amino-ethoxy)-ethoxy]- acetyl)2-(εK)-CO-(CH2)18-CO2H)AQ-Aib- TFI-(D-Glu)-YLIEGGPSSGAPPPS-NH2 | 184 | 4913.5 | N/I |
| 176 | Y-Aib-EGT-αMeF(2F)-TSDHSI-αMeL- LDEK((2-[2-(2-Amino-ethoxy)-ethoxy]- acetyl)2-(εK)-CO-(CH2)16-CO2H)AQ-Aib- TFI-(D-Glu)-YLIEGGPSSGAPPPS-NH2 | 185 | 4885.5 | N/I |
| 177 | Y-Aib-EGT-αMeF(2F)-TSDHSI-αMeL- LDEK((2-[2-(2-Amino-ethoxy)-ethoxy]- acetyl)2-(γ-Glu)-CO-(CH2)16-CO2H)AQ-Aib- HFI-(D-Glu)-YLIEGGPSSGAPPPS-NH2 | 186 | 4922.5 | N/I |
| 178 | Y-Aib-EGT-αMeF(2F)-TSDHSI-αMeL- LDEK((2-[2-(2-Amino-ethoxy)-ethoxy]- acetyl)2-(γ-Glu)-CO-(CH2)14-CO2H)AQ-Aib- HFI-(D-Glu)-YLIEGGPSSGAPPPS-NH2 | 187 | 4894.4 | N/I |
| 179 | Y-Aib-EGT-αMeF(2F)-TSDHSI-αMeL- LDEK((2-[2-(2-Amino-ethoxy)-ethoxy]- acetyl)2-(γ-Glu)-CO-(CH2)16-CO2H)AQ-Aib- HFI-(D-Glu)-YLIEGGPSSG-NH2 | 188 | 4473.0 | N/I |
| 180 | Y-Aib-EGT-αMeF(2F)-TSDHSI-αMeL- LDEK((2-[2-(2-Amino-ethoxy)-ethoxy]- acetyl)2-(γ-Glu)-CO-(CH2)16-CO2H)AQ-Aib- HFI-(D-Glu)-YLIEGG-NH2 | 189 | 4144.6 | N/I |
| 181 | Y-Aib-EGT-αMeF(2F)-TSD-3Pal-SI-αMeL- LDEK((2-[2-(2-Amino-ethoxy)-ethoxy]- acetyl)2-(γ-Glu)-CO-(CH2)18-CO2H)AQ-Aib- EFI-(D-Glu)-YLIEGGPSSGAPPPS-NH2 | 190 | 4953.5 | N/I |
| 182 | Y-Aib-EGT-αMeF(2F)-TSD-4Pal-SI-αMeL- LDEK((2-[2-(2-Amino-ethoxy)-ethoxy]- acetyl)2-(γ-Glu)-CO-(CH2)18-CO2H)AQ-Aib- EFI-(D-Glu)-YLIEGGPSSGAPPPS-NH2 | 191 | 4953.5 | N/I |
| 183 | Y-Aib-EGT-αMeF(2F)-TSDLSI-αMeL- LDEK((2-[2-(2-Amino-ethoxy)-ethoxy]- acetyl)2-(γ-Glu)-CO-(CH2)18-CO2H)AQ-Aib- EFI-(D-Glu)-YLIEGGPSSGAPPPS-NH2 | 192 | 4918.5 | N/I |
| 184 | Y-Aib-EGT-αMeF(2F)-TSD-(D-Tyr)-SI- αMeL-LDEK((2-[2-(2-Amino-ethoxy)- ethoxy]-acetyl)2-(γ-Glu)-CO-(CH2)18- CO2H)AQ-Aib-EFI-(D-Glu)- YLIEGGPSSGAPPPS-NH2 | 193 | 4968.5 | N/I |
| 185 | Y-Aib-EGT-αMeF(2F)-TSD-(D-His)-SI- αMeL-LDEK((2-[2-(2-Amino-ethoxy)- ethoxy]-acetyl)2-(γ-Glu)-CO-(CH2)18- CO2H)AQ-Aib-EFI-(D-Glu)- YLIEGGPSSGAPPPS-NH2 | 194 | 4942.5 | N/I |
| 186 | Y-Aib-EGT-aMeF(2F)-TSD-αMeY-SI- αMeL-LDEK((2-[2-(2-Amino-ethoxy)- ethoxy]-acetyl)2-(γ-Glu)-CO-(CH2)18- CO2H)AQ-Aib-EFI-(D-Glu)- YLIEGGPSSGAPPPS-NH2 | 195 | 4982.6 | N/I |
| 187 | Y-Aib-EGT-αMeF(2F)-TSDQSI-αMeL- LDEK((2-[2-(2-Amino-ethoxy)-ethoxy]- acetyl)2-(γ-Glu)-CO-(CH2)18-CO2H)AQ-Aib- EFI-(D-Glu)-YLIEGGPSSGAPPPS-NH2 | 196 | 4933.5 | N/I |
| 188 | Y-Aib-EGT-αMeF(2F)-TSD-3Pal-SI-αMeL- LDEK((2-[2-(2-Amino-ethoxy)-ethoxy]- acetyl)2-(γ-Glu)-CO-(CH2)18-CO2H)AQ-Aib- EFI-Aib-YLIEGGPSSGAPPPS-NH2 | 197 | 4909.5 | N/I |
| 189 | Y-Aib-EGT-αMeF(2F)-TSD-4Pal-SI-αMeL- LDEK((2-[2-(2-Amino-ethoxy)-ethoxy]- acetyl)2-(γ-Glu)-CO-(CH2)18-CO2H)AQ-Aib- EFI-Aib-YLIEGGPSSGAPPPS-NH2 | 198 | 4909.5 | N/I |
| 190 | H-Aib-EGT-αMeF(2F)-TSD-3Pal-SI-αMeL- LDEK((2-[2-(2-Amino-ethoxy)-ethoxy]- acetyl)2-(γ-Glu)-CO-(CH2)18-CO2H)AQ-Aib- EFI-(D-Glu)-YLIEGGPSSGAPPPS-NH2 | 199 | 4927.5 | N/I |
| 191 | Y-Aib-EGT-αMeF(2F)-TSDVSI-αMeL- LDEK((2-[2-(2-Amino-ethoxy)-ethoxy]- acetyl)2-(γ-Glu)-CO-(CH2)18-CO2H)AQ-Aib- EFI-(D-Glu)-YLIEGGPSSGAPPPS-NH2 | 200 | 4904.5 | N/I |
| 192 | Y-Aib-EGT-αMeF(2F)-TSDASI-αMeL- LDEK((2-[2-(2-Amino-ethoxy)-ethoxy]- acetyl)₂-(γ-Glu)-CO-(CH₂)₁₈-CO₂H)AQ-Aib- EFI-(D-Glu)-YLIEGGPSSGAPPPS-NH₂ | 201 | 4876.4 | N/I |
| 193 | Y-αMePro-EGTFTSDYSILLDEK((2-[2-(2- Amino-ethoxy)-ethoxy]-acetyl)₂-(γ-Glu)-CO- (CH₂)₁₈- CO₂H)AQQAFIEYLIEGGPSSGAPPPS-NH₂ | 202 | 4933.5 | N/I |
| 194 | Y-αMePro-EGTFTSDYSILLDEK((2-[2-(2- Amino-ethoxy)-ethoxy]-acetyl)2-(γ-Glu)-CO- (CH2)18- CO2H)AQHAFIEYLIEGGPSSGAPPPS-NH2 | 203 | 4942.5 | N/I |
| 195 | Y-αMePro-EGTFTSDYSILLDRK((2-[2-(2- Amino-ethoxy)-ethoxy]-acetyl)2-(γ-Glu)-CO- (CH2)18- CO2H)AQQAFIEYLIEGGPSSGAPPPS-NH2 | 204 | 4960.6 | N/I |
| 196 | Y-αMePro-EGTFTSDYSILLDRK((2-[2-(2- Amino-ethoxy)-ethoxy]-acetyl)2-(γ-Glu)-CO- (CH2)18- CO2H)AQHAFIEYLIEGGPSSGAPPPS-NH2 | 205 | 4969.6 | N/I |
| 197 | Y-αMePro-EGTFTSDYSILLDEK((2-[2-(2- Amino-ethoxy)-ethoxy]-acetyl)2-(γ-Glu)-CO- (CH2)16-CO2H)AQQAFIEYLIEGGPSSG- NH2 | 206 | 4456.0 | N/I |
| 198 | (D-Tyr)-αMePro-EGTFTSDYSILLDEK((2- [2-(2-Amino-ethoxy)-ethoxy]-acetyl)2-(γ- Glu)-CO-(CH2)16- CO2H)AQQAFIEYLIEGGPSSG-NH2 | 207 | 4456.0 | N/I |
| 199 | DesTyr-Aib-EGTFTSDYSILLDEK((2-[2-(2- Amino-ethoxy)-ethoxy]-acetyl)2-(γ-Glu)-CO- (CH2)18- CO2H)AQQAFIEYLIEGGPSSGAPPPS-NH2 | 208 | 4892.5 | N/I |
| 200 | DesTyr-AEGTFTSDYSILLDEK((2-[2-(2- Amino-ethoxy)-ethoxy]-acetyl)2-(γ-Glu)-CO- (CH2)18- CO2H)AQQAFIEYLIEGGPSSGAPPPS-NH2 | 209 | 4878.4 | N/I |
| 201 | DesHis-αMePro-EGTFTSDYSILLDEK((2- [2-(2-Amino-ethoxy)-ethoxy]-acetyl)2-(γ- Glu)-CO-(CH2)18- CO2H)AQQAFIEYLIEGGPSSGAPPPS-NH2 | 210 | 4892.5 | N/I |
| 202 | Y-Aib-EGT-αMeF(2F)-TSD-4Pal-SI-αMeL- LDKK((2-[2-(2-Amino-ethoxy)-ethoxy]- acetyl)-(γ-Glu)-(2-[2-(2-Amino-ethoxy)- ethoxy]-acetyl)-CO-(CH2)18-CO2H)AQ-Aib- EFI-(D-Glu)-YLIEGGPSSGAPPPS-NH2 | 211 | 4938.6 | N/I |
| 203 | Y-Aib-EGT-αMeF(2F)-TSD-4Pal-SI-αMeL- LDKK((2-[2-(2-Amino-ethoxy)-ethoxy]- acetyl)2-(γ-Glu)-CO-(CH2)18-CO2H)AQ-Aib- EFI-(D-Glu)-YLIEGGPSSGAPPPS-NH2 | 212 | 4952.6 | N/I |
| 204 | Y-Aib-EGT-αMeF(2F)-TSD-4Pal-SI-αMeL- LDEK((2-[2-(2-Amino-ethoxy)-ethoxy]- acetyl)2-(γ-Glu)-CO-(CH2)16-CO2H)AQ-Aib- EFI-(D-Glu)-YLIEGGPSSGAPPPS-NH2 | 213 | 4924.5 | N/I |
| 205 | Y-Aib-EGT-αMeF(2F)-TSD-4Pal-SI-αMeL- LDEK((2-[2-(2-Amino-ethoxy)-ethoxy]- acetyl)2-CO-(CH2)16-CO2H)AQ-Aib-EFI-(D- Glu)-YLIEGGPSSGAPPPS-NH2 | 214 | 4795.4 | N/I |
| 206 | Y-Aib-EGT-αMeF(2F)-TSD-4Pal-SI-αMeL- LDEK((2-[2-(2-Amino-ethoxy)-ethoxy]- acetyl)2-CO-(CH2)18-CO2H)AQ-Aib-EFI-(D- Glu)-YLIEGGPSSGAPPPS-NH2 | 215 | 4823.5 | N/I |
| 207 | Y-Aib-EGT-αMeF(2F)-TSD-4Pal-SI-αMeL- LDEK((2-[2-(2-Amino-ethoxy)-ethoxy]- acetyl)2-(εK)-CO-(CH2)18-CO2H)AQ-Aib- EFI-(D-Glu)-YLIEGGPSSGAPPPS-NH2 | 216 | 4923.6 | N/I |
| 208 | Y-Aib-EGT-αMeF(2F)-TSDYSI-αMeL- LDEK((2-[2-(2-Amino-ethoxy)-ethoxy]- acetyl)2-(γ-Glu)-CO-(CH2)14-CO2H)AQ-Aib- EFI-(D-Glu)-YLIEGGPSSGAPPPS-NH2 | 217 | 4912.4 | N/I |
| 209 | Y-Aib-EGT-αMeF(2F)-TSDYSI-αMeL-LD- Dab-K((2-[2-(2-Amino-ethoxy)-ethoxy]- acetyl)2-(γ-Glu)-CO-(CH2)16-CO2H)AQ-Aib- EFI-(D-Glu)-YLIEGGPSSGAPPPS-NH2 | 218 | 4911.4 | N/I |
| 210 | Y-Aib-EGT-αMeF(2F)-TSDYSI-αMeL-LD- Dap-K((2-[2-(2-Amino-ethoxy)-ethoxy]- acetyl)2-(γ-Glu)-CO-(CH2)16-CO2H)AQ-Aib- EFI-(D-Glu)-YLIEGGPSSGAPPPS-NH2 | 219 | 4897.5 | N/I |
| 211 | Y-Aib-EGT-αMeF(2F)-TSDYSI-αMeL- LDKK((2-[2-(2-Amino-ethoxy)-ethoxy]- acetyl)-(γ-Glu)-(2-[2-(2-Amino-ethoxy)- ethoxy]-acetyl)-CO-(CH2)18-CO2H)AQ-Aib- EFI-(D-Glu)-YLIEGGPSSGAPPPS-NH2 | 220 | 4953.6 | N/I |
| 212 | Y-Aib-EGT-αMeF(2F)-TSDYSI-αMeL- LDKK((2-[2-(2-Amino-ethoxy)-ethoxy]- acetyl)2-(γ-Glu)-CO-(CH2)18-CO2H)AQ-Aib- EFI-(D-Glu)-YLIEGGPSSGAPPPS-NH2 | 221 | 4967.5 | N/I |
| 213 | Y-Aib-EGT-αMeF-TSDYSI-αMeL- LDEK((2-[2-(2-Amino-ethoxy)-ethoxy]- acetyl)2-(γ-Glu)-CO-(CH2)16-CO2H)AQ-Aib- EFI-(D-Glu)-YLIEGGPSSGAPPPS-NH2 | 222 | 4922.4 | N/I |
| 214 | Y-Aib-EGT-αMeF(2F)-TSDYSI-αMeL - LDEK((2-[2-(2-Amino-ethoxy)-ethoxy]- acetyl)2-CO-(CH2)16-CO2H)AQ-Aib-EFI-(D- Glu)-YLIEGGPSSGAPPPS-NH2 | 223 | 4811.4 | N/I |
| 215 | Y-Aib-EGT-αMeF(2F)-TSDYSI-αMeL- LDEK((2-[2-(2-Amino-ethoxy)-ethoxy]- acetyl)2-CO-(CH2)18-CO2H)AQ-Aib-EFI-(D- Glu)-YLIEGGPSSGAPPPS-NH2 | 224 | 4839.4 | N/I |
| 216 | Y-Aib-EGT-αMeF(2F)-TSDYSI-αMeL- LDEK((2-[2-(2-Amino-ethoxy)-ethoxy]- acetyl)2-(εK)-CO-(CH2)18-CO2H)AQ-Aib- EFI-(D-Glu)-YLIEGGPSSGAPPPS-NH2 | 225 | 4967.5 | N/I |
| 217 | Y-Aib-EGT-αMeF(2F)-TSDYSI-αMeL-LD- Dab-K((2-[2-(2-Amino-ethoxy)-ethoxy]- acetyl)2-(γ-Glu)-CO-(CH2)18-CO2H)AQ-Aib- EFI-(D-Glu)-YLIEGGPSSGAPPPS-NH2 | 226 | 4939.5 | N/I |
| 218 | Y-Aib-EGT-αMeF(2F)-TSDYSI-αMeL-LD- Dap-K((2-[2-(2-Amino-ethoxy)-ethoxy]- acetyl)2-(γ-Glu)-CO-(CH2)18-CO2H)AQ-Aib- EFI-(D-Glu)-YLIEGGPSSGAPPPS-NH2 | 227 | 4925.5 | N/I |
| 219 | Y-Aib-EGT-αMeF(2F)-TSDYSI-αMeL- LDEK((2-[2-(2-Amino-ethoxy)-ethoxy]- acetyl)2-(γ-Glu)-CO-(CH2)16-CO2H)AQ-Aib- EFI-(D-Glu)-YLIEGGPSSG-NH2 | 228 | 4491.0 | N/I |
| 220 | Y-Aib-EGT-αMeF(2F)-TSDYSI-αMeL- LDEK((2-[2-(2-Amino-ethoxy)-ethoxy]- acetyl)2-(γ-Glu)-CO-(CH2)16-CO2H)AQ-Aib- EFI-(D-Glu)-YLIEGG-NH2 | 229 | 4162.6 | N/I |
| 221 | Y-Aib-EGT-αMeF(2F)-TSDYSI-αMeL- LDEK((2-[2-(2-Amino-ethoxy)-ethoxy]- acetyl)2-(γ-Glu)-CO-(CH2)16-CO2H)AQ-Aib- EFI-(D-Glu)-YLIEGGPSSGAPPPS-NH2 | 230 | 4940.5 | N/I |
| 222 | Y-Aib-EGT-αMeF(2F)-TSDYSI-αMeL- LDEK((2-[2-(2-Amino-ethoxy)-ethoxy]- acetyl)2-(γ-Glu)-CO-(CH2)18-CO2H)AQ-Aib- EFI-(D-Glu)-αMeY-LIEGGPSSGAPPPS- NH2 | 231 | 4982.6 | N/I |
| 223 | Y-Aib-EGT-αMeF(2F)-TSDYSI-αMeL- LDEK((2-[2-(2-Amino-ethoxy)-ethoxy]- acetyl)2-(γ-Glu)-CO-(CH2)18-CO2H)AQ-Aib- EFIE-αMeY-LIEGGPSSGAPPPS-NH2 | 232 | 4982.6 | N/I |
| 224 | Y-Aib-EGT-αMeF(2F)-TSDYSI-Aib- LDEK((2-[2-(2-Amino-ethoxy)-ethoxy]- acetyl)2-(γ-Glu)-CO-(CH2)18-CO2H)AQ-Aib- EFI-(D-Glu)-YLIEGGPSSGAPPPS-NH2 | 233 | 4926.4 | N/I |
| 225 | Y-Aib-EGT-αMeF-TSDYSI-Aib-LDEK((2- [2-(2-Amino-ethoxy)-ethoxy]-acetyl)2-(γ- Glu)-CO-(CH2)18-CO2H)AQ-Aib-EFI-(D- Glu)-YLIEGGPSSGAPPPS-NH2 | 234 | 4908.5 | N/I |
| 226 | Y-Aib-EGT-αMeF(2F)-TSDYSILLDEK((2- [2-(2-Amino-ethoxy)-ethoxy]-acetyl)2-(γ- Glu)-CO-(CH2)18-CO2H)AQ-Aib-EFI-(D- Glu)-YLIEGGPSSGAPPPS-NH2 | 235 | 4954.5 | N/I |
| 227 | Y-Aib-EGT-αMeF-TSDYSI-αMeL- LDEK((2-[2-(2-Amino-ethoxy)-ethoxy]- acetyl)2-(γ-Glu)-CO-(CH2)18-CO2H)AQ-Aib- EFI-(D-Glu)-YLIEGGPSSGAPPPS-NH2 | 236 | 4950.5 | N/I |
| 228 | Y-Aib-EGT-αMeF-TSDYSI-αMeL- LDKK((2-[2-(2-Amino-ethoxy)-ethoxy]- acetyl)2-(γ-Glu)-CO-(CH2)18-CO2H)AQ-Aib- EFI-(D-Glu)-YLIEGGPSSG-NH2 | 237 | 4500.1 | N/I |
| 229 | Y-Aib-EGT-αMeF(2F)-TSDYS-αMeL- LDEK((2-[2-(2-Amino-ethoxy)-ethoxy]- acetyl)2-(γ-Glu)-CO-(CH2)18-CO2H)AQ-Aib- EFI-(D-Glu)-YLIEGGPSSGAPPPS-NH2 | 238 | 4855.4 | N/I |
| 230 | Y-Aib-EGT-αMeF(2F)-TSDYSI-αMeL- LDHK((2-[2-(2-Amino-ethoxy)-ethoxy]- acetyl)2-(γ-Glu)-CO-(CH2)18-CO2H)AQ-Aib- EFI-(D-Glu)-YLIEGGPSSGAPPPS-NH2 | 239 | 4976.6 | N/I |
| 231 | Y-Aib-EGT-αMeF(2F)-TSDYSI-αMeL- LDKK((2-[2-(2-Amino-ethoxy)-ethoxy]- acetyl)2-(γ-Glu)-CO-(CH2)16-CO2H)AQ-Aib- EFI-(D-Glu)-YLIEGGPSSGAPPPS-NH2 | 240 | 4939.5 | N/I |
| 232 | Y-Aib-EGT-αMeF(2F)-TSD-4Pal-SI-αMeL- LD-Orn-K((2-[2-(2-Amino-ethoxy)-ethoxy]- acetyl)2-(γ-Glu)-CO-(CH2)16-CO2H)AQ-Aib- EFI-(D-Glu)-YLIEGGPSSGAPPPS-NH2 | 241 | 4910.5 | N/I |
| 233 | Y-Aib-EGT-αMeF(2F)-TSD-4Pal-SI-αMeL- LD-Dab-K((2-[2-(2-Amino-ethoxy)-ethoxy]- acetyl)2-(γ-Glu)-CO-(CH2)16-CO2H)AQ-Aib- EFI-(D-Glu)-YLIEGGPSSGAPPPS-NH2 | 242 | 4896.5 | N/I |
| 234 | Y-Aib-EGT-αMeF(2F)-TSD-4Pal-SILLD- Dab-K((2-[2-(2-Amino-ethoxy)-ethoxy]- acetyl)2-(γ-Glu)-CO-(CH2)16-CO2H)AQ-Aib- EFI-(D-Glu)-YLIEGGPSSGAPPPS-NH2 | 243 | 4882.4 | N/I |
| 235 | Y-Aib-EGT-αMeF-TSD-4Pal-SI-αMeL-LD- Dab-K((2-[2-(2-Amino-ethoxy)-ethoxy]- acetyl)2-(γ-Glu)-CO-(CH2)16-CO2H)AQ-Aib- EFI-(D-Glu)-YLIEGGPSSGAPPPS-NH2 | 244 | 4878.5 | N/I |
| 236 | Y-Aib-EGT-αMeF(2F)-TSDYSI-αMeL-LD- Orn-K((2-[2-(2-Amino-ethoxy)-ethoxy]- acetyl)2-(γ-Glu)-CO-(CH2)16-CO2H)AQ-Aib- EFI-(D-Glu)-αMeY-LIEGGPSSGAPPPS- NH2 | 245 | 4939.5 | N/I |
| 237 | Y-Aib-EGT-αMeF(2F)-TSDASI-αMeL-LD- Orn-K((2-[2-(2-Amino-ethoxy)-ethoxy]- acetyl)2-(γ-Glu)-CO-(CH2)16-CO2H)AQ-Aib- EFI-(D-Glu)-αMeY-LIEGGPSSGAPPPS- NH2 | 246 | 4847.4 | N/I |
| 238 | Y-Aib-EGT-αMeF(2F)-TSDLSI-αMeL-LD- Orn-K((2-[2-(2-Amino-ethoxy)-ethoxy]- acetyl)2-(γ-Glu)-CO-(CH2)16-CO2H)AQ-Aib- EFI-(D-Glu)-αMeY-LIEGGPSSGAPPPS- NH2 | 247 | 4889.5 | N/I |
| 239 | Y-Aib-EGT-αMeF(2F)-TSD-4Pal-SI-αMeL- LD-Orn-K((2-[2-(2-Amino-ethoxy)-ethoxy]- acetyl)2-(γ-Glu)-CO-(CH2)16-CO2H)AQ-Aib- TFI-(D-Glu)-αMeY-LIEGGPSSGAPPPS- NH2 | 248 | 4896.5 | N/I |
| 240 | Y-Aib-EGT-αMeF(2F)-TSD-4Pal-SI-αMeL- LD-Orn-K((2-[2-(2-Amino-ethoxy)-ethoxy]- acetyl)2-(γ-Glu)-CO-(CH2)16-CO2H)AQ-Aib- AFI-(D-Glu)-αMeY-LIEGGPSSGAPPPS- NH2 | 249 | 4866.5 | N/I |
| 241 | Y-Aib-EGT-αMeF(2F)-TSDVSI-αMeL-LD- Orn-K((2-[2-(2-Amino-ethoxy)-ethoxy]- acetyl)2-(γ-Glu)-CO-(CH2)16-CO2H)AQ-Aib- TFI-(D-Glu)-αMeY-LIEGGPSSGAPPPS- NH2 | 250 | N/I | N/I |
| 242 | Y-Aib-EGT-αMeF(2F)-TSDVSI-αMeL-LD- Orn-K((2-[2-(2-Amino-ethoxy)-ethoxy]- acetyl)2-(γ-Glu)-CO-(CH2)16-CO2H)AQ-Aib- AFI-(D-Glu)-αMeY-LIEGGPSSGAPPPS- NH2 | 251 | N/I | N/I |
| 243 | Y-Aib-EGT-αMeF(2F)-TSDLSI-αMeL-LD- Orn-K((2-[2-(2-Amino-ethoxy)-ethoxy]- acetyl)2-(γ-Glu)-CO-(CH2)16-CO2H)AQ-Aib- TFI-(D-Glu)-αMeY-LIEGGPSSGAPPPS- NH2 | 252 | N/I | N/I |
| 244 | Y-Aib-EGT-αMeF(2F)-TSDLSI-αMeL-LD- Orn-K((2-[2-(2-Amino-ethoxy)-ethoxy]- acetyl)2-(γ-Glu)-CO-(CH2)16-CO2H)AQ-Aib- AFI-(D-Glu)-αMeY-LIEGGPSSGAPPPS- NH2 | 253 | N/I | N/I |
| 245 | Y-Aib-EGT-αMeF(2F)-TSDASI-αMeL-LD- Orn-K((2-[2-(2-Amino-ethoxy)-ethoxy]- acetyl)2-(γ-Glu)-CO-(CH2)16-CO2H)AQ-Aib- TFI-(D-Glu)-αMeY-LIEGGPSSGAPPPS- NH2 | 254 | N/I | N/I |
| 246 | Y-Aib-EGT-αMeF(2F)-TSDASI-αMeL-LD- Orn-K((2-[2-(2-Amino-ethoxy)-ethoxy]- acetyl)2-(γ-Glu)-CO-(CH2)16-CO2H)AQ-Aib- AFI-(D-Glu)-αMeY-LIEGGPSSGAPPPS- NH2 | 255 | N/I | N/I |
| 247 | Y-Aib-EGT-αMeF(2F)-TSDYSI-Aib-LD- Orn-K((2-[2-(2-Amino-ethoxy)-ethoxy]- acetyl)2-(γ-Glu)-CO-(CH2)16-CO2H)AQ-Aib- EFI-(D-Glu)-YLIEGGPSSGAPPPS-NH2 | 256 | 4883.4 | N/I |
| 248 | Y-Aib-EGT-αMeF(2F)-TSDYSILLD-Orn- K((2-[2-(2-Amino-ethoxy)-ethoxy]-acetyl)2- (γ-Glu)-CO-(CH2)16-CO2H)AQ-Aib-EFI-(D- Glu)-YLIEGGPSSGAPPPS-NH2 | 257 | 4911.5 | N/I |
| 249 | Y-Aib-EGT-αMeF(2F)-TSDYSI-Nle-LD- Orn-K((2-[2-(2-Amino-ethoxy)-ethoxy]- acetyl)2-(γ-Glu)-CO-(CH2)16-CO2H)AQ-Aib- EFI-(D-Glu)-YLIEGGPSSGAPPPS-NH2 | 258 | 4911.5 | N/I |
| 250 | Y-Aib-EGT-αMeF(2F)-TSDYSI-Aib-LD- Orn-K((2-[2-(2-Amino-ethoxy)-ethoxy]- acetyl)2-(γ-Glu)-CO-(CH2)18-CO2H)AQ-Aib- EFI-(D-Glu)-YLIEGGPSSGAPPPS-NH2 | 259 | 4911.5 | N/I |
| 251 | Y-Aib-EGT-αMeF(2F)-TSD-4Pal-SI-αMeL- LD-Orn-K((2-[2-(2-Amino-ethoxy)-ethoxy]- acetyl)2-(εK)-CO-(CH2)18-CO2H)AQ-Aib- AFI-(D-Glu)-αMeY-LIEGGPSSGAPPPS- NH2 | 260 | 4893.6 | N/I |
| 252 | Y-Aib-EGT-αMeF(2F)-TSD-4Pal-SI-αMeL- LD-Orn-K((2-[2-(2-Amino-ethoxy)-ethoxy]- acetyl)2-(εK)-CO-(CH2)18-CO2H)AQ-Aib- AFI-(D-Glu)-αMeY-LIAGGPSSGAPPPS- NH2 | 261 | 4835.6 | N/I |
| 253 | Y-Aib-EGTFTSDYSILLDKK((2-[2-(2- Amino-ethoxy)-ethoxy]-acetyl)2-(γ-Glu)-CO- (CH2)18- CO2H)AQAAFIEYLIEGGPSSGAPPPS-NH2 | 262 | 4849.5 | N/I |
| 254 | Y-Aib-EGTFTSDYSILLDKK((2-[2-(2- Amino-ethoxy)-ethoxy]-acetyl)2-(γ-Glu)-CO- (CH2)18- CO2H)AQQAFIEYLIEGGPSSGAPPPS-NH2 | 263 | 4906.5 | N/I |
| 255 | Y-Aib-EGTFTSDYSILLDKK((2-[2-(2- Amino-ethoxy)-ethoxy]-acetyl)2-(γ-Glu)-CO- (CH2)18- CO2H)AQHAFIEYLIEGGPSSGAPPPS-NH2 | 264 | 4915.5 | N/I |
| 256 | Y-Aib-EGTFTSDYSILLDKK((2-[2-(2- Amino-ethoxy)-ethoxy]-acetyl)2-(γ-Glu)-CO- (CH2)18- CO2H)AQKAFIEYLIEGGPSSGAPPPS-NH2 | 265 | 4906.6 | N/I |
| 257 | Y-Aib-EGTFTSDYSILLDKK((2-[2-(2- Amino-ethoxy)-ethoxy]-acetyl)2-(γ-Glu)-CO- (CH2)18- CO2H)AQRAFIEYLIEGGPSSGAPPPS-NH2 | 266 | 4934.6 | N/I |
| 258 | Y-Aib-EGTFTSDYSILLDEK((2-[2-(2- Amino-ethoxy)-ethoxy]-acetyl)2-(γ-Glu)-CO- (CH2)18- CO2H)AQKAFIEYLIEGGPSSGAPPPS-NH2 | 267 | 4907.5 | N/I |
| 259 | Y-Aib-EGTFTSDYSILLDEK((2-[2-(2- Amino-ethoxy)-ethoxy]-acetyl)2-(γ-Glu)-CO- (CH2)18-CO2H)AQ-Aib- AFIEYLIEGGPSSGAPPPS-NH2 | 268 | 4864.4 | N/I |
| 260 | Y-Aib-EGTFTSDHSILLDKK((2-[2-(2- Amino-ethoxy)-ethoxy]-acetyl)2-(γ-Glu)-CO- (CH2)18-CO2H)AQ-Aib- AFIEYLIEGGPSSGAPPPS-NH2 | 269 | 4837.5 | N/I |
| 261 | Y-Aib-EGTFTSDYSILLDKK((2-[2-(2- Amino-ethoxy)-ethoxy]-acetyl)2-(γ-Glu)-CO- (CH2)18- CO2H)AQEAFIEYLIEGGPSSGAPPPS-NH2 | 270 | 4907.5 | N/I |
| 262 | Y-Aib-EGTFTSDYSILLDKK((2-[2-(2- Amino-ethoxy)-ethoxy]-acetyl)2-(γ-Glu)-CO- (CH2)18- CO2H)AQTAFIEYLIEGGPSSGAPPPS-NH2 | 271 | 4879.5 | N/I |
| 263 | Y-Aib-EGTFTSDYSILLDKK((2-[2-(2- Amino-ethoxy)-ethoxy]-acetyl)2-(γ-Glu)-CO- (CH2)18- CO2H)AQSAFIEYLIEGGPSSGAPPPS-NH2 | 272 | 4865.5 | N/I |
| 264 | Y-Aib-EGT-αMeF(2F)-TSD-4Pal-SI-αMeL- LD-Orn-K((2-[2-(2-Amino-ethoxy)-ethoxy]- acetyl)2-(γ-Glu)-CO-(CH2)16-CO2H)AQ-Aib- EFI-(D-Glu)-αMeY-LIEGGPSSG-NH2 | 273 | 4475.0 | N/I |
| 265 | Y-Aib-EGT-αMeF(2F)-TSD-4Pal-SI-αMeL- LD-Orn-K((2-[2-(2-Amino-ethoxy)-ethoxy]- acetyl)2-(γ-Glu)-CO-(CH2)16-CO2H)AQ-Aib- EFI-(D-Glu)-αMeY-LIEGG-NH2 | 274 | 4146.7 | N/I |
| 266 | Y-Aib-EGTFTSDYSILLDKK((2-[2-(2- Amino-ethoxy)-ethoxy]-acetyl)2-(γ-Glu)-CO- (CH2)16-CO2H)AQ-Aib-AFIEYLIEGGPSSG- NH2 | 275 | 4385.94 | 4386.6 |
| 267 | Y-Aib-EGTFTSDYSILLDKK((2-[2-(2- Amino-ethoxy)-ethoxy]-acetyl)₂-(γ-Glu)-CO- (CH₂)₁₆-CO₂H)AQ-Aib-AFIEYLIEGG-NH₂ | 276 | 4057.62 | N/I |
| 268 | Y-Aib-EGTFTSDYSILLDEK((2-[2-(2- Amino-ethoxy)-ethoxy]-acetyl)2-(γ-Glu)-CO- (CH2)16-CO2H)AQ-Aib-AFIEYLIEGGPSSG- NH2 | 277 | 4386.88 | N/I |
| 269 | Y-Aib-EGTFTSDYSILLDEK((2-[2-(2- Amino-ethoxy)-ethoxy]-acetyl)2-(γ-Glu)-CO- (CH2)16-CO2H)AQ-Aib-AFIEYLIEGG-NH2 | 278 | 4058.56 | N/I |
| 270 | Y-Aib-EGTFTSDYSILLDKK((2-[2-(2- Amino-ethoxy)-ethoxy]-acetyl)₂-(γ-Glu)-CO- (CH₂)₁₆-CO₂H)AQ-Aib-EFIEYLIEGGPSSG- NH₂ | 279 | 4443.98 | N/I |
| 271 | Y-Aib-EGTFTSDYSILLDKK((2-[2-(2- Amino-ethoxy)-ethoxy]-acetyl)2-(γ-Glu)-CO- (CH2)16-CO2H)AQ-Aib-EFIEYLIEGG-NH2 | 280 | 4115.66 | N/I |
| 272 | Y-Aib-EGTFTSDYSILLDKK((2-[2-(2- Amino-ethoxy)-ethoxy]-acetyl)2-(γ-Glu)-CO- (CH2)16-CO2H)AQ-Aib-AFIEYLIAGGPSSG- NH2 | 281 | 4327.91 | 4327.8 |
| 273 | Y-Aib-EGTFTSDYSILLDKK((2-[2-(2- Amino-ethoxy)-ethoxy]-acetyl)2-(γ-Glu)-CO- (CH2)16-CO2H)AQ-Aib-AFIEYLIAGG-NH2 | 282 | 3999.58 | N/I |
| 274 | Y-Aib-EGTFTSDYSILLDKK((2-[2-(2- Amino-ethoxy)-ethoxy]-acetyl)2-(γ-Glu)-CO- (CH2)16-CO2H)AQPAFIEYLIEGGPSSG- NH2 | 283 | 4397.95 | N/I |
| 275 | Y-Aib-EGTFTSDYSILLDKK((2-[2-(2- Amino-ethoxy)-ethoxy]-acetyl)2-(γ-Glu)-CO- (CH2)16-CO2H)AQPAFIEYLIEGG-NH2 | 284 | 4069.63 | N/I |
| 276 | Y-Aib-EGT-αMeF(2F)-TSDYSI-αMeL- LDEKAQ-Aib-EFI-(D-Glu)- YLIEGGPSSGAPPPS-NH2 | 285 | 4224.59 | N/I |
| 277 | Y-Aib-EGT-αMeF(2F)-TSDYSI-αMeL- LDEQAQ-Aib-EFI-(D-Glu)- YLIEGGPSSGAPPPS-NH2 | 286 | 4224.55 | N/I |
| 278 | Y-Aib-EGT-αMeF(2F)-TSDYSI-αMeL- LDEKAQ-Aib-EFI-(D-Glu)- YLIEGGPSSGAPPPSC-NH2 | 287 | 4327.74 | N/I |
| 279 | Y-Aib-EGT-αMeF(2F)-TSDYSI-αMeL- LDEQAQ-Aib-EFI-(D-Glu)- YLIEGGPSSGAPPPSC-NH2 | 288 | 4327.69 | N/I |
| 280 | Y-Aib-EGT-αMeF(2F)-TSDYSI-αMeL-LD- Orn-KAQ-Aib-EFI-(D-Glu)- YLIEGGPSSGAPPPS-NH2 | 289 | 4210.61 | N/I |
| 281 | Y-Aib-EGT-αMeF(2F)-TSDYSI-αMeL-LD- Orn-QAQ-Aib-EFI-(D-Glu)- YLIEGGPSSGAPPPS-NH2 | 290 | 4209.58 | N/I |
| 282 | Y-Aib-EGT-αMeF(2F)-TSDYSI-αMeL-LD- Orn-KAQ-Aib-EFI-(D-Glu)- YLIEGGPSSGAPPPSC-NH2 | 291 | 4312.77 | N/I |
| 283 | Y-Aib-EGT-αMeF(2F)-TSDYSI-αMeL-LD- Orn-QAQ-Aib-EFI-(D-Glu)- YLIEGGPSSGAPPPSC-NH2 | 292 | 4312.73 | N/I |
| 284 | Y-Aib-EGT-αMeF(2F)-TSD-4Pal-SI-αMeL- LD-Orn-KAQ-Aib-EFI-(D-Glu)-αMeY- LIEGGPSSGAPPPS-NH2 | 293 | 4208.64 | N/I |
| 285 | Y-Aib-EGT-αMeF(2F)-TSD-4Pal-SI-αMeL- LD-Orn-QAQ-Aib-EFI-(D-Glu)-αMeY- LIEGGPSSGAPPPS-NH2 | 294 | 4208.6 | N/I |
| 286 | Y-Aib-EGT-αMeF(2F)-TSD-4Pal-SI-αMeL- LD-Orn-KAQ-Aib-EFI-(D-Glu)-αMeY- LIEGGPSSGAPPPSC-NH2 | 295 | 4311.78 | N/I |
| 287 | Y-Aib-EGT-αMeF(2F)-TSD-4Pal-SI-αMeL- LD-Orn-QAQ-Aib-EFI-(D-Glu)-αMeY- LIEGGPSSGAPPPSC-NH2 | 296 | 4311.74 | N/I |

| | | | | |
|---|---|---|---|---|
| N/I means Not Included | | | | |

### Example 288

Y-Aib-EGTFTSDYSILLDKK((2-[2-(2-Amino-ethoxy)-ethoxy]-acetyl)2-(γ-Glu)-CO-
(CH2)18-CO2H)AQ-Aib-AFIEYLIAGGPSSGAPPPS-NH2 (SEQ ID NO:303)

The structure of SEQ ID NO:303 is depicted below using the standard single letter amino acid codes with the exception of residues Aib2, K17, Aib20, and Ser39, where the structures of these amino acid residues have been expanded: 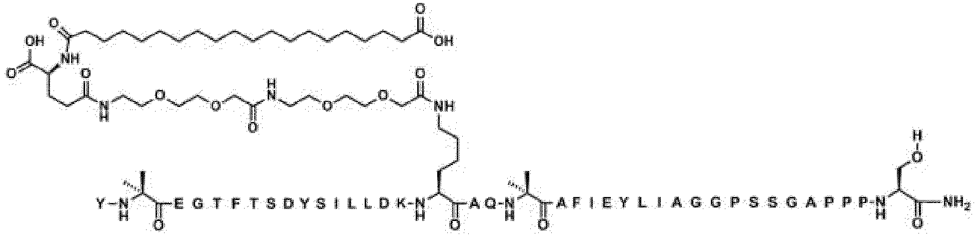 The compound according to SEQ ID NO:303 is prepared substantially as described by the procedures of Example 1. The molecular weight is determined by LC- MS (obsd: M+3 =1602.5; Calc M+3 =1602.8).

### Example 289

Y-Aib-EGTFTSDYSI-αMeL-LDKK((2-[2-(2-Amino-ethoxy)-ethoxy]-acetyl)2-(γ-Glu)-
CO-(CH2)18-CO2H)AQ-Aib-EFIEYLIAGGPSSGAPPPS-NH2 (SEQ ID NO:304)

The structure of SEQ ID NO:304 is depicted below using the standard single letter amino acid codes with the exception of residues Aib2, αMeL13, K17, Aib20, and Ser39, where the structures of these amino acid residues have been expanded: 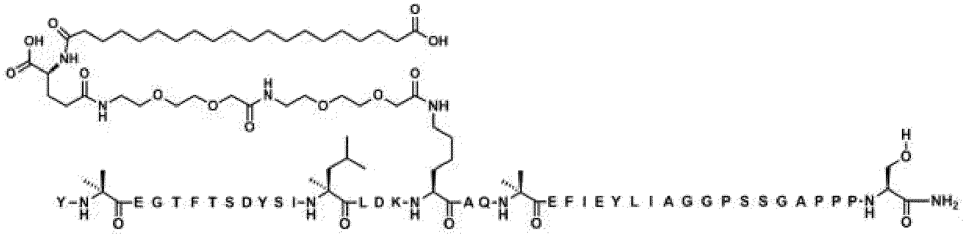 The compound according to SEQ ID NO:304 is prepared substantially as described by the procedures of Example 1. The molecular weight is determined by LC- MS (obsd: M+3 =1626.8; Calc M+3 =1626.8).

### Example 290

(D-Tyr)-Aib-EGTFTSDYSI-αMeL-LDKK((2-[2-(2-Amino-ethoxy)-ethoxy]-acetyl)₂-(γ-
Glu)-CO-(CH₂)₁₈-CO₂H)AQ-Aib-EFIEYLIAGGPSSGAPPPS-NH₂ (SEQ ID NO:305)

The structure of SEQ ID NO:305 is depicted below using the standard single letter amino acid codes with the exception of residues D-Tyr1, Aib2, αMeL13, K17, Aib20, and Ser39, where the structures of these amino acid residues have been expanded: 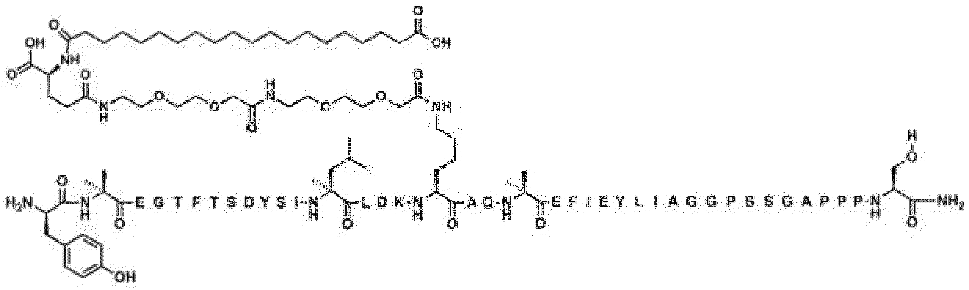 The compound according to SEQ ID NO:305 is prepared substantially as described by the procedures of Example 1. The molecular weight is determined by LC- MS (obsd: M+3 =1626.6; Calc M+3 =1626.8).

### Example 291

(D-Tyr)-Aib-EGTFTSDYSI-αMeL-LD-Orn-K((2-[2-(2-Amino-ethoxy)-ethoxy]-acetyl)2-
(γ-Glu)-CO-(CH2)18-CO2H)AQ-Aib-AFI-(D-Glu)-YLIAGGPSSGAPPPS-NH2 (SEQ ID
NO:306)

The structure of SEQ ID NO:306 is depicted below using the standard single letter amino acid codes with the exception of residues D-Tyr1, Aib2, αMeL13, Orn16, K17, Aib20, D-Glu24, and Ser39, where the structures of these amino acid residues have been expanded: 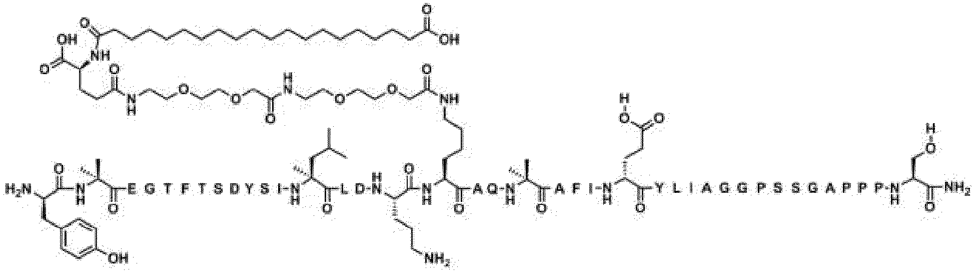 The compound according to SEQ ID NO:306 is prepared substantially as described by the procedures of Example 1. The molecular weight is determined by LC- MS (obsd: M+3 =1602.4; Calc M+3 =1602.8).

### Example 292

(D-Tyr)-Aib-EGTFTSDYSI-αMeL-LDKK((2-[2-(2-Amino-ethoxy)-ethoxy]-acetyl)₂-(γ-
Glu)-CO-(CH₂)₁₈-CO₂H)AQ-Aib-EFIE-αMeY-LIAGGPSSGAPPPS-NH₂ (SEQ ID
NO:307)

The structure of SEQ ID NO:307 is depicted below using the standard single letter amino acid codes with the exception of residues D-Tyr1, Aib2, αMeL13, K17, Aib20, αMeY25, and Ser39, where the structures of these amino acid residues have been expanded: 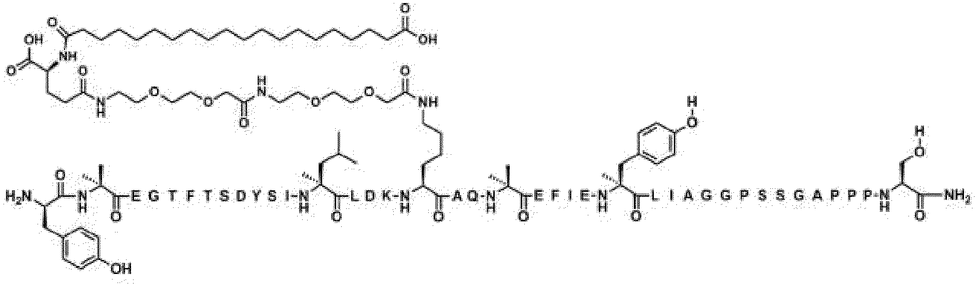 The compound according to SEQ ID NO:307 is prepared substantially as described by the procedures of Example 1. The molecular weight is determined by LC- MS (obsd: M+3 =1631.3; Calc M+3 =1631.5).

### Example 293

(D-Tyr)-Aib-EGTFTSDYSI-αMeL-LD-Orn-K((2-[2-(2-Amino-ethoxy)-ethoxy]-acetyl)2-
(γ-Glu)-CO-(CH2)18-CO2H)AQ-Aib-EFIE-αMeY-LIAGGPSSGAPPPS-NH2
(SEQ ID NO:308)

The structure of SEQ ID NO:308 is depicted below using the standard single letter amino acid codes with the exception of residues D-Tyr1, Aib2, αMeL13, Orn16, K17, Aib20, αMeY25, and Ser39, where the structures of these amino acid residues have been expanded: 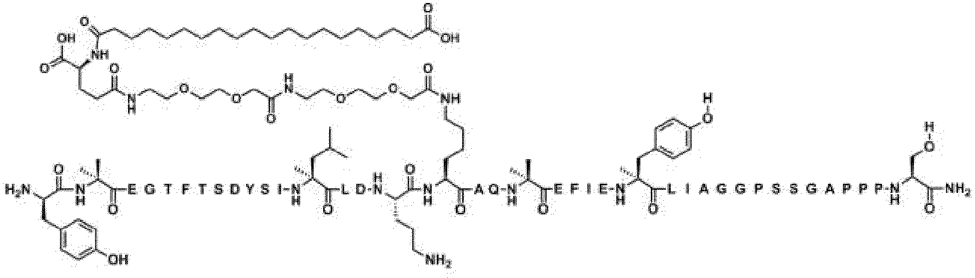 The compound according to SEQ ID NO:308 is prepared substantially as described by the procedures of Example 1. The molecular weight is determined by LC- MS (obsd: M+3 =1626.5; Calc M+3 =1626.8).

### Example 294 through Example 381

The compounds according to Examples 294 (SEQ ID NO:309) through Example 381 (SEQ ID NO:396) are prepared substantially as described by the procedures of Example 1.

| Example | Compound Name | SEQ ID NO | Calculated MW (average) | Found MW (average) |
|---|---|---|---|---|
| 294 | Y-Aib-EGTFTSDYSILLDKK((2-[2-(2- Amino-ethoxy)-ethoxy]-acetyl)2-(γ-Glu)-CO- (CH2)16-CO2H)AQQAFIEYLIEGG-NH2 | 309 | 4100.6 | N/I |
| 295 | Y-Aib-EGTFTSDYSILLDKK((2-[2-(2- Amino-ethoxy)-ethoxy]-acetyl)2-(γ-Glu)-CO- (CH2)16-CO2H)AQHAFIEYLIEGG-NH2 | 310 | 4109.7 | N/I |
| 296 | Y-Aib-EGTFTSDYSILLDKK((2-[2-(2- Amino-ethoxy)-ethoxy]-acetyl)2-(γ-Glu)-CO- (CH2)16-CO2H)AQKAFIEYLIEGGPSSG- NH2 | 311 | 4429.0 | N/I |
| 297 | Y-Aib-EGTFTSDYSILLDKK((2-[2-(2- Amino-ethoxy)-ethoxy]-acetyl)2-(γ-Glu)-CO- (CH2)16-CO2H)AQ-Aib-EFIEYLIAGG-NH2 | 312 | 4057.6 | N/I |
| 298 | Y-Aib-EGTFTSDYSILLDKK((2-[2-(2- Amino-ethoxy)-ethoxy]-acetyl)2-(γ-Glu)-CO- (CH2)16-CO2H)AQ-Aib- AFVEYLIEGGPSSG-NH2 | 313 | 4313.9 | N/I |
| 299 | Y-Aib-EGTFTSDYSILLDKK((2-[2-(2- Amino-ethoxy)-ethoxy]-acetyl)2-(γ-Glu)-CO- (CH2)16-CO2H)AQ-Aib- AFLEYLIEGGPSSG-NH2 | 314 | 4385.9 | N/I |
| 300 | Y-Aib-EGTFTSDYSI-αMeL-LDKK((2-[2- (2-Amino-ethoxy)-ethoxy]-acetyl)2-(γ-Glu)- CO-(CH2)16-CO2H)AQ-Aib- AFIEYLIEGGPSSG-NH2 | 315 | 4400.0 | N/I |
| 301 | Y-Aib-EGT-αMeF-TSDYSILLDKK((2-[2- (2-Amino-ethoxy)-ethoxy]-acetyl)2-(γ-Glu)- CO-(CH2)16-CO2H)AQ-Aib- AFIEYLIEGGPSSG-NH2 | 316 | 4400.0 | 4399.2 |
| 302 | Y-Aib-EGT-αMeF(2F)-TSDYSILLDKK((2- [2-(2-Amino-ethoxy)-ethoxy]-acetyl)2-(γ- Glu)-CO-(CH2)16-CO2H)AQ-Aib- AFIEYLIEGGPSSG-NH2 | 317 | 4418.0 | N/I |
| 303 | Y-Aib-EGTFTSDYSI-αMeL-LDEK((2-[2- (2-Amino-ethoxy)-ethoxy]-acetyl)2-(γ-Glu)- CO-(CH2)16-CO2H)AQ-Aib- AFIEYLIEGGPSSG-NH2 | 318 | 4400.9 | 4400.7 |
| 304 | Y-Aib-EGTFTSDYSI-αMeL-LDKK((2-[2- (2-Amino-ethoxy)-ethoxy]-acetyl)2-(γ-Glu)- CO-(CH2)16-CO2H)AQ-Aib- EFIEYLIEGGPSSG-NH2 | 319 | 4458.0 | N/I |
| 305 | Y-Aib-EGTFTSDYSI-αMeL-LDKK((2-[2- (2-Amino-ethoxy)-ethoxy]-acetyl)2-(γ-Glu)- CO-(CH2)16-CO2H)AQ-Aib- AFIEYLIAGGPSSG-NH2 | 320 | 4341.9 | N/I |
| 306 | Y-Aib-EGTFTSDYSI-αMeL-LDKK((2-[2- (2-Amino-ethoxy)-ethoxy]-acetyl)2-(γ-Glu)- CO-(CH2)16-CO2H)AQ-Aib- EFIEYLIAGGPSSG-NH2 | 321 | 4400.0 | N/I |
| 307 | Y-Aib-EGT-αMeF-TSDYSILLDEK((2-[2- (2-Amino-ethoxy)-ethoxy]-acetyl)2-(γ-Glu)- CO-(CH2)16-CO2H)AQ-Aib- AFIEYLIEGGPSSG-NH2 | 322 | 4400.9 | N/I |
| 308 | Y-Aib-EGT-αMeF-TSDYSILLDKK((2-[2- (2-Amino-ethoxy)-ethoxy]-acetyl)2-(γ-Glu)- CO-(CH2)16-CO2H)AQ-Aib- EFIEYLIEGGPSSG-NH2 | 323 | 4458.0 | N/I |
| 309 | Y-Aib-EGT-αMeF-TSDYSILLDKK((2-[2- (2-Amino-ethoxy)-ethoxy]-acetyl)2-(γ-Glu)- CO-(CH2)16-CO2H)AQ-Aib- AFIEYLIAGGPSSG-NH2 | 324 | 4341.9 | N/I |
| 310 | Y-Aib-EGT-αMeF-TSDYSILLDKK((2-[2- (2-Amino-ethoxy)-ethoxy]-acetyl)2-(γ-Glu)- CO-(CH2)16-CO2H)AQ-Aib- EFIEYLIAGGPSSG-NH2 | 325 | 4400.0 | N/I |
| 311 | Y-Aib-EGT-αMeF(2F)-TSDYSILLDEK((2- [2-(2-Amino-ethoxy)-ethoxy]-acetyl)2-(γ- Glu)-CO-(CH2)16-CO2H)AQ-Aib- AFIEYLIEGGPSSG-NH2 | 326 | 4418.9 | N/I |
| 312 | Y-Aib-EGT-αMeF(2F)-TSDYSILLDKK((2- [2-(2-Amino-ethoxy)-ethoxy]-acetyl)2-(γ- Glu)-CO-(CH2)16-CO2H)AQ-Aib- EFIEYLIEGGPSSG-NH2 | 327 | 4476.0 | N/I |
| 313 | Y-Aib-EGT-αMeF(2F)-TSDYSILLDKK((2- [2-(2-Amino-ethoxy)-ethoxy]-acetyl)2-(γ- Glu)-CO-(CH2)16-CO2H)AQ-Aib- AFIEYLIAGGPSSG-NH2 | 328 | 4359.9 | N/I |
| 314 | Y-Aib-EGT-αMeF(2F)-TSDYSILLDKK((2- [2-(2-Amino-ethoxy)-ethoxy]-acetyl)2-(γ- Glu)-CO-(CH2)16-CO2H)AQ-Aib- EFIEYLIAGGPSSG-NH2 | 329 | 4418.0 | N/I |
| 315 | Y-Aib-EGTFTSDYSILLDKK((2-[2-(2- Amino-ethoxy)-ethoxy]-acetyl)2-(γ-Glu)-CO- (CH2)16-CO2H)AQ-Aib- AFIEYLIEGGPSSGAPPPS-NH2 | 330 | 4835.5 | N/I |
| 316 | Y-Aib-EGTFTSDYSILLDKK((2-[2-(2- Amino-ethoxy)-ethoxy]-acetyl)2-(γ-Glu)-CO- (CH2)16-CO2H)AQ-Aib- AFIEYLIAGGPSSGAPPPS-NH2 | 331 | 4777.4 | 4777.2 |
| 317 | Y-Aib-EGTFTSDYSILLDKK((2-[2-(2- Amino-ethoxy)-ethoxy]-acetyl)2-(γ-Glu)-CO- (CH2)18-CO2H)AQ-Aib-AFIEYLIAGGPSSG- NH2 | 332 | 4356.0 | N/I |
| 318 | Y-Aib-EGTFTSDYSILLDKK((2-[2-(2- Amino-ethoxy)-ethoxy]-acetyl)2-(γ-Glu)-CO- (CH2)18-CO2H)AQ-Aib-EFIEYLIAGGPSSG- NH2 | 333 | 4414.0 | N/I |
| 319 | Y-Aib-EGTFTSDYSILLDKK((2-[2-(2- Amino-ethoxy)-ethoxy]-acetyl)2-(γ-Glu)-CO- (CH2)18-CO2H)AQ-Aib- EFIEYLIAGGPSSGAPPPS-NH2 | 334 | 4863.5 | N/I |
| 320 | Y-Aib-EGTFTSDYSILLDKK((2-[2-(2- Amino-ethoxy)-ethoxy]-acetyl)₂-(γ-Glu)-CO- (CH₂)₁₆-CO₂H)AQ-Aib-DFIEYLIEGGPSSG- NH₂ | 335 | 4430.0 | N/I |
| 321 | Y-Aib-EGTFTSDYSILLDKK((2-[2-(2- Amino-ethoxy)-ethoxy]-acetyl)2-(γ-Glu)-CO- (CH2)16-CO2H)AQ-Aib-TFIEYLIEGGPSSG- NH2 | 336 | 4416.0 | N/I |
| 322 | Y-Aib-EGTFTSDYSILLDKK((2-[2-(2- Amino-ethoxy)-ethoxy]-acetyl)₂-(γ-Glu)-CO- (CH₂)₁₆-CO₂H)AQ-Aib-HFIEYLIEGGPSSG- NH₂ | 337 | 4452.0 | N/I |
| 323 | Y-Aib-EGTFTSDYSI-αMeL-LDEK((2-[2- (2-Amino-ethoxy)-ethoxy]-acetyl)2-(γ-Glu)- CO-(CH2)16-CO2H)AQ-Aib- AFIEYLIEGGPSSGAPPPS-NH2 | 338 | 4850.4 | N/I |
| 324 | Y-Aib-EGTFTSDYSI-αMeL-LDKK((2-[2- (2-Amino-ethoxy)-ethoxy]-acetyl)2-(γ-Glu)- CO-(CH2)18-CO2H)AQ-Aib- AFIEYLIAGGPSSGAPPPS-NH2 | 339 | 4819.5 | N/I |
| 325 | Y-Aib-EGT-αMeF(2F)-TSDYSILLDKK((2- [2-(2-Amino-ethoxy)-ethoxy]-acetyl)2-(γ- Glu)-CO-(CH2)18-CO2H)AQ-Aib- AFIEYLIEGGPSSGAPPPS-NH2 | 340 | 4895.5 | N/I |
| 326 | F-Aib-EGTFTSDYSILLDKK((2-[2-(2- Amino-ethoxy)-ethoxy]-acetyl)2-(γ-Glu)-CO- (CH2)18-CO2H)AQ-Aib- AFIEYLIEGGPSSGAPPPS-NH2 | 341 | 4847.5 | N/I |
| 327 | F-Aib-EGTFTSDYSI-αMeL-LDKK((2-[2-(2- Amino-ethoxy)-ethoxy]-acetyl)₂-(γ-Glu)-CO- (CH₂)₁₈-CO₂H)AQ-Aib- AFIEYLIEGGPSSGAPPPS-NH₂ | 342 | 4861.5 | N/I |
| 328 | F-Aib-EGTFTSDYSI-αMeL-LDKK((2-[2-(2- Amino-ethoxy)-ethoxy]-acetyl)2-(γ-Glu)-CO- (CH2)16-CO2H)AQ-Aib- AFIEYLIEAGPSSGAPPPS-NH2 | 343 | 4847.5 | N/I |
| 329 | (D-Tyr)-Aib-EGT-αMeF- TSDYSILLDKK((2-[2-(2-Amino-ethoxy)- ethoxy]-acetyl)2-(γ-Glu)-CO-(CH2)18- CO2H)AQ-Aib-AFIEYLIEGGPSSGAPPPS- NH2 | 344 | 4877.5 | N/I |
| 330 | Y-Aib-EGTFTSDYSILLDEK((2-[2-(2- Amino-ethoxy)-ethoxy]-acetyl)2-(γ-Glu)-CO- (CH2)18-CO2H)AQ-Aib- AFIEYLIAGGPSSGAPPPS-NH2 | 345 | 4806.4 | 4805.4 |
| 331 | Y-Aib-EGTFTSDYSI-αMeL-LDEK((2-[2- (2-Amino-ethoxy)-ethoxy]-acetyl)₂-(γ-Glu)- CO-(CH₂)₁₈-CO₂H)AQ-Aib- EFIEYLIAGGPSSGAPPPS-NH₂ | 346 | 4878.5 | N/I |
| 332 | Y-Aib-EGTFTSDYSI-αMeL-LDEK((2-[2- (2-Amino-ethoxy)-ethoxy]-acetyl)2-(γ-Glu)- CO-(CH2)18-CO2H)AQ-Aib- AFIEYLIAGGPSSGAPPPS-NH2 | 347 | 4820.4 | N/I |
| 333 | Y-Aib-EGTFTSDYSI-αMeL-LDKK((2-[2- (2-Amino-ethoxy)-ethoxy]-acetyl)2-(γ-Glu)- CO-(CH2)18-CO2H)AQ-Aib- EFIEYLIEGGPSSGAPPPS-NH2 | 348 | 4935.6 | N/I |
| 334 | (D-Tyr)-Aib-EGTFTSDYSI-αMeL- LDKK((2-[2-(2-Amino-ethoxy)-ethoxy]- acetyl)₂-(γ-Glu)-CO-(CH₂)₁₈-CO₂H)AQ-Aib- AFIEYLIEGGPSSGAPPPS-NH₂ | 349 | 4877.5 | N/I |
| 335 | (D-Tyr)-Aib-EGT-αMeF-TSDYSI-αMeL- LDKK((2-[2-(2-Amino-ethoxy)-ethoxy]- acetyl)2-(γ-Glu)-CO-(CH2)18-CO2H)AQ-Aib- AFIEYLIEGGPSSGAPPPS-NH2 | 350 | 4891.6 | N/I |
| 336 | (D-Tyr)-Aib-EGT-αMeF(2F)-TSDYSI- αMeL-LDKK((2-[2-(2-Amino-ethoxy)- ethoxy]-acetyl)₂-(γ-Glu)-CO-(CH₂)₁₈- CO₂H)AQ-Aib-AFIEYLIEGGPSSGAPPPS- NH₂ | 351 | 4909.5 | N/I |
| 337 | F-Aib-EGTFTSDYSI-αMeL-LDKK((2-[2-(2- Amino-ethoxy)-ethoxy]-acetyl)2-(γ-Glu)-CO- (CH2)18-CO2H)AQ-Aib- AFIEYLIAGGPSSGAPPPS-NH2 | 352 | 4803.5 | N/I |
| 338 | F-Aib-EGTFTSDYSI-αMeL-LDKK((2-[2-(2- Amino-ethoxy)-ethoxy]-acetyl)₂-(γ-Glu)-CO- (CH₂)₁₈-CO₂H)AQ-Aib- EFIEYLIAGGPSSGAPPPS-NH₂ | 353 | 4861.5 | N/I |
| 339 | Y-Aib-EGTFTSDYSI-αMeL-LDKK((2-[2- (2-Amino-ethoxy)-ethoxy]-acetyl)-(γ-Glu)- CO-(CH2)18-CO2H)AQ-Aib- EFIEYLIAGGPSSGAPPPS-NH2 | 354 | 4732.4 | 4732.2 |
| 340 | (D-Tyr)-Aib-EGTFTSDYSI-αMeL- LDKK((2-[2-(2-Amino-ethoxy)-ethoxy]- acetyl)2-(γ-Glu)-CO-(CH2)18-CO2H)AQ-Aib- AFIEYLIAGGPSSGAPPPS-NH2 | 355 | 4819.5 | 4818.8 |
| 341 | (D-Tyr)-Aib-EGTFTSDYSI-αMeL- LDEK((2-[2-(2-Amino-ethoxy)-ethoxy]- acetyl)₂-(γ-Glu)-CO-(CH₂)₁₈-CO₂H)AQ-Aib- AFIEYLIAGGPSSGAPPPS-NH₂ | 356 | 4820.4 | N/I |
| 342 | (D-Tyr)-Aib-EGTFTSDYSI-αMeL- LDEK((2-[2-(2-Amino-ethoxy)-ethoxy]- acetyl)₂-(γ-Glu)-CO-(CH₂)₁₈-CO₂H)AQ-Aib- EFIEYLIAGGPSSGAPPPS-NH₂ | 357 | 4878.5 | N/I |
| 343 | Y-Aib-EGTFTSDYSI-αMeL-LDEK((2-[2- (2-Amino-ethoxy)-ethoxy]-acetyl)2-(γ-Glu)- CO-(CH2)18-CO2H)AQ-Aib-AFI-(D-Glu)- YLIAGGPSSGAPPPS-NH2 | 358 | 4820.4 | N/I |
| 344 | Y-Aib-EGTFTSDYSI-αMeL-LDEK((2-[2- (2-Amino-ethoxy)-ethoxy]-acetyl)2-(γ-Glu)- CO-(CH2)18-CO2H)AQ-Aib- DFIEYLIAGGPSSGAPPPS-NH2 | 359 | 4864.4 | N/I |
| 345 | (D-Tyr)-Aib-EGT-αMeF-TSDYSI-αMeL- LDKK((2-[2-(2-Amino-ethoxy)-ethoxy]- acetyl)2-(γ-Glu)-CO-(CH2)18-CO2H)AQ-Aib- EFIEYLIAGGPSSGAPPPS-NH2 | 360 | 4891.6 | N/I |
| 346 | (D-Tyr)-Aib-EGTFTSDYSI-αMeL-LD-Orn- K((2-[2-(2-Amino-ethoxy)-ethoxy]-acetyl)2- (γ-Glu)-CO-(CH2)18-CO2H)AQ-Aib- AFIEYLIAGGPSSGAPPPS-NH2 | 361 | 4805.5 | 4804.8 |
| 347 | (D-Tyr)-Aib-EGTFTSDYSI-αMeL-LD-Dab- K((2-[2-(2-Amino-ethoxy)-ethoxy]-acetyl)2- (γ-Glu)-CO-(CH2)18-CO2H)AQ-Aib- AFIEYLIAGGPSSGAPPPS-NH2 | 362 | 4791.4 | N/I |
| 348 | (D-Tyr)-Aib-EGTFTSDYSI-αMeL-LD-Dap- K((2-[2-(2-Amino-ethoxy)-ethoxy]-acetyl)2- (γ-Glu)-CO-(CH2)18-CO2H)AQ-Aib- AFIEYLIAGGPSSGAPPPS-NH2 | 363 | 4807.5 | N/I |
| 349 | (D-Tyr)-Aib-EGTFTSDYSI-αMeL-LD-Orn- K((2-[2-(2-Amino-ethoxy)-ethoxy]-acetyl)₂- (γ-Glu)-CO-(CH₂)₁₈-CO₂H)AQ-Aib- EFIEYLIAGGPSSGAPPPS-NH₂ | 364 | 4863.5 | 4862.7 |
| 350 | (D-Tyr)-Aib-EGTFTSDYSI-αMeL-LD-Dab- K((2-[2-(2-Amino-ethoxy)-ethoxy]-acetyl)2- (γ-Glu)-CO-(CH2)18-CO2H)AQ-Aib- EFIEYLIAGGPSSGAPPPS-NH2 | 365 | 4849.5 | N/I |
| 351 | (D-Tyr)-Aib-EGTFTSDYSI-αMeL-LD-Dap- K((2-[2-(2-Amino-ethoxy)-ethoxy]-acetyl)2- (γ-Glu)-CO-(CH2)18-CO2H)AQ-Aib- EFIEYLIAGGPSSGAPPPS-NH2 | 366 | 4835.5 | N/I |
| 352 | (D-Tyr)-Aib-EGTFTSDYSI-αMeL- LDKK((2-[2-(2-Amino-ethoxy)-ethoxy]- acetyl)₂-(γ-Glu)-CO-(CH₂)₁₈-CO₂H)AQ-Aib- AFI-(D-Glu)-YLIAGGPSSGAPPPS-NH₂ | 367 | 4819.5 | N/I |
| 353 | (D-Tyr)-Aib-EGTFTSDYSI-αMeL- LDKK((2-[2-(2-Amino-ethoxy)-ethoxy]- acetyl)2-(γ-Glu)-CO-(CH2)18-CO2H)AQ-Aib- EFI-(D-Glu)-YLIEGGPSSGAPPPS-NH2 | 368 | 4935.6 | N/I |
| 354 | (D-Tyr)-Aib-EGTFTSDYSI-αMeL-LD-Orn- K((2-[2-(2-Amino-ethoxy)-ethoxy]-acetyl)2- (γ-Glu)-CO-(CH2)18-CO2H)AQ-Aib-EFI-(D- Glu)-YLIAGGPSSGAPPPS-NH2 | 369 | 4863.5 | N/I |
| 355 | (D-Tyr)-Aib-EGTFTSDYSI-αMeL-LD-Orn- K((2-[2-(2-Amino-ethoxy)-ethoxy]-acetyl)2- (γ-Glu)-CO-(CH2)18-CO2H)AQ-Aib-EFI-(D- Glu)-YLIEGGPSSGAPPPS-NH2 | 370 | 4921.5 | N/I |
| 356 | (D-Tyr)-Aib-EGTFTSDYSI-αMeL- LDKK((2-[2-(2-Amino-ethoxy)-ethoxy]- acetyl)2-(γ-Glu)-CO-(CH2)18-CO2H)AQ-Aib- AFI-(D-Glu)-YLIEGGPSSGAPPPS-NH2 | 371 | 4877.5 | N/I |
| 357 | (D-Tyr)-Aib-EGTFTSDYSI-αMeL-LD-Orn- K((2-[2-(2-Amino-ethoxy)-ethoxy]-acetyl)2- (γ-Glu)-CO-(CH2)18-CO2H)AQ-Aib-AFI-(D- Glu)-YLIEGGPSSGAPPPS-NH2 | 372 | 4863.5 | N/I |
| 358 | (D-Tyr)-Aib-EGTFTSDYSI-αMeL-LD-Orn- K((2-[2-(2-Amino-ethoxy)-ethoxy]-acetyl)₂- (γ-Glu)-CO-(CH₂)₁₈-CO₂H)AQ-Aib- AFVEYLIAGGPSSGAPPPS-NH₂ | 373 | 4791.4 | N/I |
| 359 | (D-Tyr)-Aib-EGTFTSDYSI-αMeL-LD-Orn- K((2-[2-(2-Amino-ethoxy)-ethoxy]-acetyl)2- (γ-Glu)-CO-(CH2)18-CO2H)AQ-Aib- EFVEYLIAGGPSSGAPPPS-NH2 | 374 | 4849.5 | N/I |
| 360 | (D-Tyr)-Aib-EGTFTSDYSI-αMeL-LD-Orn- K((2-[2-(2-Amino-ethoxy)-ethoxy]-acetyl)₂- (γ-Glu)-CO-(CH₂)₁₈-CO₂H)AQ-Aib- AFVEYLIEGGPSSGAPPPS-NH₂ | 375 | 4849.5 | N/I |
| 361 | (D-Tyr)-Aib-EGTFTSDYSI-αMeL- LDKK((2-[2-(2-Amino-ethoxy)-ethoxy]- acetyl)2-(γ-Glu)-CO-(CH2)18-CO2H)AQ-Aib- AFV-(D-Glu)-YLIAGGPSSGAPPPS-NH2 | 376 | 4805.5 | N/I |
| 362 | (D-Tyr)-Aib-EGTFTSDYSI-αMeL-LD-Orn- K((2-[2-(2-Amino-ethoxy)-ethoxy]-acetyl)2- (γ-Glu)-CO-(CH2)18-CO2H)AQ-Aib-AFV-(D- Glu)-YLIAGGPSSGAPPPS-NH2 | 377 | 4791.4 | N/I |
| 363 | (D-Tyr)-Aib-EGTFTSDYSI-αMeL-LD-Orn- K((2-[2-(2-Amino-ethoxy)-ethoxy]-acetyl)2- (γ-Glu)-CO-(CH2)16-CO2H)AQ-Aib-AFI-(D- Glu)-YLIAGGPSSGAPPPS-NH2 | 378 | 4777.4 | N/I |
| 364 | (D-Tyr)-Aib-EGTFTSDYSI-αMeL-LD-Orn- K((2-[2-(2-Amino-ethoxy)-ethoxy]-acetyl)2- (γ-Glu)-CO-(CH2)16-CO2H)AQ-Aib-AFV-(D- Glu)-YLIAGGPSSGAPPPS-NH2 | 379 | 4763.4 | N/I |
| 365 | (D-Tyr)-Aib-EGTFTSDYSI-αMeL- LDKK((2-[2-(2-Amino-ethoxy)-ethoxy]- acetyl)₂-(γ-Glu)-CO-(CH₂)₁₈-CO₂H)AQ-Aib- AFIE-αMeY-LIAGGPSSGAPPPS-NH₂ | 380 | 4833.5 | 4832.4 |
| 366 | (D-Tyr)-Aib-EGTFTSDYSI-αMeL-LD-Orn- K((2-[2-(2-Amino-ethoxy)-ethoxy]-acetyl)₂- (γ-Glu)-CO-(CH₂)₁₈-CO₂H)AQ-Aib-AFIE- αMeY-LIAGGPSSGAPPPS-NH₂ | 381 | 4819.5 | 4818.3 |
| 367 | Y-Aib-EGTFTSDYSI-αMeL-LDKK((2-[2- (2-Amino-ethoxy)-ethoxy]-acetyl)2-(γ-Glu)- CO-(CH2)18-CO2H)AQ-Aib-EFIE-αMeY- LIAGGPSSGAPPPS-NH2 | 382 | 4891.6 | N/I |
| 368 | (D-Tyr)-Aib-EGTFTSDYSI-αMeL-LD-Orn- K((2-[2-(2-Amino-ethoxy)-ethoxy]-acetyl)₂- (γ-Glu)-CO-(CH₂)₁₆-CO₂H)AQ-Aib- EFIEYLIAGGPSSGAPPPS-NH₂ | 383 | 4835.5 | N/I |
| 369 | (D-Tyr)-Aib-EGTFTSDYSI-αMeL-LD-Orn- K((2-[2-(2-Amino-ethoxy)-ethoxy]-acetyl)2- (γ-Glu)-CO-(CH2)18-CO2H)AQ-Aib- DFIEYLIAGGPSSGAPPPS-NH2 | 384 | 4849.5 | N/I |
| 370 | (D-Tyr)-Aib-EGTFTSDYSI-αMeL-LD-Orn- K((2-[2-(2-Amino-ethoxy)-ethoxy]-acetyl)2- (γ-Glu)-CO-(CH2)18-CO2H)AQ-Aib- EFIEYLIAGGPSSG-NH2 | 385 | 4414.0 | N/I |
| 371 | (D-Tyr)-Aib-EGTFTSDYSI-αMeL-LD-Orn- K((2-[2-(2-Amino-ethoxy)-ethoxy]-acetyl)-(γ- Glu)-CO-(CH2)18-CO2H)AQ-Aib- EFIEYLIAGGPSSGAPPPS-NH2 | 386 | 4718.3 | N/I |
| 372 | (D-Tyr)-Aib-EGTFTSDYSI-αMeL- LDKK((2-[2-(2-Amino-ethoxy)-ethoxy]- acetyl)-(γ-Glu)-CO-(CH2)18-CO2H)AQ-Aib- EFIE-αMeY-LIAGGPSSGAPPPS-NH2 | 387 | 4746.4 | N/I |
| 373 | (D-Tyr)-Aib-EGTFTSDYSI-αMeL- LDKK((2-[2-(2-Amino-ethoxy)-ethoxy]- acetyl)-(γ-Glu)-CO-(CH2)18-CO2H)AQ-Aib- AFIE-αMeY-LIAGGPSSGAPPPS-NH2 | 388 | 4688.3 | N/I |
| 374 | (D-Tyr)-Aib-EGTFTSDYSI-αMeL- LDKK((2-[2-(2-Amino-ethoxy)-ethoxy]- acetyl)2-(γ-Glu)-CO-(CH2)16-CO2H)AQ-Aib- EFIE-αMeY-LIAGGPSSGAPPPS-NH2 | 389 | 4863.5 | N/I |
| 375 | (D-Tyr)-Aib-EGTFTSDYSI-αMeL-LD-Orn- K((2-[2-(2-Amino-ethoxy)-ethoxy]-acetyl)₂- (γ-Glu)-CO-(CH₂)₁₆-CO₂H)AQ-Aib-EFIE- αMeY-LIAGGPSSGAPPPS-NH₂ | 390 | 4849.5 | N/I |
| 376 | (D-Tyr)-Aib-EGTFTSDYSI-αMeL- LDKK((2-[2-(2-Amino-ethoxy)-ethoxy]- acetyl)2-(γ-Glu)-CO-(CH2)16-CO2H)AQ-Aib- AFIE-αMeY-LIAGGPSSGAPPPS-NH2 | 391 | 4805.5 | N/I |
| 377 | (D-Tyr)-Aib-EGTFTSDYSI-αMeL-LD-Orn- K((2-[2-(2-Amino-ethoxy)-ethoxy]-acetyl)₂- (γ-Glu)-CO-(CH₂)₁₆-CO₂H)AQ-Aib-AFIE- αMeY-LIAGGPSSGAPPPS-NH₂ | 392 | 4791.4 | 4790.7 |
| 378 | (D-Tyr)-Aib-EGTFTSDYSI-αMeL- LDKK((2-[2-(2-Amino-ethoxy)-ethoxy]- acetyl)-(γ-Glu)-CO-(CH2)18-CO2H)AQ-Aib- EFIEYLIAGGPSSGAPPPS-NH2 | 393 | 4732.4 | N/I |
| 379 | (D-Tyr)-Aib-EGTFTSDYSI-αMeL- LDKK((2-[2-(2-Amino-ethoxy)-ethoxy]- acetyl)2-(γ-Glu)-CO-(CH2)18-CO2H)AQ-Aib- EFIE-αMeY-LIEGGPSSGAPPPS-NH2 | 394 | 4949.5 | N/I |
| 380 | (D-Tyr)-Aib-EGTFTSDYSI-αMeL-LD-Orn- K((2-[2-(2-Amino-ethoxy)-ethoxy]-acetyl)₂- (γ-Glu)-CO-(CH₂)₁₈-CO₂H)AQ-Aib-EFIE- αMeY-LIEGGPSSGAPPPS-NH₂ | 395 | 4935.5 | N/I |
| 381 | (D-Tyr)-Aib-EGTFTSDYSI-αMeL- LDEK((2-[2-(2-Amino-ethoxy)-ethoxy]- acetyl)2-(γ-Glu)-CO-(CH2)18-CO2H)AQ-Aib- AFIE-αMeY-LIAGGPSSGAPPPS-NH2 | 396 | 4934.4 | N/I |

| | | | | |
|---|---|---|---|---|
| N/I means Not Included | | | | |

### Binding Assays

Glucagon (referred to as Gcg) is a Reference Standard prepared at Eli Lilly and Company. GLP-1, 7-36-NH₂ (referred to as GLP-1) is obtained from CPC Scientific (Sunnyvale, CA, 97.2% purity, 100 µM aliquots in 100% DMSO). GIP 1-42 (referred to as GIP) is prepared at Lilly Research Laboratories using peptide synthesis and HPLC chromatography as described above (>80% purity, 100 µM aliquots in 100% DMSO). [¹²⁵I]-radiolabeled Gcg, GLP-1, or GIP is prepared using [¹²⁵I]-lactoperoxidase and obtained from Perkin Elmer (Boston, MA).

Stably transfected cell lines are prepared by subcloning receptor cDNA into a pcDNA3 expression plasmid and transfected into human embryonic kidney (HEK) 293 (hGcgR and hGLP-1R) or Chinese Hamster Ovary (CHO) (hGIPR) cells followed by selection with Geneticin (hGLP-1R and hGIPR) or hygromycin B (hGcgR).

Two methods are used for the preparation of crude cell membranes.

Method 1: Frozen cell pellets are lysed on ice in hypotonic buffer containing 50 mM Tris HCl, pH 7.5, and Roche Complete^{™} Protease Inhibitors with EDTA. The cell suspension is disrupted using a glass Potter-Elvehjem homogenizer fitted with a Teflon^{®} pestle for 25 strokes. The homogenate is centrifuged at 4°C at 1100 x g for 10 minutes. The supernatant is collected and stored on ice while the pellets are resuspended in homogenization buffer and rehomogenized as described above. The homogenate is centrifuged at 1100 x g for 10 minutes. The second supernatant is combined with the first supernatant and centrifuged at 35000 x g for 1 hour at 4°C. The resulting membrane pellet is resuspended in homogenization buffer containing protease inhibitors at approximately 1 to 3 mg/mL, quick frozen in liquid nitrogen and stored as aliquots in a -80°C freezer until use.

Method 2: Frozen cell pellets are lysed on ice in hypotonic buffer containing 50 mM Tris HCl, pH 7.5, 1 mM MgCl₂, Roche Complete^{™} EDTA-free Protease Inhibitors and 25 units/ml DNAse I (Invitrogen). The cell suspension is disrupted using a glass Potter-Elvehjem homogenizer fitted with a Teflon^{®} pestle for 20 to 25 strokes. The homogenate is centrifuged at 4°C at 1800 x g for 15 minutes. The supernatant is collected and stored on ice while the pellets are resuspended in homogenization buffer (without DNAse I) and rehomogenized as described above. The homogenate is centrifuged at 1800 x g for 15 minutes. The second supernatant is combined with the first supernatant and centrifuged an additional time at 1800 x g for 15 minutes. The overall supernatant is then centrifuged at 25000 x g for 30 minutes at 4°C. The resulting membrane pellet is resuspended in homogenization buffer (without DNAse I) containing protease inhibitors at approximately 1 to 3 mg/mL and stored as aliquots in a -80°C freezer until use.

### Binding Determination Methods

The equilibrium binding dissociation constants (K_{d}) for the various receptor/radioligand interactions are determined from homologous competition binding analysis instead of saturation binding due to high propanol content in the [¹²⁵I] stock material. The K_{d} values determined for the receptor preparations were as follows: hGcgR (3.9 nM), hGLP-1R (1.2 nM) and hGIPR (0.14 nM).

### [¹²⁵I]-Glucagon Binding

The human Gcg receptor binding assays are performed using a Scintillation Proximity Assay (SPA) format with wheat germ agglutinin (WGA) beads (Perkin Elmer). The binding buffer contains 25 mM 4-(2-hydroxyethyl)-1-piperazineethanesulfonic acid (HEPES), pH 7.4, 2.5 mM CaCl₂, 1 mM MgCl₂, 0.1% (w/v) bacitracin (Research Products), 0.003% (w/v) Polyoxyethylenesorbitan monolaurate (TWEEN^{®}-20), and Roche Complete^{™} Protease Inhibitors without EDTA. Peptides and Gcg are thawed and 3-fold serially diluted in 100% DMSO (10 point concentration response curves). Next, 5 µL serially diluted compound or DMSO is transferred into Corning^{®} 3632 clear bottom assay plates containing 45 µL assay binding buffer or unlabeled Gcg control (non-specific binding or NSB, at 1 µM final). Then, 50 µL [¹²⁵I]-Gcg (0.15 nM final), 50 µL human GcgR membranes (1.5 µg/well) and 50 µL of WGA SPA beads (80 to 150 µg/well) are added with a Biotek Multiflo dispenser. Plates are sealed and mixed on a plate shaker (setting 6) for 1 minute and read with a PerkinElmer Trilux MicroBeta^{®} scintillation counter after 12 hours of incubation/settling time at room temperature. Final assay concentration ranges for peptides tested in response curves is typically 1150 nM to 0.058 nM and for the control Gcg from 1000 nM to 0.05 nM.

### [¹²⁵I]-GLP-1 Binding

The human GLP-1 receptor binding assay is performed using an SPA format with WGA beads. The binding buffer contains 25 mM HEPES, pH 7.4, 2.5 mM CaCl₂, 1 mM MgCl₂, 0.1% (w/v) bacitracin, 0.003% (w/v) TWEEN^{®}-20, and Roche Complete^{™} Protease Inhibitors without EDTA. Peptides and GLP-1 are thawed and 3-fold serially diluted in 100% DMSO (10 point concentration response curves). Next, 5 µL serially diluted compound or DMSO is transferred into Corning^{®} 3632 clear bottom assay plates containing 45 µL assay binding buffer or unlabeled GLP-1 control (non-specific binding or NSB, at 0.25 µM final). Then, 50 µL [¹²⁵I]-GLP-1 (0.15 nM final), 50 µL human GLP-1R membranes (0.5 µg/well and 50 µL of WGA SPA beads (100 to 150 µg/well) are added with a Biotek Multiflo dispenser. Plates are sealed and mixed on a plate shaker (setting 6) for 1 minute and read with a PerkinElmer Trilux MicroBeta^{®} scintillation counter after 5 to 12 hours of incubation/settling time at room temperature. Final assay concentration ranges for peptides tested in response curves are typically 1150 nM to 0.058 nM and for the control GLP-1, 250 nM to 0.013 nM.

### [125I]-GIP Binding

The human GIP receptor binding assay is performed using an SPA format with WGA beads. The binding buffer contains 25 mM HEPES, pH 7.4, 2.5 mM CaCl₂, 1 mM MgCl₂, 0.1% (w/v) bacitracin, 0.003% (w/v) TWEEN^{®}-20, and Roche Complete^{™} Protease Inhibitors without EDTA. Peptides and GIP are thawed and 3 fold serially diluted in 100% DMSO (10 point concentration response curves). Next, 5 µL serially diluted compound or DMSO is transferred into Corning^{®} 3632 clear bottom assay plates containing 45 µL assay binding buffer or unlabeled GIP control (non-specific binding or NSB, at 0.25 µM final). Then, 50 µL [¹²⁵I]-GIP (0.075-0.15 nM final), 50 µL human GIPR membranes (3 µg/well) and 50 µL of WGA SPA beads (100 to 150 µg/well) are added with a Biotek Multiflo dispenser. Plates are sealed and mixed on a plate shaker (setting 6) for 1 minute and read with a PerkinElmer Trilux MicroBeta^{®} scintillation counter after 2.5 to 12 hours of incubation/settling time at room temperature. Final assay concentration ranges for peptides tested in response curves is typically 1150 to 0.058 nM or 115 nM to 0.0058 nM and for the control GIP, 250 nM to 0.013 nM.

### Binding Assay Data Analysis

Raw CPM data for concentration curves of peptides, Gcg, GLP-1, or GIP are converted to percent inhibition by subtracting nonspecific binding (binding in the presence of excess unlabeled Gcg, GLP-1, or GIP, respectively) from the individual CPM values and dividing by the total binding signal, also corrected by subtracting nonspecific binding. Data are analyzed using four-parameter (curve maximum, curve minimum, IC₅₀, Hill slope) nonlinear regression routines (Genedata Screener, version 12.0.4, Genedata AG, Basal, Switzerland). The affinity constant (Kᵢ) is calculated from the absolute IC₅₀ value based upon the equation Kᵢ = IC₅₀/(1 + D/K_{d}) where D is the concentration of radioligand used in the experiment, IC₅₀ is the concentration causing 50% inhibition of binding and K_{d} is the equilibrium binding dissociation constant of the radioligand (described above). Values for Kᵢ are reported as the geometric mean, with error expressed as the standard error of the mean (SEM) and n is equal to the number of independent replicates (determined in assays performed on different days). Geometric Means are calculated as follows:$Geometric Mean = 10 \left(Arithmetic, , Mean, , of, , Log, , Ki, , Values\right) )$ The Ki Ratio (Ki for native control peptide/Ki for test compound) at each receptor and each species is calculated. The Ki Ratio is a rapid indication of the apparent affinity of a peptide compared to the native control peptide. A Ki Ratio < 1 indicates that the test peptide has a lower affinity (higher Ki value) for the receptor than the native peptide, whereas a Ki Ratio >1 indicates that the test peptide has a higher affinity (lower Ki value) for the receptor than the native peptide.

n=1/x means that only one value out of the total number of replicates (x) is used to express the mean. SEM is only calculated when n=2 or greater non-qualified results exist. Means are expressed as GeoMetric means with the standard error of the mean (SEM) and the number of replicates (n) indicated in parenthesis.

**Table 1. In vitro Binding Affinity (Kᵢ) of indicated Examples and comparator molecules for human GLP-1R, GcgR and GIPR.**

| **Example or comparator** | hGLcgR Ki, nM (SEM, n) | hGIPR Ki, nM (SEM, n) | hGLP1R Ki, (nM) (SEM, n) |
|---|---|---|---|
| hGcg | 3.65 (0.26, n=10) | | |
| hGIP amide | | 0.0922 (0.0085, n=11) | |
| hGLP-1 amide | | | 0.614 (0.066, n=12) |
| 1 | 207 (13.8, n=5) | 0.0546 (0.0120, n=5) | 6.67 (1.25, n=6) |
| 2 | 361 (55.1, n=5) | 0.0600 (0.0150, n=5) | 2.35 (0.220, n=5) |
| 3 | 242 (56.2, n=6) | 0.0458 (0.00357, n=6) | 2.23 (0.366, n=6) |
| 4 | 686 (n=1/5) | 0.0528 (0.00647, n=5) | 1.63 (0.260, n=5) |
| 5 | 519 (109, n=4) | 0.0611 (0.00592, n=4) | 0.902 (0.114, n=4) |
| 6 | 55.8 (10.2, n=2) | 0.0835 (0.00437, n=2) | 6.71 (1.25, n=2) |
| 7 | 198 | 0.252 | 43.3 |
| 8 | 206 (25.7, n=2) | 0.0772 (0.0155, n=2) | 2.84 (0.753, n=2) |
| 9 | 375 (87.5, n=2) | 0.127 (0.0118, n=2) | 14.9 (2.15, n=2) |
| 10 | 226 (67.4, n=2) | 0.109 (0.0927, n=2) | 9.33 (1.49, n=2) |
| 11 | 174 (25.3, n=2) | 0.226 (0.0728, n=2) | 15.7 (4.37, n=2) |
| 12 | 684 (141, n=2) | 0.167 (0.0853, n=2) | 12.9 (2.71, n=2) |
| 13 | >1060 (n=1/2) | 0.296 (0.0291, n=2) | 31.1 (11.9, n=2) |
| 14 | 160 | 0.0494 | 29.6 |
| 15 | 130 | 0.284 | 2.19 |
| 16 | 371 | 0.0841 | 2.78 |
| 17 | 261 (115, n=2) | 0.606 (0.363, n=2) | 7.63 (2.47, n=2) |
| 18 | 50.1 | 0.0798 | 0.319 |
| 19 | 60.5 | 0.0518 | 0.24 |
| 20 | 228 (65.3, n=2) | 0.0849 (0.0168, n=2) | 3.30 (1.01, n=2) |
| 21 | 149 | 0.529 | 14.5 |
| 22 | 53.4 | 0.624 | 23.1 |
| 23 | >1010 | 0.258 | 6.32 |
| 24 | 49.8 | 0.232 | 5.04 |
| 25 | 81.1 | 0.179 | 4.8 |
| 26 | >960 | 0.176 | 4.22 |
| 27 | 315 | 0.103 | 3.68 |
| 28 | >902 | 0.24 | 21.1 |
| 29 | 132 | 0.377 | 8 |
| 30 | 123 | 0.151 | 6.2 |
| 31 | 290 | 0.0275 | 6.58 |
| 32 | 44.7 | 0.0205 | 3.96 |
| 33 | >979 | 6.4 | 361 |
| 34 | 134 | 0.0467 | 3.41 |
| 35 | >964 | 0.0358 | 54.6 |
| 36 | 413 | 0.141 | 16.4 |
| 37 | 255 | 0.0523 | 3.84 |
| 38 | >974 | 0.104 | 31.3 |
| 39 | 161 | 0.0499 | 16.8 |
| 40 | 150 | 0.0345 | 7.56 |
| 41 | 165 | 0.0551 | 13.4 |
| 42 | 160 | 0.0514 | 13.2 |
| 43 | 134 | 0.101 | 11.8 |
| 44 | 121 | 0.0516 | 10.6 |
| 45 | 11.1 | 0.0463 | 5.65 |
| 46 | 133 | 0.0852 | 13.4 |
| 48 | 111 | 0.074 | 15.7 |
| 49 | 236 | 0.087 | 12.3 |
| 50 | 220 (61.2, n=2) | 0.0568 (0.00744, n=2) | 4.71 (1.22, n=2) |
| 51 | 195 (65.9, n=2) | 0.0620 (0.0131, n=2) | 5.62 (0.658, n=2) |
| 52 | >1100 | 0.0342 | 5.81 |
| 53 | 216 | 0.188 | 1.23 |
| 54 | 333 | 0.965 | 1.66 |
| 55 | >1100 | 6.24 | 7.29 |
| 56 | >1060 | 0.148 | 10.3 |
| 57 | 26.1 (4.31, n=2) | 0.0583 (0.0131, n=2) | 3.00 (0.293, n=2) |
| 58 | 339 | 0.105 | 2.77 |
| 59 | 292 (11.8, n=2) | 0.136 (0.00422, n=2) | 8.20 (4.13, n=2) |
| 60 | 237 | 0.0655 | 9.55 |
| 61 | 110 | 0.102 | 11.1 |
| 62 | 168 | 0.0545 | 2.03 |
| 63 | 273 | 0.141 | 7.79 |
| 64 | 260 | 0.0866 | 4.86 |
| 65 | 194 | 0.0643 | 4.53 |
| 66 | 93.7 | 0.106 | 7.53 |
| 67 | 270 | 0.061 | 10.2 |
| 68 | 99.2 | 0.0243 | 1.58 |
| 69 | 22.1 (4.18, n=3) | 0.0300 (0.00657, n=3) | 1.22 (0.353, n=3) |
| 74 | 69.8 | 0.0279 | 5.99 |
| 75 | 283 | 0.103 | 24.4 |
| 76 | 14.4 | 0.0659 | 2.64 |
| 78 | 215 (66.2, n=3) | 0.163 (0.0356, n=2) | 3.94 (1.21, n=2) |
| 79 | 429 (n=1/2) | 0.0313 | 2.69 |
| 80 | 347 (n=1/2) | 0.0931 | 2.16 |
| 81 | 344 | 0.198 | 2.88 |
| 82 | >1060 | 14.9 | 6.82 |
| 83 | 320 | 0.142 | 7.1 |
| 84 | >1100 | 0.143 | 10.2 |
| 85 | >894 (n=1/2) | 0.621 | 1.87 |
| 86 | >1060 | 0.0401 | 3.74 |
| 87 | 278 (n=1/2) | 0.0340 (0.00150, n=2) | 1.79 (0.417, n=2) |
| 88 | 545 (57.8, n=2) | 0.0717 | 4.24 |
| 89 | 324 (22.9, n=2) | 0.045 | 2.64 |
| 90 | 245 (7.55, n=2) | 0.0472 | 4.76 |
| 91 | 540 | 1.8 | 5.23 |
| 93 | 15.7 | 0.0859 | 1.89 |
| 99 | 23.6 | 0.027 | 1.15 |
| 100 | 44 | 0.115 | 4.13 |
| 101 | 117 | 0.0953 | 8.1 |
| 103 | 40.3 | 0.0645 | 6.68 |
| 104 | 123 (17.1, n=5) | 0.0565 (0.0153, n=5) | 3.91 (0.955, n=5) |
| 105 | 20.4 | 0.119 | 0.871 |
| 106 | 515 | 0.179 | 1.2 |
| 107 | 303 | 0.0425 | 0.867 |
| 108 | 171 | 0.0732 | 3 |
| 109 | 43.1 | 0.0279 | 1.34 |
| 110 | 73.9 | 0.0395 | 4.38 |
| 115 | 9.89 | 0.0302 | 3.43 |
| 116 | 137 (13.9, n=2) | 0.0597 (0.0486, n=2) | 6.80 (1.85, n=2) |
| 117 | 192 (14.6, n=3) | 0.0497 (0.0111, n=3) | 6.96 (1.95, n=3) |
| 118 | 53.0 (7.07, n=3) | 0.0859 (0.00402, n=3) | 6.10 (0.870, n=3) |
| 119 | 30.6 | 0.0925 | 9.87 |
| 120 | 93.6 | 0.11 | 11.7 |
| 121 | 51.9 | 0.177 | 3.16 |
| 122 | 43.3 (8.07, n=2) | 0.190 (0.0189, n=2) | 3.36 (0.799, n=2) |
| 123 | 80.1 (11.7, n=6) | 0.0469 (0.00804, n=6) | 1.31 (0.197, n=6) |
| 124 | 41.5 (9.39, n=2) | 0.0424 (0.00200, n=2) | 4.87 (0.277, n=2) |
| 125 | 54.4 (0.365, n=2) | 0.0624 (0.0117, n=2) | 3.19 (0.123, n=2) |
| 126 | 101 (11.5, n=2) | 0.0644 (0.0267, n=2) | 1.46 (0.299, n=2) |
| 127 | 43.6 | 0.126 | 1.86 |
| 128 | 433 (203, n=2) | 0.0625 (0.0355, n=2) | 1.88 (0.296, n=2) |
| 129 | 14.9 | 0.0278 | 1 |
| 130 | >1060 | 0.177 | 3.66 |
| 133 | 216 | 0.157 | 11.4 (2.31, n=2) |
| 134 | 60.5 | 0.14 | 12.7 (0.947, n=2) |
| 135 | 454 | 0.161 | 3.01 |
| 137 | 98.1 (14.8, n=3) | 0.0373 (0.00200, n=3) | 1.24 (0.341, n=3) |
| 138 | 61.2 (4.65, n=2) | 0.0295 (0.00145, n=2) | 0.926 (0.201, n=2) |
| 139 | 105 (6.68, n=2) | 0.0360 (0.00446, n=2) | 1.25 (0.0904, n=2) |
| 140 | 175 (40.1, n=3) | 0.0474 (0.00461, n=3) | 1.46 (0.0630, n=3) |
| 142 | 53.1 (1.60, n=2) | 0.0275 (0.00210, n=2) | 1.06 (0.300, n=2) |
| 143 | 65.5 | 0.0304 | 1.15 |
| 144 | 77 | 0.0341 | 1.78 |
| 145 | 158 | 0.0652 | 2.22 |
| 147 | 64.9 (19.9, n=2) | 0.0981 (0.0285, n=2) | 4.47 (0.742, n=2) |
| 149 | 127 | 0.0708 | 26.1 |
| 150 | 63.2 | 0.0649 | 30.5 |
| 152 | 93.4 | 0.117 | 48 |
| 153 | 43.8 | 0.0578 | 22.2 |
| 154 | 762 (51.7, n=3) | 0.0610 (0.00457, n=3) | 5.64 (2.52, n=3) |
| 157 | 179 (82.9, n=3) | 0.0937 (0.0160, n=3) | 8.97 (2.28, n=3) |
| 158 | 285 (17.4, n=2) | 0.114 (0.0193, n=2) | 11.8 (5.32, n=2) |
| 160 | >1060 (n=1/2) | 5.98 (1.46, n=2) | 14.4 (3.72, n=2) |
| 163 | 117 | 0.116 | 10.8 |
| 181 | 413 (132, n=2) | 0.145 (0.0856, n=2) | 7.28 (0.798, n=2) |
| 182 | 565 (335, n=2) | 0.0669 (0.0311, n=2) | 4.64 (0.655, n=2) |
| 183 | 304 (128, n=2) | 0.0869 (0.0118, n=2) | 4.11 (0.369, n=2) |
| 189 | 146 (7.81, n=2) | 0.128 (0.0817, n=2) | 8.81 (0.434, n=2) |
| 191 | 348 (54.7, n=2) | 0.144 (0.0676, n=2) | 4.52 (1.95, n=2) |
| 192 | >1110 (n=1/2) | 0.118 (0.108, n=2) | 2.89 (0.516, n=2) |
| 202 | 394 | 0.0579 | 5.38 |
| 203 | 845 (n=1/2) | 0.0337 (0.00260, n=2) | 3.90 (1.10, n=2) |
| 204 | >1150 | 0.0704 | 1.9 |
| 205 | 438 | 0.0367 | 3.05 |
| 206 | 176 (126, n=2) | 0.0814 (0.00608, n=2) | 5.27 (0.359, n=2) |
| 207 | 74.2 | 0.0786 | 1.37 |
| 208 | >1060 | 0.0537 | 2.13 |
| 209 | >1060 (n=1/2) | 0.0664 (0.0267, n=2) | 1.43 (0.466, n=2) |
| 210 | >1010 | 0.0399 | 1.58 |
| 211 | 131 | 0.0243 | 2.64 |
| 212 | 205 (1.77, n=2) | 0.0978 (0.0730, n=2) | 2.76 (0.561, n=2) |
| 213 | 544 | 0.365 | 2.75 |
| 214 | 126 | 0.0304 | 1.99 |
| 215 | 75.2 | 0.0666 | 6.85 |
| 216 | 45.2 | 0.0559 | 2.34 |
| 217 | 516 | 0.0376 | 2.02 |
| 218 | 270 | 0.0593 | 2.54 |
| 219 | 373 | 0.0689 | 2.01 |
| 220 | 377 | 0.0919 | 2.71 |
| 221 | 154 (n=1/2) | 0.0414 (0.00291, n=2) | 1.77 (0.900, n=2) |
| 222 | 71.3 (11.9, n=2) | 0.0495 (0.0210, n=2) | 3.59 (0.660, n=2) |
| 223 | 46.5 | 0.0921 | 5.62 |
| 224 | 627 (267, n=2) | 0.0482 (0.0174, n=2) | 6.86 (1.85, n=2) |
| 225 | 714 (n=1/2) | 0.0622 (0.0208, n=2) | 8.79 (4.24, n=2) |
| 226 | 200 | 0.0254 | 4.1 |
| 227 | 113 | 0.0146 | 2.01 |
| 228 | 182 | 0.028 | 2.43 |
| 229 | >1100 | 2.47 | 36.2 |
| 230 | 494 | 0.042 | 4.68 |
| 231 | 440 | 0.0394 | 3.03 |
| 232 | >1150 | 0.0544 | 5.62 |
| 233 | >1150 | 0.0445 | 5.99 |
| 234 | >1100 | 0.0563 | 10.9 |
| 235 | >1200 | 0.0581 | 7.65 |
| 236 | 200 (15.1, n=2) | 0.0425 (0.00194, n=2) | 1.05 (0.173, n=2) |
| 237 | >1060 | 0.131 | 1.04 |
| 238 | 230 | 0.0403 | 0.548 |
| 239 | 596 (215, n=3) | 0.101 (0.0172, n=3) | 2.71 (0.0420, n=3) |
| 240 | 204 | 0.0284 | 0.552 |
| 241 | 167 (45.6, n=2) | 0.0420 (0.0118, n=2) | 0.799 (0.401, n=2) |
| 242 | 95.9 (14.1, n=2) | 0.0604 (0.00642, n=2) | 0.853 (0.0475, n=2) |
| 243 | 145 (5.05, n=2) | 0.0325 (0.00840, n=2) | 0.670 (0.0478, n=2) |
| 244 | 87.8 (2.39, n=2) | 0.0308 (0.0150, n=2) | 0.820 (0.141, n=2) |
| 246 | >1010 (n=1/3) | 0.0509 (0.0147, n=3) | 0.812 (0.0900, n=3) |
| 247 | >1100 | >55.1 | 4.39 |
| 248 | >1050 | 0.0397 | 2.4 |
| 249 | >1000 | 0.0394 | 2.35 |
| 250 | 198 | 0.0171 | 1.72 |
| 251 | 21.2 | 0.0249 | 1.09 |
| 252 | 26 | 0.00971 | 0.383 |
| 253 | >912 | 0.138 | 2.57 |
| 254 | 148 | 0.108 | 2.58 |
| 255 | 257 | 0.0772 | 2.58 |
| 264 | 388 | 0.015 | 0.412 |
| 265 | 567 | 0.0224 | 0.537 |
| 266 | 193 (28.2, n=2) | 0.0666 (0.0189, n=2) | 2.01 (0.256, n=2) |
| 267 | 349 (178, n=2) | 0.0628 (0.00765, n=2) | 1.57 (0.109, n=2) |
| 268 | >1190 | 0.0814 | 3.98 |
| 269 | >1100 | 0.152 | 7.1 |
| 270 | >1190 | 0.117 | 8.27 |
| 271 | >1150 | 0.107 | 5.09 |
| 272 | 550 (243, n=2) | 0.0353 (0.00276, n=2) | 1.22 (0.291, n=2) |
| 273 | 724 | 0.0698 | 1.13 |
| 288 | 345 (35.7, n=3) | 0.0580 (0.0105, n=3) | 1.60 (0.866, n=3) |
| 289 | >1050 (n=1/3) | 0.0457 (0.0220, n=3) | 2.63 (1.74, n=3) |
| 290 | 308 (n=1/3) | 0.0617 (0.0115, n=3) | 2.44 (0.162, n=3) |
| 291 | >872 (n=1/2) | 0.129 (0.0346, n=3) | 3.16 (0.270, n=3) |
| 292 | 595 | 0.0547 | 1.19 |
| 293 | 668 | 0.0775 | 1.64 |
| 294 | 629 | 0.205 | 2.92 |
| 295 | >1000 | 0.181 | 4.12 |
| 296 | >1000 | 0.444 | 3.33 |
| 297 | >1240 | 0.0958 | 2.98 |
| 298 | >1370 | 0.0578 | 3.03 |
| 299 | >1040 | 0.734 | 54.3 |
| | | | |
| 300 | 251 | 0.0504 | 2.13 |
| 301 | 44.4 (6.17, n=3) | 0.0273 (0.00127, n=3) | 0.875 (0.0889, n=3) |
| 302 | 18.5 | 0.0289 | 0.617 |
| 303 | 502 (189, n=3) | 0.0580 (0.0151, n=3) | 3.69 (1.86, n=3) |
| 304 | >855 | 0.0499 | 4.44 |
| 305 | 352 (30.8, n=2) | 0.0250 (0.00586, n=2) | 0.830 (0.481, n=2) |
| 306 | >1040 | 0.0349 | 2.86 |
| 307 | 117 | 0.0773 | 5.1 |
| 308 | 94.2 | 0.0288 | 1.01 |
| 309 | 64.1 | 0.0264 | 1.04 |
| 310 | 174 (8.97, n=2) | 0.0315 (0.00162, n=2) | 1.70 (0.144, n=2) |
| 311 | 115 | 0.0497 | 11.5 |
| 312 | 106 | 0.0348 | 1.63 |
| 313 | 27.6 | 0.0261 | 0.815 |
| 314 | 116 | 0.027 | 0.717 |
| 315 | 539 | 0.0677 | 2.28 |
| 316 | 654 (76.5, n=3) | 0.0418 (0.00224, n=3) | 0.957 (0.180, n=3) |
| 317 | 253 | 0.0215 | 2.63 |
| 318 | 730 (n=1/2) | 0.0452 (0.00883, n=2) | 7.52 (0.256, n=2) |
| 319 | >984 | 0.0349 | 3.61 |
| 320 | >1040 | 0.136 | 5.17 |
| 321 | 770 | 0.064 | 4.2 |
| 322 | 1030 | 0.175 | 2.31 |
| 323 | 300 | 0.0516 | 1.65 |
| 324 | 449 | 0.0278 | 0.609 |
| 325 | 13 | 0.0209 | 0.475 |
| 326 | 207 | 0.251 | 2.92 |
| 327 | 114 (8.53, n=2) | 0.0667 (0.0211, n=2) | 2.10 (0.287, n=2) |
| 328 | >1450 (n=1/2) | 0.136 (0.0602, n=2) | 3.98 (0.339, n=2) |
| 329 | 17.0 (2.51, n=2) | 0.0439 (0.0105, n=2) | 3.28 (0.327, n=2) |
| 330 | >1050 | 0.114 | 12.7 |
| 331 | >969 (n=1/2) | 0.0851 (0.00508, n=2) | 11.4 (0.160, n=2) |
| 332 | 397 (272, n=2) | 0.0497 (0.00681, n=2) | 7.87 (0.333, n=2) |
| 333 | 578 (68.8, n=2) | 0.0634 (0.00255, n=2) | 4.25 (0.180, n=2) |
| 334 | 192 | 0.0646 | 2.17 |
| 335 | 27.1 | 0.0444 | 2.54 |
| 336 | 17.1 | 0.0277 | 2.44 |
| 337 | 335 | 0.0363 | 1.61 |
| 338 | >1060 | 0.0831 | 3.23 |
| 339 | 873 (19.4, n=2) | 0.0388 (0.0198, n=2) | 2.69 (0.218, n=2) |
| 340 | 250 (60.8, n=2) | 0.0507 (0.0177, n=2) | 2.08 (0.0742, n=2) |
| 341 | 39.7 | 0.0559 | 6.49 |
| 342 | >1000 | 0.129 | 15.4 |
| 343 | >1070 | 0.0374 | 13.3 |
| 344 | >1080 | 0.0507 | 14.8 |
| | | | |
| 346 | 310 (26.0, n=3) | 0.0559 (0.0248, n=2) | 1.67 (0.911, n=2) |
| 349 | >1060 (n=1/3) | 0.0800 (0.0215, n=3) | 1.72 (0.0730, n=3) |
| 352 | >1030 (n=1/2) | 0.0726 (0.00687, n=3) | 3.03 (0.673, n=3) |
| 354 | >953 | 0.175 (0.0209, n=2) | 7.85 (0.190, n=2) |
| 356 | >1010 | 0.350 (0.0397, n=2) | 10.3 (1.53, n=2) |
| 357 | >977 | 0.316 (0.0233, n=2) | 7.02 (1.19, n=2) |
| 358 | 915 (n=1/2) | 0.0636 (0.00684, n=3) | 1.37 (0.189, n=3) |
| 359 | >982 (n=1/2) | 0.0874 (0.0159, n=3) | 2.59 (0.833, n=3) |
| 360 | 485 | 0.128 (0.00895, n=2) | 1.74 (0.0269, n=2) |
| 362 | >1050 | 0.337 (0.00484, n=2) | 6.95 (0.446, n=2) |
| 363 | >1020 (n=1/2) | 0.170 (0.0113, n=3) | 3.89 (0.864, n=3) |
| 364 | >1150 (n=1/2) | 0.672 (0.0431, n=3) | 17.2 (2.26, n=3) |
| 367 | 777 | 0.0282 | 0.809 |

### Functional Activity (with BSA)

Functional activity is determined in hGLP-1R, hGcgR and hGIP-R expressing HEK-293 clonal cell lines. Each receptor over-expressing cell line is treated with peptide (20 point CRC, 2.75-fold Labcyte Echo direct dilution) in DMEM (Gibco Cat# 31053) supplemented with 1X GlutaMAX^{™} supplement (L-alanyl-L-glutamine dipeptide Gibco^{®}), 0.25% FBS (Fetal Bovine Serum), 0.05% fraction V BSA (Bovine Serum Albumin), 250 µM 3-isobutyl-1-methylxanthine (IBMX) and 20 mM 4-(2-hydroxyethyl)-1-piperazineethanesulfonic acid (HEPES) in a 20 µl assay volume.

After 60-minute incubation at room temperature, the resulting increase in intracellular cAMP is quantitatively determined using the CisBio cAMP Dynamic 2 homogeneous time-resolved fluorescence (HTRF) Assay Kit. The cAMP levels within the cell are detected by adding the cAMP-d2 conjugate in cell lysis buffer followed by the antibody anti-cAMP-Eu³⁺-Cryptate, also in cell lysis buffer. The resulting competitive assay is incubated for at least 60 minutes at room temperature and then detected using an instrument with excitation at 320 nm and emission at 665 nm and 620 nm. Envision units (emission at 665nm/620nm*10,000) are inversely proportional to the amount of cAMP present and are converted to nM cAMP per well using a cAMP standard curve.

The amount of cAMP generated (nM) in each well is converted to a percent of the maximal response observed with either human GLP-1(7-36)NH₂, human Gcg, or human GIP(1-42)NH₂. A relative EC₅₀ value is derived by non-linear regression analysis using the percent maximal response vs. the concentration of peptide added, fitted to a four-parameter logistic equation.

EC₅₀ determination of human GLP-1(7-36)NH₂ at human GLP-1R, human Gcg at human GcgR, and human GIP(1-42)NH₂ at human GIP-R: the peptide concentration ranges were 448 pM to 99.5 nM. EC₅₀ determination of Examples at human GLP-1R, human GcgR, and human GIP-R: the peptide concentration ranges are 51.5 fM to 11.4 µM.

**Table 2. Functional cAMP Potency (EC₅₀) for Example and comparator peptides (hGcg, hGIP amide, and hGLP-1 amide) in the presence of FBS.**

| | **cAMP EC₅₀,nM (SEM, n)** | | |
|---|---|---|---|
| **Example or comparator** | GcgR | GIPR | GLP-1R |
| hGcg | 0.0125 ± 0.0011 (n=12) | | |
| hGIP amide | | 0.0979 (0.0088, n=12) | |
| hGLP-1 amide | | | 0.0424 (0.0043, n=12) |
| Example 1 | >11400 (n=1/9) | 38.5 (20.2, n=12) | 53.3 (21.4, n=12) |
| Example 2 | >10900 (n=1/12) | 2.64 (0.696, n=12) | 6.52 (1.76, n=11) |
| Example 3 | >10900 (n=1/7) | 9.24 (2.23, n=7) | 19.4 (7.02, n=7) |
| Example 4 | >10900 (n=1/5) | 1.03 (0.181, n=5) | 2.02 (0.596, n=4) |
| Example 5 | >10900 | 1.98 | 1.61 |

### cAMP Pharmacological Functional Assay in presence of casein

An additional set of cAMP assays are conducted in HEK293 cells expressing the human GLP-1 receptor (GLP-1R), gastric inhibitory peptide receptor (GIPR), Glucagon receptor (GcgR). Pharmacological activity of the hGLP1R/GIPR peptides are determined in HEK293 cells stably expressing the human GLP-1 receptor (GLP-1R), gastric inhibitory peptide receptor (GIPR), or GLP-2 receptor (GLP-2R). Each receptor over-expressing cell line (20 µl) is treated with the test peptide in DMEM (Gibco Cat# 31053) supplemented with 0.1% Casein (Sigma Cat# C4765), 250 µM IBMX, 1X GlutaMAXTM (Gibco Cat# 35050), and 20 mM HEPES (HyClone Cat# SH30237.01) in a 20 µl assay volume. After 60 minute incubation at room temperature, the resulting increase in intracellular cAMP is quantitatively determined using the CisBio cAMP Dynamic 2 HTRF Assay Kit (62AM4PEJ). The Lysis buffer containing cAMP-d2 conjugate (20 µl) and the antibody anti-cAMP-Eu3+-Cryptate (20 µl) are then added to determine the cAMP level. After 1h-incubation at room temperature, HTRF signal is detected with an Envision 2104 plate reader (PerkinElmer). Fluorescent emission at 620 nm and at 665 nm is measured and the ratio between 620 nm and at 665 nm is calculated and then are converted to nM cAMP per well using a cAMP standard curve. Dose response curves of compounds are plotted as the percentage of stimulation normalized to minimum (buffer only) and maximum (maximum concentration of each control ligand) values and analyzed using a four parameter non-liner regression fit with a variable slope (Genedata Screener 13). EC50 is the concentration of compound causing half-maximal simulation in a dose response curve. A relative EC₅₀ value is derived by non-linear regression analysis using the percent maximal response vs. the concentration of peptide added, fitted to a four-parameter logistic equation.

Using Homogeneous Time Resolved Fluorescence methods, assays are conducted to determine the intrinsic potency of Example and comparator molecules performed in the presence of casein (instead of serum albumin) as a nonspecific blocker, which does not interact with the fatty acid moieties of the analyzed molecules.
Intracellular cAMP levels are determined by extrapolation using a standard curve. Dose response curves of compounds are plotted as the percentage of stimulation normalized to minimum (buffer only) and maximum (maximum concentration of each control ligand) values and analyzed using a four parameter non-linear regression fit with a variable slope (Genedata Screener 13). EC₅₀ is the concentration of compound causing half-maximal simulation in a dose response curve. Each relative EC50 value for the Geometric mean calculation is determined from a curve fitting.

Concentration response curves of compounds are plotted as the percentage of stimulation normalized to minimum (buffer only) and maximum (maximum concentration of each control ligand) values and analyzed using a four parameter non-liner regression fit with a variable slope (Genedata Screener 13). EC50 is the concentration of compound causing half-maximal simulation in a dose response curve.
The EC₅₀ summary statistics are computed as follows:
Geometric mean:$GM = {10}^{∧} \left(arithmetic mean of {log}_{10} transformed {EC}_{50} values\right) .$
The standard error of the mean is reported: 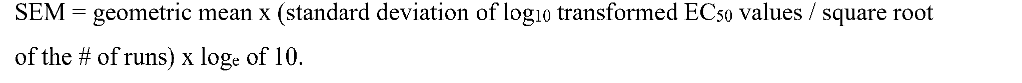 SEM = geometric mean x (standard deviation of log10 transformed EC50 values / square root of the # of runs) x loge of 10.
The log transform accounts for the EC₅₀ values falling on a multiplicative, rather than an arithmetic scale.

Each day, the assay is run, the test peptides are run plus the native ligands GIP and GLP-1, buffer only as baseline (minimum) and the highest concentration of the respective GIP and GLP-1 standard is used as maximum for calculations. For illustration, as shown by Example 1, the test peptide is tested in 8 runs of the assay. For avoidance of doubt, hGIP amide and hGLP-1 amide EC50 in Table 3 are illustrative of geometric mean values from a series of 18 assay values, and values will vary each day compared to the zero buffer. Accordingly, each Example will use the geometric mean of those values to normalize the Example assay runs.

**Table 3. Functional activation of hGLP-1R, hGIPR, hGcgR in the presence of 0.1% Casein.**

| **Example or comparator** | **hGIPR cAMP Rel EC₅₀ nM (SEM, n)** | **hGIPR cAMP EC₅₀ ratio (SEM, n)** | **hGLP1R cAMP Rel EC₅₀ nM (SEM, n)** | **hGLP1R cAMP EC₅₀ ratio (SEM, n)** |
|---|---|---|---|---|
| hGIP amide | 0.170 (0.012, n=18) | | | |
| hGLP-1 amide | | | 0.0396 (0.0030, n=16) | |
| 1 | 0.0356 (0.00576, n=8) | 4.65 (0.514, n=8) | 0.0410 (0.00720, n=7) | 1.12 (0.0949, n=7) |
| 2 | 0.0339 (0.00650, n=5) | 5.89 (1.10, n=5) | 0.0441 (0.00670, n=5) | 0.888 (0.0993, n=5) |
| 3 | 0.0411 (0.00541, n=5) | 4.51 (0.355, n=5) | 0.0338 (0.00156, n=4) | 1.25 (0.0916, n=4) |
| 4 | 0.0272 (0.00358, n=6) | 5.95 (0.466, n=6) | 0.0297 (0.00319, n=6) | 1.41 (0.241, n=6) |
| 5 | 0.0309 (0.00402, n=5) | 6.27 (0.808, n=5) | 0.0164 (0.00219, n=5) | 2.69 (0.547, n=5) |
| 6 | 0.0899 (0.0196, n=2) | 2.09 (0.301, n=2) | 0.374 (0.0100, n=2) | 0.185 (0.00340, n=2) |
| 7 | 0.461 | 0.317 | 0.470 (0.0988, n=2) | 0.130 (0.0215, n=2) |
| 8 | 0.0848 (0.00744, n=6) | 1.97 (0.170, n=6) | 0.148 (0.00926, n=6) | 0.419 (0.0352, n=6) |
| 9 | 0.210 (0.0335, n=6) | 0.768 (0.122, n=6) | 0.194 (0.0284, n=6) | 0.314 (0.0384, n=6) |
| 10 | 1.28 (0.270, n=2) | 0.151 (0.0416, n=2) | 7.64 (0.786, n=2) | 0.00912 (0.000859, n=2) |
| 11 | 0.486 (0.108, n=2) | 0.399 (0.114, n=2) | 6.89 (2.68, n=3) | 0.0111 (0.00387, n=3) |
| 12 | 0.300 (0.0827, n=2) | 0.659 (0.221, n=2) | 1.15 (0.00296, n=2) | 0.0603 (0.000359, n=2) |
| 13 | 1.05 (0.236, n=2) | 0.180 (0.0272, n=2) | 5.39 (1.35, n=2) | 0.0133 (0.00338, n=2) |
| 14 | 0.284 (0.0828, n=2) | 0.545 (0.161, n=2) | 1.87 (0.534, n=2) | 0.0306 (0.00519, n=2) |
| 15 | 0.613 (0.141, n=2) | 0.273 (0.00678, n=2) | 0.0336 (0.0000222, n=2) | 1.65 (0.0634, n=2) |
| 16 | 0.975 (0.241, n=2) | 0.157 (0.0401, n=2) | 0.0437 (0.00494, n=4) | 1.43 (0.260, n=3) |
| 17 | 5.81 (0.758, n=2) | 0.0257 (0.00368, n=2) | 0.152 (0.0166, n=2) | 0.398 (0.0260, n=2) |
| 18 | 0.610 (0.200, n=2) | 0.275 (0.0204, n=2) | 0.0945 (0.00823, n=2) | 0.589 (0.0732, n=2) |
| 19 | 0.386 (0.0583, n=2) | 0.436 (0.0449, n=2) | 0.104 (0.00342, n=2) | 0.532 (0.0375, n=2) |
| 20 | 0.0556 (0.00518, n=5) | 2.81 (0.293, n=5) | 0.117 (0.0121, n=8) | 0.577 (0.0706, n=8) |
| 21 | 0.0748 (0.00682, n=8) | 1.95 (0.145, n=8) | 0.160 (0.00495, n=7) | 0.402 (0.0178, n=7) |
| 22 | 0.0842 (0.0103, n=4) | 1.86 (0.109, n=4) | 0.206 (0.0172, n=5) | 0.290 (0.0233, n=5) |
| 23 | 0.204 (0.0160, n=5) | 0.754 (0.0348, n=5) | 0.190 (0.00766, n=7) | 0.342 (0.0255, n=7) |
| 24 | 0.762 (0.206, n=2) | 0.228 (0.0443, n=2) | 13.0 (n=1/2) | 0.00416 (n=1/2) |
| 25 | 0.230 (0.0191, n=6) | 0.636 (0.0564, n=6) | 0.356 (0.0415, n=6) | 0.197 (0.0287, n=6) |
| 26 | 0.251 (0.0264, n=6) | 0.585 (0.0531, n=6) | 0.293 (0.0442, n=5) | 0.238 (0.0309, n=5) |
| 27 | 0.0789 (0.00792, n=6) | 1.90 (0.295, n=6) | 0.557 (0.0717, n=5) | 0.123 (0.0102, n=5) |
| 28 | 1.66 (0.257, n=2) | 0.106 (0.0238, n=2) | 32.7 (n=1/2) | 0.00166 (n=1/2) |
| 29 | 0.320 (0.0548, n=2) | 0.536 (0.0522, n=2) | 0.999 (0.336, n=2) | 0.0671 (0.0283, n=2) |
| 30 | 0.114 (0.00650, n=2) | 1.50 (0.0246, n=2) | 1.84 (0.280, n=2) | 0.0331 (0.00127, n=2) |
| 31 | 0.388 (0.0773, n=2) | 0.456 (0.121, n=2) | 0.891 (0.147, n=2) | 0.0708 (0.0192, n=2) |
| 32 | 0.179 (0.0200, n=5) | 0.840 (0.0750, n=5) | 0.498 (0.0124, n=5) | 0.136 (0.00728, n=5) |
| 33 | 63.3 (6.65, n=2) | 0.00222 (0.000125, n=2) | 251 (n=1/2) | 0.000256 (n=1/2) |
| 34 | 0.360 (0.0200, n=2) | 0.393 (0.00105, n=2) | 1.56 (0.132, n=2) | 0.0392 (0.00119, n=2) |
| 35 | 0.0905 (0.00717, n=2) | 1.58 (0.215, n=2) | 8.38 (0.707, n=2) | 0.00728 (0.000221, n=2) |
| 36 | 0.309 (0.0126, n=2) | 0.458 (0.00789, n=2) | 2.29 (0.390, n=2) | 0.0268 (0.00309, n=2) |
| 37 | 0.269 (0.0546, n=2) | 0.532 (0.0765, n=2) | 1.15 (0.216, n=2) | 0.0533 (0.00707, n=2) |
| 38 | 0.147 (0.0217, n=2) | 0.967 (0.0861, n=2) | 2.82 (0.0832, n=2) | 0.0217 (0.00181, n=2) |
| 39 | 0.133 (0.0242, n=2) | 1.07 (0.132, n=2) | 1.92 (0.447, n=2) | 0.0323 (0.00571, n=2) |
| 40 | 0.136 (0.0164, n=2) | 1.06 (0.188, n=2) | 0.746 (0.118, n=2) | 0.0801 (0.0105, n=2) |
| 41 | 0.229 (0.0451, n=2) | 0.637 (0.159, n=2) | 0.960 (0.0203, n=2) | 0.0637 (0.00478, n=2) |
| 42 | 0.161 (0.00967, n=2) | 0.897 (0.149, n=2) | 1.45 (0.328, n=2) | 0.0471 (0.0107, n=2) |
| 43 | 0.112 (0.0129, n=2) | 1.28 (0.0105, n=2) | 0.862 (0.0963, n=2) | 0.0777 (0.00826, n=2) |
| 44 | 0.128 (0.0118, n=2) | 1.11 (0.0174, n=2) | 0.752 (0.201, n=2) | 0.0918 (0.0245, n=2) |
| 45 | 0.106 (0.0158, n=2) | 1.75 (0.164, n=2) | 1.03 (0.194, n=2) | 0.0569 (0.00591, n=2) |
| 46 | 0.172 (0.0231, n=2) | 0.828 (0.0222, n=2) | 0.687 (0.0147, n=2) | 0.0969 (0.00159, n=2) |
| 47 | 0.287 (0.0529, n=2) | 0.654 (0.0819, n=2) | 0.702 (0.197, n=2) | 0.0890 (0.0310, n=2) |
| 48 | 0.168 (0.0126, n=2) | 0.863 (0.156, n=2) | 0.691 (0.0491, n=2) | 0.0966 (0.00733, n=2) |
| 49 | 0.0868 (0.0288, n=2) | 1.69 (0.372, n=2) | 0.597 (0.0314, n=2) | 0.112 (0.00642, n=2) |
| 50 | 0.0794 (0.0104, n=5) | 1.96 (0.179, n=5) | 0.0961 (0.00519, n=7) | 0.675 (0.0492, n=7) |
| 51 | 0.0960 (0.0106, n=7) | 1.69 (0.201, n=7) | 0.153 (0.0110, n=7) | 0.426 (0.0376, n=7) |
| 52 | 0.0997 (0.0119, n=6) | 1.40 (0.154, n=6) | 0.132 (0.0143, n=5) | 0.514 (0.0413, n=5) |
| 53 | 0.628 (0.161, n=2) | 0.287 (0.0912, n=2) | 0.0339 (0.000266, n=2) | 1.80 (0.190, n=2) |
| 54 | 1.57 (0.144, n=2) | 0.110 (0.0181, n=2) | 0.0242 (0.00711, n=2) | 2.55 (0.456, n=2) |
| 55 | 5.45 (n=1/2) | <0.00159 | 0.0286 (0.000346, n=2) | 2.33 (0.0282, n=2) |
| 56 | 0.167 (0.0132, n=2) | 1.02 (0.00567, n=2) | 0.136 (0.00964, n=2) | 0.491 (0.0347, n=2) |
| 57 | 0.0849 (0.0107, n=6) | 1.74 (0.148, n=6) | 0.0668 (0.00654, n=7) | 0.996 (0.0730, n=7) |
| 58 | 0.266 (0.0307, n=5) | 0.522 (0.0439, n=5) | 0.180 (0.0151, n=5) | 0.378 (0.0347, n=5) |
| 59 | 0.0922 (0.0134, n=5) | 1.54 (0.186, n=5) | 0.0840 (0.00742, n=5) | 0.812 (0.0816, n=5) |
| 60 | 0.135 (0.0107, n=2) | 1.06 (0.0296, n=2) | 0.287 (0.0377, n=3) | 0.237 (0.0270, n=3) |
| 61 | 0.0739 (0.0140, n=2) | 1.94 (0.158, n=2) | 0.371 (0.0664, n=3) | 0.187 (0.0341, n=3) |
| 62 | 0.0601 (0.00469, n=5) | 2.33 (0.234, n=5) | 0.159 (0.0151, n=5) | 0.463 (0.0644, n=5) |
| 63 | 0.0925 (0.0106, n=7) | 1.58 (0.156, n=7) | 0.190 (0.0195, n=5) | 0.386 (0.0466, n=5) |
| 64 | 0.0916 (0.0104, n=6) | 1.57 (0.192, n=6) | 0.172 (0.0184, n=5) | 0.429 (0.0598, n=5) |
| 65 | 0.143 (0.0264, n=2) | 1.04 (0.295, n=2) | 0.289 (0.0215, n=3) | 0.233 (0.0144, n=3) |
| 66 | 0.0743 (0.00740, n=5) | 1.89 (0.209, n=5) | 0.285 (0.0291, n=5) | 0.255 (0.0245, n=5) |
| 67 | 0.0913 (0.00577, n=2) | 1.61 (0.0648, n=2) | 0.447 (0.0958, n=2) | 0.123 (0.0314, n=2) |
| 68 | 0.0881 (0.00725, n=2) | 1.67 (0.0990, n=2) | 0.153 (0.0169, n=2) | 0.354 (0.0550, n=2) |
| 69 | 0.0712 (0.0156, n=4) | 2.73 (0.431, n=3) | 0.0844 (0.00548, n=4) | 0.850 (0.0870, n=4) |
| 70 | 0.480 (0.0706, n=2) | 0.351 (0.0135, n=2) | 0.283 (0.0281, n=3) | 0.207 (0.0330, n=3) |
| 71 | 0.166 (0.0417, n=2) | 1.02 (0.0668, n=2) | 1.02 (0.227, n=3) | 0.0604 (0.0170, n=3) |
| 72 | 0.252 (0.0456, n=2) | 0.715 (0.251, n=2) | 0.906 (0.127, n=3) | 0.0645 (0.0103, n=3) |
| 73 | 0.979 (0.317, n=2) | 0.174 (0.0239, n=2) | 2.55 (0.337, n=3) | 0.0225 (0.00178, n=3) |
| 74 | 0.0866 (0.00653, n=2) | 1.70 (0.166, n=2) | 0.467 (0.00217, n=2) | 0.115 (0.00580, n=2) |
| 75 | 0.214 (0.0234, n=2) | 0.690 (0.0905, n=2) | 1.49 (0.00142, n=2) | 0.0359 (0.00168, n=2) |
| 76 | 0.124 (0.000853, n=2) | 1.24 (0.0255, n=2) | 0.125 (0.00942, n=2) | 0.630 (0.0992, n=2) |
| 77 | 135 (22.8, n=2) | 0.00102 (0.000331, n=2) | >2000 (n=1/2) | <0.0000325 (n=1/2) |
| 78 | 0.532 (0.120, n=2) | 0.293 (0.0571, n=2) | 0.544 (0.0263, n=2) | 0.143 (0.00495, n=2) |
| 79 | 0.0732 (0.0127, n=2) | 2.11 (0.305, n=2) | 2.02 (0.562, n=2) | 0.0410 (0.0142, n=2) |
| 80 | 0.140 (0.0130, n=2) | 1.09 (0.0709, n=2) | 0.351 (0.0522, n=2) | 0.227 (0.0516, n=2) |
| 81 | 0.428 (0.0445, n=2) | 0.373 (0.0131, n=2) | 1.29 (0.00950, n=2) | 0.0630 (0.00192, n=2) |
| 82 | 24.3 (8.06, n=2) | 0.00679 (0.00175, n=2) | 26.1 (6.81, n=2) | 0.00325 (0.000941, n=2) |
| 83 | 0.182 (0.0176, n=2) | 0.804 (0.0637, n=2) | 0.387 (0.0648, n=2) | 0.166 (0.0265, n=2) |
| 84 | 0.0915 (0.00451, n=2) | 1.59 (0.0514, n=2) | 0.374 (0.0762, n=2) | 0.173 (0.0337, n=2) |
| 85 | 1.67 (0.143, n=2) | 0.111 (0.00296, n=2) | 0.0518 (0.00318, n=2) | 1.13 (0.0246, n=2) |
| 86 | 0.0452 (0.00765, n=2) | 3.29 (0.605, n=2) | 0.271 (0.0756, n=2) | 0.243 (0.0647, n=2) |
| 87 | 0.0945 (0.0277, n=2) | 1.1 | 0.0801 | 0.786 |
| 88 | 0.475 (0.00187, n=2) | 0.392 (0.0215, n=2) | 0.640 (0.00544, n=2) | 0.0918 (0.00840, n=2) |
| 89 | 0.119 (0.0150, n=2) | 1.57 (0.107, n=2) | 0.335 (0.00771, n=2) | 0.175 (0.0105, n=2) |
| 90 | 0.0361 (0.000915, n=2) | 5.17 (0.434, n=2) | 0.532 (0.0564, n=2) | 0.110 (0.00250, n=2) |
| 91 | 19.2 (n=1/2) | 0.0103 (n=1/2) | >5000 (n=1/2) | <0.0000108 (n=1/2) |
| 92 | 0.338 | 0.546 | 0.257 | 0.258 |
| 93 | 0.185 (0.0241, n=2) | 0.956 (0.292, n=2) | 0.115 (0.0143, n=3) | 0.498 (0.0199, n=3) |
| 94 | 0.425 | 0.434 | 0.338 | 0.196 |
| 95 | 1.17 (0.0326, n=2) | 0.145 (0.0227, n=2) | 1.01 (0.229, n=3) | 0.0576 (0.00824, n=3) |
| 96 | 0.712 (0.0736, n=2) | 0.238 (0.0195, n=2) | 0.590 (0.0509, n=3) | 0.0977 (0.0101, n=3) |
| 97 | 1.85 (0.214, n=2) | 0.0953 (0.0278, n=2) | 2.18 (0.347, n=3) | 0.0268 (0.00442, n=3) |
| 98 | 0.0718 | 2.43 | 0.157 | 0.384 |
| 99 | 0.0643 (0.0120, n=5) | 2.27 (0.208, n=5) | 0.0986 (0.0104, n=4) | 0.629 (0.0938, n=4) |
| 100 | 0.120 (0.0169, n=2) | 1.48 (0.191, n=2) | 0.0790 (0.00439, n=2) | 0.724 (0.0809, n=2) |
| 101 | 0.0704 (0.00913, n=5) | 2.07 (0.138, n=5) | 0.0579 (0.00589, n=4) | 1.06 (0.131, n=4) |
| 102 | 0.178 | 0.983 | 0.0628 (0.00458, n=2) | 0.913 (0.118, n=2) |
| 103 | 0.0693 (0.0165, n=4) | 2.01 (0.183, n=4) | 0.101 (0.0132, n=3) | 0.655 (0.108, n=3) |
| 104 | 0.0323 (0.00474, n=8) | 4.93 (0.595, n=8) | 0.0248 (0.00679, n=6) | 1.85 (0.242, n=6) |
| 105 | 1.85 (0.133, n=2) | 0.101 (0.00134, n=2) | 0.0401 (0.00740, n=2) | 1.51 (0.396, n=2) |
| 106 | 6.54 (n=1/2) | 0.0302 (n=1/2) | 0.0413 (0.000823, n=2) | 1.42 (0.0898, n=2) |
| 107 | 0.241 (0.0209, n=5) | 0.747 (0.0859, n=5) | 0.0539 (0.00678, n=5) | 1.10 (0.128, n=5) |
| 108 | 0.0885 (0.00413, n=2) | 2.19 (0.0798, n=2) | 0.294 (0.0441, n=2) | 0.218 (0.0253, n=2) |
| 109 | 0.109 (0.0252, n=2) | 1.83 (0.397, n=2) | 0.350 (0.0142, n=2) | 0.182 (0.0134, n=2) |
| 110 | 0.218 (0.0657, n=2) | 0.929 (0.263, n=2) | 0.179 (0.0168, n=2) | 0.358 (0.0452, n=2) |
| 111 | 0.141 (0.0253, n=2) | 1.1 | 0.308 (0.0144, n=2) | 0.209 (0.0151, n=2) |
| 112 | 0.124 (0.00217, n=2) | 1.53 | 0.160 (0.0115, n=2) | 0.402 (0.0185, n=2) |
| 113 | 0.166 (0.0364, n=2) | 1.39 | 0.233 (0.0133, n=2) | 0.275 (0.0228, n=2) |
| 114 | 0.133 (0.0220, n=2) | 1.65 | 0.267 | 0.234 |
| 115 | 0.102 (0.0163, n=5) | 1.85 (0.319, n=5) | 0.0880 (0.00660, n=4) | 0.743 (0.0516, n=4) |
| 116 | 0.0867 (0.0141, n=5) | 2.13 (0.255, n=5) | 0.0703 (0.0111, n=4) | 0.956 (0.147, n=4) |
| 117 | 0.0648 (0.00602, n=7) | 2.44 (0.263, n=6) | 0.0615 (0.00275, n=7) | 0.998 (0.0681, n=7) |
| 118 | 0.0538 (0.00395, n=7) | 3.42 (0.247, n=6) | 0.0588 (0.00577, n=6) | 1.13 (0.115, n=6) |
| 119 | 0.216 (0.0107, n=2) | 0.901 (0.0541, n=2) | 0.0913 (0.00729, n=2) | 0.702 (0.0788, n=2) |
| 120 | 0.122 (0.0397, n=2) | 1.67 (0.509, n=2) | 0.201 (0.0318, n=2) | 0.319 (0.0398, n=2) |
| 121 | 0.0760 (0.0189, n=2) | 1.44 | 0.0849 (0.0136, n=2) | 0.760 (0.128, n=2) |
| 122 | 0.0923 (0.0122, n=2) | 1.73 (0.399, n=2) | 0.0760 (0.0221, n=2) | 0.856 (0.223, n=2) |
| 123 | 0.0423 (0.00604, n=8) | 4.04 (0.586, n=8) | 0.0344 (0.00562, n=5) | 1.58 (0.180, n=5) |
| 124 | 0.0762 (0.00816, n=4) | 2.18 (0.283, n=4) | 0.0475 (0.00318, n=4) | 1.29 (0.0473, n=4) |
| 125 | 0.0381 (0.00482, n=5) | 3.79 (0.152, n=5) | 0.0478 (0.00569, n=4) | 1.31 (0.229, n=4) |
| 126 | 0.0685 (0.00653, n=4) | 2.53 (0.361, n=3) | 0.0715 (0.00249, n=4) | 0.869 (0.0332, n=4) |
| 127 | 0.158 (0.0401, n=2) | 0.917 | 0.145 (0.00951, n=2) | 0.443 (0.0176, n=2) |
| 128 | 0.0694 (0.00856, n=4) | 2.46 (0.344, n=3) | 0.0924 (0.0147, n=4) | 0.700 (0.119, n=4) |
| 129 | 0.106 (0.00707, n=2) | 1.88 | 0.121 (0.0220, n=2) | 0.543 (0.112, n=2) |
| 130 | 1.55 | 0.116 | 0.368 | 0.182 |
| 131 | 0.183 | 1.03 | 0.156 | 0.425 |
| 132 | 0.0657 | 2.88 | 0.26 | 0.255 |
| 133 | 0.0879 (0.0298, n=2) | 1.89 (1.13, n=2) | 0.0436 | 1.52 |
| 134 | 0.104 (0.00639, n=2) | 1.43 (0.244, n=2) | 0.0654 (0.00394, n=2) | 0.942 (0.00985, n=2) |
| 135 | 0.373 | 0.482 | 0.131 | 0.509 |
| 136 | 3.39 | 0.0544 | 0.06 | 1.1 |
| 137 | 0.0333 (0.00461, n=8) | 4.47 (0.381, n=8) | 0.0397 (0.00540, n=5) | 1.25 (0.114, n=5) |
| 138 | 0.0581 (0.00498, n=3) | 2.18 (0.423, n=3) | 0.0471 (0.00784, n=3) | 1.12 (0.105, n=3) |
| 139 | 0.0370 (0.00460, n=5) | 3.61 (0.444, n=5) | 0.0417 (0.00760, n=5) | 1.13 (0.105, n=5) |
| 140 | 0.0349 (0.00428, n=5) | 3.76 (0.415, n=5) | 0.0424 (0.00487, n=5) | 1.16 (0.0926, n=5) |
| 141 | 0.2 | 0.538 | 0.0975 | 0.414 |
| 142 | 0.0489 (0.00436, n=2) | 2.84 (0.637, n=2) | 0.0426 (0.00626, n=2) | 1.22 (0.0942, n=2) |
| 143 | 0.0515 (0.0105, n=2) | 2.78 (0.920, n=2) | 0.0745 (0.0109, n=2) | 0.714 (0.152, n=2) |
| 144 | 0.0375 (0.00249, n=3) | 3.60 (0.373, n=3) | 0.0608 (0.00352, n=3) | 0.851 (0.0845, n=3) |
| 145 | 0.0514 (0.0124, n=3) | 2.46 (0.300, n=3) | 0.0427 (0.00108, n=2) | 1.22 (0.116, n=2) |
| 146 | 0.0583 | 1.98 | 0.0604 | 0.951 |
| 147 | 0.0304 (0.00146, n=3) | 4.14 (0.241, n=3) | 0.0527 (0.00623, n=3) | 1.13 (0.117, n=3) |
| 148 | 0.0430 (0.00713, n=3) | 2.70 (0.462, n=3) | 0.0626 (0.00751, n=3) | 0.859 (0.167, n=3) |
| 149 | 0.0711 (0.0109, n=2) | 1.82 (0.0572, n=2) | 0.0916 (0.0149, n=2) | 0.484 (0.0343, n=2) |
| 150 | 0.0511 (0.00569, n=2) | 2.64 (0.761, n=2) | 0.0575 (0.00855, n=2) | 0.790 (0.186, n=2) |
| 151 | 0.41 | 0.262 | 1.60 (0.338, n=2) | 0.0278 (0.00331, n=2) |
| 152 | 0.0504 (0.00566, n=2) | 2.57 (0.188, n=2) | 0.0915 (0.0128, n=2) | 0.484 (0.0236, n=2) |
| 153 | 0.0634 | 1.7 | 0.0904 (0.0113, n=2) | 0.501 (0.107, n=2) |
| 154 | 0.0266 (0.00348, n=7) | 5.90 (0.513, n=7) | 0.0393 (0.00511, n=5) | 1.23 (0.117, n=5) |
| 155 | 0.266 | 0.584 | 0.495 | 0.0978 |
| 156 | >30.0 | <0.00519 | 0.638 | 0.0759 |
| 157 | 0.0453 (0.00495, n=6) | 3.15 (0.444, n=6) | 0.0431 (0.00557, n=4) | 1.25 (0.135, n=4) |
| 158 | 0.0454 (0.0102, n=5) | 3.22 (0.527, n=5) | 0.0374 (0.00374, n=5) | 1.34 (0.0947, n=5) |
| 159 | 4.6 | 0.039 | 0.33 | 0.176 |
| 160 | 21.0 (0.568, n=2) | 0.00712 (0.00165, n=2) | 0.0461 (0.00206, n=2) | 1.24 (0.0824, n=2) |
| 161 | 0.254 | 0.706 | 4.8 | 0.0121 |
| 162 | 4.56 | 0.0393 | 36 | 0.00161 |
| 163 | 0.121 | 1.29 | 0.0316 | 1.54 |
| 164 | 3.09 | 0.0581 | 15.6 | 0.00373 |
| 165 | 1.04 | 0.172 | 5.81 | 0.01 |
| 166 | 0.355 | 0.504 | 4.08 | 0.0143 |
| 167 | 0.617 | 0.291 | 1.2 | 0.0487 |
| 168 | 0.572 | 0.313 | 1.8 | 0.0323 |
| 169 | 0.86 | 0.122 | 3.37 | 0.0136 |
| 170 | 0.569 | 0.185 | 10.4 | 0.00438 |
| 171 | 0.223 | 0.471 | 0.75 | 0.0609 |
| 172 | 1.05 | 0.1 | 1.2 | 0.038 |
| 173 | 0.586 | 0.179 | 1.23 | 0.0373 |
| 174 | 0.217 | 0.483 | 0.472 | 0.0968 |
| 175 | 0.0881 | 1.19 | 1.11 | 0.0412 |
| 176 | 0.523 | 0.201 | 1.07 | 0.0427 |
| 177 | 1.4 | 0.0749 | 6.79 | 0.00673 |
| 178 | 3.08 | 0.0341 | 13.1 | 0.00349 |
| 179 | 1.83 | 0.0575 | 2.7 | 0.0169 |
| 180 | 0.79 | 0.133 | 2.16 | 0.0212 |
| 181 | 0.0459 (0.0122, n=5) | 4.10 (0.698, n=5) | 0.0592 (0.00966, n=6) | 0.718 (0.0586, n=6) |
| 182 | 0.0442 (0.0133, n=5) | 4.37 (0.940, n=5) | 0.0463 (0.00578, n=6) | 0.873 (0.0433, n=6) |
| 183 | 0.0615 (0.0175, n=5) | 3.10 (0.600, n=5) | 0.0551 (0.0125, n=5) | 0.843 (0.121, n=5) |
| 184 | 0.0477 | 2.45 | 0.392 | 0.135 |
| 185 | 0.632 | 0.185 | 7.16 | 0.00739 |
| 186 | | | 0.0939 | 0.572 |
| 187 | 0.0371 (0.00593, n=6) | 4.61 (0.839, n=6) | 0.0577 (0.00695, n=6) | 0.702 (0.0431, n=6) |
| 188 | 0.121 | 0.969 | 0.13 | 0.408 |
| 189 | 0.0775 (0.0140, n=5) | 2.41 (0.314, n=5) | 0.0608 (0.00976, n=6) | 0.668 (0.0450, n=6) |
| 190 | 0.738 | 0.158 | 0.0241 | 2.2 |
| 191 | 0.0645 (0.0139, n=3) | 2.53 (0.131, n=3) | 0.0356 (0.00490, n=3) | 1.60 (0.182, n=3) |
| 192 | 0.0615 (0.0139, n=5) | 2.83 (0.340, n=5) | 0.0289 (0.00261, n=5) | 1.73 (0.0510, n=5) |
| 193 | 0.336 | 0.538 | 0.162 | 0.359 |
| 194 | 0.423 | 0.427 | 0.284 | 0.205 |
| 195 | 0.193 | 0.936 | 0.0966 | 0.602 |
| 196 | 0.277 | 0.653 | 0.175 | 0.332 |
| 197 | 0.211 | 0.855 | 0.248 | 0.189 |
| 198 | >30.0 | <0.00602 | 0.139 | 0.337 |
| 199 | >30.0 | <0.00602 | 0.0422 | 1.11 |
| 200 | 12.3 | 0.0146 | 0.0818 | 0.573 |
| 201 | >30.0 | <0.00602 | 0.0385 | 1.22 |
| 202 | 0.0392 (0.00958, n=3) | 4.79 (0.700, n=3) | 0.0608 (0.00408, n=2) | 0.712 (0.0866, n=2) |
| 203 | 0.0387 (0.00465, n=4) | 4.95 (0.383, n=4) | 0.0679 (0.0116, n=3) | 0.671 (0.130, n=3) |
| 204 | 0.0424 (0.0132, n=3) | 4.58 (1.01, n=3) | 0.0654 (0.0166, n=2) | 0.689 (0.207, n=2) |
| 205 | 0.0281 (0.000581, n=2) | 5.71 (0.519, n=2) | 0.0261 (0.00703, n=3) | 1.46 (0.102, n=3) |
| 206 | 0.0409 (0.00271, n=2) | 4.75 (0.0828, n=2) | 0.0270 (0.00477, n=3) | 1.42 (0.139, n=3) |
| 207 | 0.0395 (0.0103, n=3) | 4.76 (0.715, n=3) | 0.0359 (0.00622, n=2) | 1.23 (0.275, n=2) |
| 208 | 0.0371 (0.00797, n=4) | 5.33 (0.808, n=4) | 0.0753 (0.00608, n=3) | 0.587 (0.0362, n=3) |
| 209 | 0.0308 (0.00636, n=5) | 5.73 (0.721, n=5) | 0.0374 (0.00451, n=4) | 1.12 (0.108, n=4) |
| 210 | 0.0383 (0.0124, n=4) | 5.40 (1.22, n=4) | 0.0432 (0.00554, n=3) | 1.03 (0.118, n=3) |
| 211 | 0.0442 (0.00939, n=5) | 4.59 (0.656, n=5) | 0.0337 (0.00481, n=4) | 1.28 (0.203, n=4) |
| 212 | 0.0501 (0.0132, n=4) | 4.17 (0.999, n=4) | 0.0572 (0.00467, n=3) | 0.771 (0.0330, n=3) |
| 213 | 0.0523 (0.0140, n=4) | 3.87 (0.721, n=4) | 0.0710 (0.0146, n=4) | 0.694 (0.110, n=4) |
| 214 | 0.0251 (0.00459, n=4) | 6.92 (0.628, n=4) | 0.0221 (0.00364, n=5) | 1.74 (0.122, n=5) |
| 215 | 0.0525 (0.00720, n=3) | 3.53 (0.379, n=3) | 0.0529 (0.00177, n=2) | 0.813 (0.0175, n=2) |
| 216 | 0.0401 (0.0151, n=3) | 4.91 (1.22, n=3) | 0.0327 (0.00291, n=2) | 1.31 (0.0447, n=2) |
| 217 | 0.0563 (0.0165, n=3) | 3.41 (0.665, n=3) | 0.0383 (0.00448, n=2) | 1.14 (0.193, n=2) |
| 218 | 0.0413 (0.00450, n=3) | 4.43 (0.113, n=3) | 0.0423 (0.00335, n=2) | 1.02 (0.137, n=2) |
| 219 | 0.0341 (0.00595, n=5) | 5.86 (0.825, n=5) | 0.0403 (0.00221, n=4) | 1.03 (0.0601, n=4) |
| 220 | 0.0315 (0.00564, n=5) | 6.30 (0.786, n=5) | 0.0312 (0.00267, n=4) | 1.36 (0.176, n=4) |
| 221 | 0.0445 (0.0102, n=4) | 4.50 (0.773, n=4) | 0.0570 (0.0101, n=3) | 0.795 (0.138, n=3) |
| 222 | 0.0306 (0.00648, n=4) | 5.84 (0.924, n=4) | 0.0248 (0.00375, n=5) | 1.55 (0.110, n=5) |
| 223 | 0.0670 (0.00561, n=2) | 2.93 (0.487, n=2) | 0.0363 (0.00532, n=3) | 0.962 (0.0435, n=3) |
| 224 | 0.0545 (0.00995, n=3) | 3.52 (0.580, n=3) | 0.0349 (0.00788, n=4) | 1.06 (0.145, n=4) |
| 225 | 0.101 (0.0194, n=2) | 1.99 (0.536, n=2) | 0.0670 (0.0107, n=3) | 0.523 (0.0355, n=3) |
| 226 | 0.0461 (0.00446, n=2) | 4.27 (0.762, n=2) | 0.0284 (0.00805, n=3) | 1.25 (0.167, n=3) |
| 227 | 0.0414 (0.00954, n=2) | 4.73 (0.688, n=2) | 0.0329 (0.00627, n=3) | 1.07 (0.0870, n=3) |
| 228 | 0.0503 (0.00265, n=2) | 3.86 (0.119, n=2) | 0.0282 (0.00274, n=2) | 1.10 (0.218, n=2) |
| 229 | 4.79 | 0.0298 | 2.11 | 0.0139 |
| 230 | 0.0431 | 3.31 | 0.0491 | 0.599 |
| 231 | 0.0253 | 5.64 | 0.0611 | 0.481 |
| 232 | 0.027 | 5.28 | 0.0724 | 0.406 |
| 233 | 0.0288 | 4.95 | 0.0549 | 0.535 |
| 234 | 0.0372 | 3.83 | 0.0926 | 0.317 |
| 235 | 0.0372 | 3.83 | 0.136 | 0.216 |
| 236 | 0.0249 (0.00475, n=5) | 6.76 (0.804, n=5) | 0.0231 (0.00333, n=5) | 1.65 (0.260, n=5) |
| 237 | 0.0883 | 2.16 | 0.0187 | 2.34 |
| 238 | 0.0296 | 7.33 | 0.0241 | 1.15 |
| 239 | 0.0353 (0.00282, n=4) | 5.27 (0.434, n=4) | 0.0376 (0.00836, n=4) | 0.987 (0.213, n=4) |
| 240 | 0.0223 | 9.73 | 0.0393 | 0.706 |
| 241 | 0.0257 (0.00164, n=3) | 7.12 (0.471, n=3) | 0.0175 (0.00373, n=3) | 1.89 (0.340, n=3) |
| 242 | 0.0333 (0.00196, n=3) | 5.58 (0.823, n=3) | 0.0164 (0.00209, n=3) | 1.96 (0.186, n=3) |
| 243 | 0.0214 (0.00212, n=3) | 8.69 (1.21, n=3) | 0.0265 (0.00423, n=3) | 1.22 (0.158, n=3) |
| 244 | 0.0225 (0.00136, n=3) | 8.27 (1.30, n=3) | 0.0252 (0.00415, n=3) | 1.29 (0.174, n=3) |
| 245 | 0.0552 | 2.82 | 0.0222 | 1.55 |
| 246 | 0.0258 (0.00180, n=6) | 6.36 (0.340, n=6) | 0.0144 (0.00106, n=5) | 2.56 (0.216, n=5) |
| 247 | 0.0622 | 2.58 | 0.0491 | 0.614 |
| 248 | 0.0328 (0.000561, n=2) | 5.08 (0.118, n=2) | 0.0428 (0.00493, n=2) | 0.800 (0.00908, n=2) |
| 249 | 0.0437 (0.00337, n=2) | 3.84 (0.449, n=2) | 0.0446 (0.0131, n=2) | 0.778 (0.129, n=2) |
| 250 | 0.0376 | 4.27 | 0.0306 | 0.985 |
| 251 | 0.0292 | 5.12 | 0.081 | 1.37 |
| 252 | 0.093 | 1.61 | 0.0483 | 1.44 |
| 253 | 0.219 | 0.684 | 0.109 | 0.638 |
| 254 | 0.215 | 0.695 | 0.0553 | 1.26 |
| 255 | 0.102 | 1.47 | 0.0407 | 1.71 |
| 256 | 0.643 | 0.233 | 0.0506 | 1.38 |
| 257 | 0.474 | 0.316 | 0.0779 | 0.895 |
| 258 | 2.43 | 0.0616 | 0.174 | 0.401 |
| 259 | 0.257 | 0.582 | 0.145 | 0.482 |
| 260 | 0.617 | 0.242 | 0.408 | 0.171 |
| 261 | 0.16 | 0.936 | 0.0948 | 0.75 |
| 262 | 0.13 | 1.15 | 0.0943 | 0.754 |
| 263 | 0.317 | 0.473 | 0.0785 | 0.906 |
| 264 | 0.0196 (0.00197, n=2) | 8.40 (1.51, n=2) | 0.0229 (0.00180, n=2) | 3.19 (0.327, n=2) |
| 265 | 0.0229 (0.00918, n=2) | 7.56 (2.21, n=2) | 0.0223 (0.00115, n=2) | 3.27 (0.0894, n=2) |
| 266 | 0.0442 (0.0109, n=6) | 2.82 (0.430, n=6) | 0.0883 (0.0104, n=6) | 0.627 (0.103, n=6) |
| 267 | 0.108 (0.0203, n=5) | 1.50 (0.0726, n=5) | 0.0540 (0.0118, n=5) | 0.974 (0.256, n=5) |
| 268 | 0.239 (0.00366, n=3) | 0.851 (0.0652, n=3) | 0.0572 (0.0135, n=5) | 0.935 (0.247, n=5) |
| 269 | 0.257 (0.0546, n=3) | 0.825 (0.187, n=3) | 0.0595 (0.0158, n=3) | 0.828 (0.202, n=3) |
| 270 | 0.328 (0.0226, n=3) | 0.627 (0.0795, n=3) | 0.128 (0.0355, n=4) | 0.400 (0.132, n=4) |
| 271 | 0.334 (0.0609, n=3) | 0.614 (0.0761, n=3) | 0.0352 (0.00104, n=2) | 1.35 (0.0587, n=2) |
| 272 | 0.0464 (0.0119, n=6) | 2.97 (0.286, n=6) | 0.0435 (0.0117, n=4) | 1.19 (0.355, n=4) |
| 273 | 0.0790 (0.0165, n=5) | 2.15 (0.333, n=5) | 0.0352 (0.0230, n=3) | 1.85 (0.845, n=3) |
| 274 | >30.0 | <0.00741 | 14.5 (5.14, n=2) | 0.00353 (0.000807, n=2) |
| 275 | >30.0 | <0.00741 | 14.0 (0.390, n=2) | 0.00357 (0.000336, n=2) |
| 276 | 0.0757 | 1.43 | 0.108 (0.0236, n=2) | 0.491 (0.117, n=2) |
| 277 | 0.0554 | 2.02 | 0.133 | 0.536 |
| 278 | 0.293 | 0.845 | 0.123 (0.0121, n=2) | 0.517 (0.0592, n=2) |
| 279 | 0.564 | 0.439 | 0.11 | 0.461 |
| 280 | 0.204 | 1.53 | 0.0767 | 0.663 |
| 281 | 0.166 | 1.87 | 0.16 | 0.318 |
| 282 | 0.323 | 0.962 | 0.247 | 0.23 |
| 283 | 0.301 | 1.03 | 0.155 | 0.329 |
| 284 | 0.113 | 2.74 | 0.0462 | 1.1 |
| 285 | 0.0884 | 3.52 | 0.072 | 0.706 |
| 286 | 0.184 | 1.69 | 0.0602 | 0.845 |
| 287 | 0.15 | 2.08 | 0.112 | 0.455 |
| 288 | 0.0732 (0.0140, n=7) | 1.20 (0.268, n=7) | 0.172 (0.0288, n=4) | 0.393 (0.128, n=4) |
| 289 | 0.0228 (0.00219, n=5) | 4.13 (0.741, n=5) | 0.0544 (0.00661, n=6) | 1.33 (0.325, n=6) |
| 290 | 0.0629 (0.0118, n=5) | 1.09 (0.176, n=5) | 0.179 (0.0336, n=6) | 0.279 (0.0844, n=6) |
| 291 | 0.118 (0.0226, n=4) | 0.746 (0.0858, n=4) | 0.150 (0.0253, n=5) | 0.188 (0.0294, n=5) |
| 292 | 0.0682 (0.0192, n=2) | 1.10 (0.0631, n=2) | 0.183 (0.0328, n=2) | 0.143 (0.0191, n=2) |
| 293 | 0.0562 (0.00736, n=2) | 1.36 (0.278, n=2) | 0.132 (0.000967, n=2) | 0.197 (0.00743, n=2) |
| 294 | 0.183 | 0.75 | 0.185 | 0.249 |
| 295 | 0.281 | 0.481 | 0.116 | 0.398 |
| 296 | 0.198 | 0.564 | 0.0867 | 0.82 |
| 297 | 0.0451 (0.00597, n=4) | 3.00 (0.569, n=4) | 0.111 (0.0151, n=5) | 0.445 (0.0482, n=5) |
| 298 | 0.0430 (0.00578, n=6) | 2.87 (0.349, n=6) | 0.0957 (0.0139, n=7) | 0.507 (0.0734, n=7) |
| 299 | 0.932 (0.507, n=2) | 0.146 (0.0780, n=2) | 2.54 (0.629, n=4) | 0.0244 (0.00551, n=4) |
| 300 | 0.0234 (0.00482, n=7) | 5.38 (1.40, n=7) | 0.0867 (0.0105, n=9) | 0.636 (0.0568, n=9) |
| 301 | 0.0346 (0.00764, n=12) | 4.11 (0.921, n=11) | 0.0441 (0.00684, n=7) | 1.61 (0.433, n=7) |
| 302 | 0.0308 (0.00225, n=8) | 3.78 (0.554, n=7) | 0.0275 (0.00247, n=6) | 1.96 (0.175, n=6) |
| 303 | 0.0254 (0.00381, n=9) | 4.20 (1.10, n=9) | 0.104 (0.0157, n=6) | 0.602 (0.0702, n=6) |
| 304 | 0.0296 (0.00440, n=7) | 2.68 (0.425, n=7) | 0.191 (0.0386, n=5) | 0.481 (0.151, n=5) |
| 305 | 0.0225 (0.00306, n=7) | 4.28 (0.953, n=7) | 0.107 (0.0149, n=6) | 0.685 (0.0980, n=6) |
| 306 | 0.0191 (0.00502, n=4) | 3.97 (0.582, n=4) | 0.0711 (0.0202, n=4) | 0.912 (0.339, n=4) |
| 307 | 0.0285 (0.00345, n=5) | 2.48 (0.481, n=5) | 0.0437 (0.00943, n=4) | 1.29 (0.238, n=4) |
| 308 | 0.0262 (0.00432, n=8) | 4.06 (1.01, n=8) | 0.0392 (0.00818, n=4) | 1.45 (0.307, n=4) |
| 309 | 0.0389 (0.00473, n=4) | 1.64 (0.313, n=4) | 0.0330 (0.00555, n=3) | 1.27 (0.312, n=3) |
| 310 | 0.0176 (0.00109, n=4) | 5.56 (1.23, n=4) | 0.0283 (0.00985, n=3) | 1.43 (0.262, n=3) |
| 311 | 0.0334 (0.00431, n=4) | 3.50 (0.928, n=4) | 0.0393 (0.0132, n=3) | 1.04 (0.220, n=3) |
| 312 | 0.0207 (0.00251, n=5) | 4.76 (1.32, n=5) | 0.0262 (0.00799, n=3) | 1.53 (0.342, n=3) |
| 313 | 0.0233 (0.00223, n=2) | 2.87 (0.830, n=2) | 0.0388 (0.00647, n=3) | 0.867 (0.197, n=3) |
| 314 | 0.0290 (0.0123, n=2) | 2.81 (0.669, n=2) | 0.0290 (0.00867, n=3) | 1.48 (0.476, n=3) |
| 315 | 0.0408 (0.00771, n=4) | 2.06 (0.452, n=4) | 0.0651 (0.0141, n=4) | 1.00 (0.245, n=4) |
| 316 | 0.0240 (0.00478, n=7) | 3.75 (0.723, n=7) | 0.122 (0.00791, n=6) | 0.644 ( 0.146, n=6) |
| 317 | 0.0948 (0.0240, n=4) | 1.01 (0.478, n=4) | 0.172 (0.00643, n=3) | 0.333 (0.104, n=3) |
| 318 | 0.0547 (0.00365, n=4) | 1.69 (0.536, n=4) | 0.124 (0.0271, n=4) | 0.482 (0.188, n=4) |
| 319 | 0.0540 (0.0220, n=4) | 2.77 (2.07, n=4) | 0.113 (0.00689, n=3) | 0.522 (0.171, n=3) |
| 320 | 0.161 | 0.241 | 0.397 | 0.224 |
| 321 | 0.0752 | 0.517 | 0.204 | 0.437 |
| 322 | 0.146 | 0.266 | 0.711 | 0.125 |
| 323 | 0.0251 (0.00551, n=4) | 3.24 (0.508, n=4) | 0.0597 (0.00797, n=4) | 1.32 (0.527, n=4) |
| 324 | 0.0374 (0.00637, n=4) | 2.13 (0.185, n=4) | 0.0926 (0.0147, n=4) | 0.798 (0.264, n=4) |
| 325 | 0.0301 (0.00366, n=5) | 2.65 (0.180, n=5) | 0.0586 (0.0119, n=5) | 1.16 (0.224, n=5) |
| 326 | 0.0754 | 1.5 | 0.0369 | 1.4 |
| 327 | 0.0548 (0.0274, n=2/3) | 1.47 (0.273, n=2/3) | 0.27 | 0.191 |
| 328 | 0.0965 | 0.94 | 0.117 | 0.575 |
| 329 | 0.132 | 0.687 | 0.125 | 0.535 |
| 330 | 0.0919 | 0.562 | 0.199 | 0.255 |
| 331 | 0.0547 (0.0162, n=2) | 1.40 (0.145, n=2) | 0.0929 (0.0237, n=2) | 0.808 (0.277, n=2) |
| 332 | 0.0745 (0.0143, n=3) | 0.949 (0.0533, n=3) | 0.184 (0.0348, n=3) | 0.345 (0.0711, n=3) |
| 333 | 0.0492 | 2.31 | 0.131 | 0.487 |
| 334 | 0.0718 | 0.844 | 0.303 | 0.151 |
| 335 | 0.0477 | 1.27 | 0.122 | 0.374 |
| 336 | 0.0312 | 1.95 | 0.0874 | 0.523 |
| 337 | 0.0515 | 1.18 | 0.173 | 0.265 |
| 338 | 0.0472 | 1.29 | 0.174 | 0.262 |
| 339 | 0.0219 (0.00722, n=3) | 3.18 (0.768, n=3) | 0.0986 (0.0180, n=4) | 0.574 (0.231, n=4) |
| 340 | 0.0823 (0.0288, n=3) | 0.852 (0.312, n=3) | 0.252 (0.00594, n=4) | 0.183 (0.0554, n=4) |
| 341 | 0.238 | 0.213 | 0.373 | 0.0674 |
| 342 | 0.159 | 0.32 | 0.127 | 0.198 |
| 343 | 0.0422 | 1.84 | 0.124 | 0.662 |
| 344 | 0.0433 | 1.79 | 0.044 | 1.86 |
| 345 | 0.0649 | 2.16 | 0.035 | 0.937 |
| 346 | 0.144 (0.0284, n=2) | 0.604 (0.00648, n=2) | 0.128 (0.0378, n=3) | 0.210 (0.0493, n=3) |
| 347 | 0.0827 | 0.872 | 0.102 (0.0247, n=2) | 0.245 (0.0142, n=2) |
| 348 | 0.193 | 0.373 | 0.113 (0.00703, n=2) | 0.224 (0.0269, n=2) |
| 349 | 0.117 (0.00129, n=2) | 0.756 (0.131, n=2) | 0.121 (0.0287, n=3) | 0.214 (0.0378, n=3) |
| 350 | 0.189 | 0.741 | 0.107 | 0.307 |
| 351 | 0.298 | 0.47 | 0.149 | 0.22 |
| 352 | 0.127 (0.0116, n=2) | 0.815 (0.193, n=2) | 0.142 (0.0267, n=3) | 0.201 (0.0439, n=3) |
| 353 | 0.497 | 0.145 | 1.09 (0.0881, n=2) | 0.0231 (0.00235, n=2) |
| 354 | 0.233 (0.0309, n=2) | 0.441 (0.0871, n=2) | 0.540 (0.126, n=3) | 0.0517 (0.00657, n=3) |
| 355 | 0.685 (0.318, n=2) | 0.111 (0.0498, n=2) | 0.896 (0.00185, n=2) | 0.0285 (0.00509, n=2) |
| 356 | 0.386 | 0.364 | 0.506 | 0.0647 |
| 357 | 0.384 | 0.366 | 0.181 | 0.181 |
| 358 | 0.183 (0.0347, n=2) | 0.662 (0.0284, n=2) | 0.117 (0.0257, n=2) | 0.260 (0.0901, n=2) |
| 359 | 0.172 (0.0235, n=2) | 0.706 (0.00679, n=2) | 0.131 (0.0201, n=2) | 0.227 (0.0647, n=2) |
| 360 | 0.197 | 0.53 | 0.145 | 0.171 |
| 361 | 0.401 | 0.261 | 0.955 | 0.0259 |
| 362 | 0.302 (n=1/2) | 0.347 (n=1/2) | 0.365 (0.0425, n=2) | 0.0805 (0.0202, n=2) |
| 363 | 0.145 (0.0185, n=2) | 0.835 (0.0159, n=2) | 0.208 (0.0485, n=2) | 0.146 (0.0522, n=2) |
| 364 | 0.351 (0.0809, n=2) | 0.347 (0.0290, n=2) | 1.00 (0.213, n=2) | 0.0303 (0.0103, n=2) |
| 365 | 0.0678 | 0.784 | 0.175 | 0.155 |
| 366 | 0.0889 | 0.598 | 0.366 | 0.074 |
| 367 | 0.0179 | 5.85 | 0.0641 | 0.386 |
| 368 | 0.0757 | 0.109 | 0.108 | 0.299 |
| 369 | 0.166 | 0.499 | 0.101 | 0.319 |
| 370 | 0.117 | 0.704 | 0.186 | 0.172 |
| 371 | 0.135 | 0.393 | 0.132 | 0.206 |
| 372 | 0.0781 | 0.68 | 0.365 | 0.0741 |
| 373 | 0.185 | 0.287 | 0.436 | 0.0621 |
| 374 | 0.0468 | 1.76 | 0.190 | 0.169 |
| 375 | 0.0471 | 1.13 | 0.152 | 0.178 |
| 376 | 0.0723 | 0.734 | 0.170 | 0.159 |
| 377 | 0.0544 | 0.976 | 0.136 | 0.199 |
| 378 | 0.067 | 0.793 | 0.191 | 0.142 |
| 379 | 0.079 | 0.672 | 0.238 | 0.114 |
| 380 | 0.142 | 0.374 | 0.236 | 0.115 |

As demonstrated by data in Table 3, Example compounds stimulate cAMP from human GLP-1R and GIPR in the presence of 0.1% casein.

### IN VIVO STUDIES

### Pharmacokinetics in male CD-1 mice

The pharmacokinetics of select Examples are evaluated following a single subcutaneous administration of 200 nMol/kg to male CD-1 mice. Blood samples are collected over 168 hours and resulting individual plasma concentrations are used to calculate pharmacokinetic parameters. Plasma (K₃ EDTA) concentrations are determined using a qualified LC/MS method that measures the intact mass of the Examples. Each Example and an analog as an internal standard are extracted from 100% mouse plasma using immunoaffinity based precipitation with anti-GIP/GLP1 antibodies. Instruments are combined for LC/MS detection. Mean pharmacokinetic parameters are shown in **Table 4.**

**Table 4.. Mean Pharmacokinetic Parameters of peptides Following a Single Subcutaneous Administration of 200 nMol/kg to Male CD-1 mice (N=2/timepoint non-serial sampling).**

| Example | T_{1/2} (hr) | Tₘₐₓ (hr) | Cₘₐₓ/D (kg*nmol/L/nmol) | AUCINF_D_obs (hr*kg*nmol/L/nmol) | Cl/F (mL/hr/Kg) |
|---|---|---|---|---|---|
| Example 1 | 17.54 | 12 | 4.84 | 135.61 | 7.37 |
| Example 2 | 7.55 | 6 | 5.4 | 77.23 | 12.95 |
| Example 3 | 15.04 | 6 | 4.42 | 158.49 | 6.31 |

| | | | | | |
|---|---|---|---|---|---|
| Abbreviations: T_{1/2} = half-life, Tₘₐₓ = time to maximal concentration, Cₘₐₓ = maximal plasma concentration, AUCINF_D_obs = AUCinf divided by dose, CL/F = clearance/bioavailability. Notes: Data are the mean, where n=2/timepoint/group. | | | | | |

Results from this study for Examples tested are consistent with an extended pharmacokinetic profile.

### Pharmacokinetics in male Cynomolgus Monkeys

The pharmacokinetics of select Examples are evaluated following a single subcutaneous administration of 50 nMol/kg to male cynomolgus monkeys. Blood samples are collected over 336 hours and resulting individual plasma concentrations are used to calculate pharmacokinetic parameters. Peptide plasma (K₃ EDTA) concentrations are determined using a qualified LC/MS method that measured the intact mass of the compound. Each peptide and an analog as an internal standard are extracted from 100% cynomolgus monkey plasma using immunoaffinity based precipitation with anti-GIP/GLG1 antibodies. Instruments are combined for LC/MS detection. Mean pharmacokinetic parameters are shown in **Table 5.**

**Table 5. Mean Pharmacokinetic Parameters of peptides Following a Single Subcutaneous Administration of 50 nMol/kg to Male Cynomolgus Monkeys.**

| Example | T_{1/2} (hr) | Tₘₐₓ (hr) | Cₘₐₓ/D (kg*nmol/L/nmol) | AUCINF_D_obs (hr*kg*nmol/L/nmol) | Cl/F (mL/hr/Kg) |
|---|---|---|---|---|---|
| Example 1 | 125.0 | 18 | 6.5 | 1458 | 0.69 |
| Example 2 | 102.1 | 24 | 11.7 | 2059 | 0.49 |
| Example 3 | 180.6 | 30 | 11.38 | 3420 | 0.29 |

| | | | | | |
|---|---|---|---|---|---|
| Abbreviations: T_{1/2} = half-life, Tₘₐₓ = time to maximal concentration, Cₘₐₓ = maximal plasma concentration, AUCINF_D_obs = AUCinf divided by dose, CL/F = clearance/bioavailability. Notes: Data are the mean, where n=2/group. Notes: Data are the mean, where n=2/group. As seen in **Table 5**, results from this study for Example peptides tested are consistent with an extended pharmacokinetic profile. | | | | | |

### Pharmacokinetics in male Sprague Dawley rats following subcutaneous or intrajejunal administration

The pharmacokinetics of select Examples are evaluated following a single subcutaneous (SC) administration of 50 nMol/kg (dissolved in PBS, pH 7.4) or single 1 µmol/kg (mixed with 250 mM sodium decanoate ("C10") and 12 mg/mL soybean trypsin inhibitor (SBTI)) intrajejunal (IJ) administration to male Sprague Dawley rats. Blood samples are collected over 168 hours following SC administration and 72 hours following IJ dosing. Pharmacokinetic parameters are calculated using individual plasma concentrations. A qualified LC/MS method that measures the intact mass of the Example is used to determine plasma (K₃ EDTA) concentrations. Each Example is tested with an analog peptide as an internal standard. Immunoaffinity based precipitation with anti-GIP/GLP1 antibodies is used to extract each test peptide and analog. Mean pharmacokinetic parameters for the Examples are shown in **Table 6** and **Table 7**.

**Table 6. Mean (+/- SD) Pharmacokinetic Parameters of peptides Following a Single Subcutaneous Administration of 50 nMol/kg to Male Sprague Dawley rats.**

| Example | T_{1/2} (hr) | Tₘₐₓ (hr) | Cₘₐₓ/D (kg*nmol/L/nmol) | AUCINF_D_obs (hr*kg*nmol/L/nmol) | Cl/F (mL/hr/Kg) |
|---|---|---|---|---|---|
| Example 1 | 44.7 (6.2) | 21.3 (4.6) | 3.34 (0.22) | 294.2 (30.0) | 3.42 (0.33) |
| Example 2 | 20.3 (0.9) | 14.7 (2.3) | 15.19 (0.20) | 231.7 (9.6) | 4.32 (0.17) |
| Example 3 | 32.1 (1.9) | 21.3 (4.6) | 14.71 (0.50) | 371.8 (21.8) | 2.70 (0.16) |

| | | | | | |
|---|---|---|---|---|---|
| Abbreviations: T_{1/2} = half-life, Tₘₐₓ = time to maximal concentration, Cₘₐₓ = maximal plasma concentration, AUCINF_D_obs = AUCinf divided by dose, CL/F = clearance/bioavailability. Notes: Data are the mean, where n=3/group (Table 6) As seen in table 6, results from this study using these Example peptides are consistent with an extended pharmacokinetic profile. | | | | | |

**Table 7. Mean (+/- SD) Pharmacokinetic Parameters of peptides Following a Single Intrajejunal Administration of 1 µmol/kg to Male Sprague Dawley rats.**

| Example | Tₘₐₓ (hr) | Cₘₐₓ/D (kg*nmol/L/nmol) | AUCINF_D_obs (hr*kg*nmol/L/nmol) |
|---|---|---|---|
| Example 1 | 1.33 (0.82) | 0.08 (0.05) | 1.31 (0.85) |
| Example 2 | 0.25 (0.13) | 0.56 (0.40) | 6.6 (4.4) |
| Example 3 | 0.33 (0) | 0.47 (0.16) | 8.45 (3.1) |

| | | | |
|---|---|---|---|
| Data are the mean, where n=3/group n=6/group (Table 7). | | | |

As illustrated by results in **Table 7,** these Examples are consistent with an exposure following intrajejunal administration. Intrajejunal exposure in this assay supports that the Examples may be suitable for oral formulation and administration.

### In Vivo Effect on Insulin Secretion in Male Wistar Rats

Male Wistar rats with femoral artery and femoral vein canulas (Envigo, Indianapolis, IN) (280- 320 grams) are single-housed in polycarbonate cages with filter tops. Rats maintained on a 12:12 h light-dark cycle (lights on at 6:00 A.M.) at 21°C and receive food and deionized water ad libitum. Rats are randomized by body weight and dosed 1.5 ml/kg s.c. at doses of 0.04, 0.1, 0.3, 1, 3, and 10 nmol/kg 16 hours prior to glucose administration then fasted. Animals are weighed and anesthetized with sodium pentobarbital dosed i.p. (65 mg/kg, 30 mg/ml). A time 0 blood sample is collected into EDTA tubes after which glucose is administered i.v. (0.5 mg/kg, 5 ml/kg). Blood samples are collected for glucose and insulin levels at time 2, 4, 6, 10, 20 and 30 min post intravenous administration of glucose. Plasma glucose levels are determined using a clinical chemistry analyzer. Plasma insulin is determined using an electrochemiluminescence assay (Meso Scale, Gaithersburg, MD). Glucose and insulin AUC are examined compared to the vehicle control with n = 5 animals per group. Results are presented (SEM)(N).

**Table 8: The effect of Example compounds on insulin secretion during intravenous glucose tolerance test.**

| Dose (nmol/kg, s.c.) | | | | | | | |
|---|---|---|---|---|---|---|---|
| Example | 0.0 | 0.04 | 0.1 | 0.3 | 1.0 | 3 | 10 |
| 1 | 31.3 (2.8) (5) | 32.2 (5.7) (5) | 31.5 (4.5) (5) | 24.7 (3.0) (5) | 35.1 (4.0) (5) | 43.5 (4.9) (5) | 63.9 (6.5) (5) |
| 2 | 18.9 (4.3) (5) | 32.8 (3.9) (5) | 49.1 (4.8) (5) | 82.2 (21.1) (5) | 110.9 (23.1) (5) | 108.2 (20.2) (5) | 77.3 (8.8) (5) |
| 3 | 18.5 (1.0) (5) | 26.0 (3.4) (5) | 24.6 (3.9) (5) | 44.9 (9.6) (5) | 60.1 (4.0) (5) | 95.5 (18.4) (5) | 87.7 (7.9) (5) |
| 4 | 33.7 (5.3, 5) | 34.0 (3.4, 5) | 42.0 (3.8, 5) | 86.3 (4.5, 5) | 90.2 (9.2, 5) | 108.7 (9.8, 5) | 114.6 (16.1, 5) |
| 5 | 24.4 (3.0, 5) | 28.2 (4.2, 5) | 40.2 (6.0, 5) | 41.1 (2.7, 5) | 44.1 (4.5, 5) | 54.3 (11.9, 5) | 94.2 (10.1, 5) |

The data provided by Table 8 demonstrate a dose dependent increase in insulin secretion.

**Table 9: ivGTT Insulin Secretion shown by the following data:**

| Insulin secretion (ivGTT) | |
|---|---|
| Example | (ED₅₀, nmol/kg) (SEM, n) |
| 1 | >10 |
| 2 | 0.1 (0.05, 5) |
| 3 | 0.7 (0.3, 5) |
| 4 | 0.2 (0.05, 5) |
| 5 | 3<ED₅₀<10 |

The data provided by Table 9 demonstrate dose dependent increase in insulin secretion.

### Studies in Diet-Induced Obese C57/B16 Mice

C57/Bl6 diet-induced obese (DIO) male mice (Taconic, Germantown, NY) weighing 41-50 g are used. Animals are individually housed in a temperature-controlled (24°C) facility with a 12 hour light/dark photoperiod (lights off at 10:00 AM and lights on at 10:00 PM), with free access to food and water. After 2 week acclimatization to the facility, mice are randomized to treatment groups (n=6/group) based on body weight so each group has similar starting mean body weight.

Mice are treated with either vehicle (40 mM Tris-HCl at pH 8.0) or several peptides between the dose ranges of 0.03 nmol/kg to 10 nmol/kg. Treatments are subcutaneously administered to ad libitum fed DIO mice 30-90 minutes prior to the onset of the dark cycle daily (QD) for 14 days. During the course of the study, body weight and food intake are monitored daily.

All data are expressed as mean ± SEM of 5-6 rats per group. Statistical analyses are assessed by one-way ANOVA followed by Dunnett's multiple comparison test to compare treatment groups to vehicle group or each other. Significant differences are identified at p<0.05. $Percent Body Weight = \frac{Body weight after 14 - day treatment}{Body weight before treatment started} \times 100$ "0" dose group represents the vehicle-treated mice during each study. All data are expressed as mean ± SEM of 5-6 mice per group. Statistical analyses are assessed by one-way ANOVA followed by Dunnett's multiple comparison test to compare treatment groups to '0' dose (vehicle). *Significant differences are identified at p<0.05. Body weight change after treatment with Example compounds after 15 days. "Δ from vehicle" refers to difference between body weight at day 15 between test and vehicle groups. "% change" refers to percent decrease in body weight between days 1 and 15 in test groups. Percent decrease in body weight for animals receiving vehicle is recorded, and is less than about 1% in each study. The Δ from vehicle and % change data are statistically significantly different (p<0.05) than control for all Examples at all doses tested.

**Table 10. The effect of GIP/GLP-1 receptor co-agonists on percent body weight in diet-induced obese mice after 14-day of treatment.**

| | Dose (nmol/kg, s.c., QD) | | | | | | |
|---|---|---|---|---|---|---|---|
| Peptide | 0 | 0.03 | 0.1 | 0.3 | 1 | 3 | 10 |
| Example 1 | 99.2 ±0.8 | 96.2 ±1.2 | 95.6 ±0.9 | 86.7 ±1.3* | 86.3 ±1.9* | 74.0 ±3.8* | 64.7 ±2.6* |
| Example 2 | 100.5 ±1.4 | 101.5 ±0.2 | 95.0 ±1.2 | 86.5 ±0.8* | 76.4 ±4.0* | 76.4 ±2.4* | 68.1 ±3.1* |
| Example 3 | 98.0 ±0.7 | 99.1± 1.3 | 95.6 ±1.3 | 93.0 ±1.1 | 85.6 ±0.8* | 75.9 ±4.3* | 73.6 ±1.7* |
| Example 4 | 98.3±1.1 | 96.6±0.5 | 94.7±1.8 | 88.5±1.2* | 76.9±1.4* | 66.6±3.9* | 64.5±2.2* |
| Example 5 | 98.3±1. 1 | 96.0±1. 3 | 96.7±1. 1 | 94.1±1. 8 | 82.4±1. 6* | 83.8±1. 6* | 74.9±2. 3* |
| Example 104 | 99.2±0. 8 | 94.0±0. 6 | 94.1±0. 8 | 89.0±0. 9* | 82.7±1. 5* | 70.8±4. 2* | 71.3±4. 1* |
| Example 123 | 99.2±0. 8 | 94.7±0. 9 | 90.5±1. 6* | 86.5±1. 1* | 81.3±2. 0* | 75.1±1. 8* | 68.6±1. 9* |

As illustrated by data provided in Table 10 above, Example compounds tested in the assay dose-dependently reduce body weight in the studies described.

### Proteolytic Stability Assay

The proteolytic stability assay is a useful for assessing potential for oral delivery of peptides. The stability of peptides are compared in 1% rat small intestinal fluid (rSIF). The amount of intact peptide is measured for a sample peptide at 0, 3, 15, and 30 minutes to assess proteolytic stability. The amount of intact peptide for a sample peptide is measured in 90% pig small intestinal fluid (pSIF) at 0, 30, 45, and 60 minutes to assess the proteolytic stability.

Sample preparation when rat small intestinal fluid (rSIF) is used:
Peptides are prepared at 0.4 mg/mL in 50mM Tris pH8.0. Rat small intestinal fluid is added at a ratio of 1% (v/v). The mixture is incubated at 37°C at 150 rpm. Thirty µL of each sample are removed and placed into a new tube before the rSIF is added and at 3, 15, and 60 min. At each time point, the reaction was quenched by 1% TFA in 50% ACN at 1:1. The samples are diluted 100 times using dilution buffer (1:1 of 1% TFA in 50% ACN: 50mM Tris pH8) and ready for analysis using mass spectrometry (MS).

Sample preparation when pig small intestinal fluid (pSIF) is used:
Peptides are diluted to a concentration of 0.4 mg/mL in 90% pig small intestinal fluid. After the mixing, 20 µL are immediately removed (time 0 for the time point of preincubation). The mixture is then incubated at 37°C at 150rpm. Twenty µL of each sample are removed and placed into a new tube at 30, 45, and 60 min. At each time point (0, 30, 45, 60), the reaction is quenched by 1% TFA in 50% ACN at 1:1. The sample is centrifuged at 20,000xg for 20 min at 4°C. The supernatant is diluted 100 times using dilution buffer (1:1 of 1% TFA in 50% ACN: 50mM Tris pH 8) and ready for analysis using mass spectrometry (MS).

MS Conditions: The liquid chromatography separation is carried out on a Waters Acquity UPLC using mobile phase A (0.1% formic acid in water) and B (0.1% formic acid in acetonitrile and an ACQUITY UPLC Protein BEH C4 Column (300Å, 1.7 µm, 1 mm X 50 mm) at 40°C. The gradient is 5% of B during 0-1.5, 5-90% of B during 1.5-1.8, 90-95% of B during 1.8-3.0, 95-95% of B during 3.0-3.5, 95-5% of B during 3.5-4.0, and 5-5% of B during 4.0-5.0. The MS analysis is carried out on a Waters Xevo G2-XS QTOF. The data is acquired using MSe Continuum in the range of 50-2000 m/z in positive and sensitivity mode. The data analysis is performed using MassLynx.

**Table 11. The percentage of each peptide not cleaved at different time points using rSIF.**

| | 0 min | 3 min | 15 min | 60 min |
|---|---|---|---|---|
| Example 1 | 100 | 82.4 | 41.4 | 1.6 |
| Example 2 | 100 | 75.5 | 18.3 | 0.3 |
| Example 3 | 100 | 68.8 | 25.8 | 0.3 |
| Example 4 | 100 | 97.9 | 99.3 | 89.4 |
| Example 69 | 100 | 2.2 | 0.0 | 0.0 |

The proteolytic peptide results provided in Table 11 suggest that the peptide of Example 4 may be suitable for oral formulation and delivery.

**Table 12. The percentage of each peptide not cleaved at different time points using pSIF.**

| | 0 min | 30 min | 45 min | 60 min |
|---|---|---|---|---|
| Example 4 | 100 | 73.4 | 56.4 | 60.0 |
| Example 5 | 100 | 76.9 | 56.8 | 60.7 |

The proteolytic peptide results provided in Table 12 suggest that both the peptides of Examples 4 and 5 may be suitable for oral formulation and delivery.

### In Vivo Studies

The purpose of this study is to determine the relative potential for clinical immunogenicity of a compound.

### Methods:

CD8+ T cell depleted peripheral blood mononuclear cells are prepared and labeled with Carboxyfluorescein Diacetate Succinimidyl Ester (CFSE, Invitrogen) from a cohort of 10 healthy donors. Samples are tested in triplicate with 2.0 mL media control, keyhole limpet hemocyanin ("KLH") (0.33µM), anti-chemokine receptor type 4 ("CD4+") (0.33µM), and a compound of Examples 1, 2, and 3 (10 µM). Cultures are incubated for 7 days at 37°C with 5% CO_{2.} On day 7, samples are analyzed by flow cytometry using High Throughput Sampler (HTS). Data is analyzed using FlowJo^{®} Software (FlowJo, LLC, TreeStar).

### Results and Discussion

All donors produce a positive T cell response against KLH (100%). Analysis of the frequency and magnitude of the CD4+ T cell response for Example compounds is shown in Table 13.

**Table 13: CD4+ T Cell Responses for Example compounds and Positive Control (KLH).**

| | % Donor Response | Median Response Strength in positive donors (CDI) |
|---|---|---|
| KLH | 100% (n=11) | 391 (n=10) |
| Example 1 (GG-212) | 9% (n=11) | 0.7 (n=1) |
| Example 2 (GG-353) | 22% (n=9) | 3.68 (n=2) |
| Example 3 (GG-362) | 0% (n=9) | NA (n=0) |
| Example 4 (GG-427) | 0% (n=9) | NA (n=0) |
| Example 288 (GG-709) | 10% (n=10) | 5.42 (n=1) |
| Example 289 (GG-731) | 0% (n=10) | NA (n=0) |
| Example 301 (GG-650) | 0% (n=10) | NA (n=0) |
| Example 303 (GG-679) | 0% (n=10) | NA (n=0) |
| Example 316 (GG-698) | 0% (n=10) | NA (n=0) |

| | | |
|---|---|---|
| Cell Division Index ("CDI"): proportion of divided CD4+ T cells to the total number of CD4+ T cells in stimulated versus unstimulated samples. | | |

These data show that the frequency of positive CD+ T cell response (CDI>2.5) was low for the compounds of Examples 1, 2, 3, 4, 288, 289, 301, 303 and 316, and the magnitude of the response in the few positive donors was low (CDI<6), indicating a low risk of immunogenicity using the CD4+ T cell assay.

### GLP-1R HEK293 Cell Membrane [³⁵S]GTPγS Binding Assay

The GLP-1 receptor is a G-protein coupled receptor that increases GTP-bound Gαₛ upon ligand induced receptor activation. The potency of peptides to stimulate- GLP-1R induced activation of Gαₛ is determined using preparations of purified membranes from HEK293 cells expressing the human GLP-1R. The assay is performed similarly to that as previously described (Bueno et al., J. Biol. Chem., (2016) 291, 10700 and Willard et al., Mol. Pharmacol. (2012) 82,1066). The test peptides are solubilized in DMSO and diluted in reaction buffer containing 5 µg of membrane in 20 mM HEPES pH 7.4, 50 mM NaCl, 5 mM MgCl₂, 40 µg/ml saponin, 0.1% BSA, and 500 pM ³⁵S-labeled GTPγS for 30 minutes at room temperature. Reactions are terminated by addition of 0.2% Nonidet P-40 detergent containing rabbit anti-Gαₛ polyclonal antibody and 0.5 mg of anti-rabbit polyvinyltoluene beads. Mixtures are developed for 30 minutes, centrifuged at 80 × *g* for 10 minutes, and counted for 1 minute/well using a MicroBeta TriLux instrument. Peptide concentration-response curves are fit to a four-parameter logistic model to calculate potency as an EC₅₀. Data normalization to % stimulation is performed using DMSO and GLP-1(7-36) as minimum and maximum controls for the receptor (Campbell et al, Assay Guidance Manual 2017). The potency of a sample peptide to stimulate GIPR induced activation of Gαₛ is reported in the **Table 14**. Assay results identify a petpide that is a partial agonist on the GLP-1R with respect to GLP-1R induced activation of Gα_{s.}

### GLP-1R CHO Cell β-Arrestin Recruitment Assay

Activated G-protein coupled receptors can interact with the β-arrestin family of signalling proteins. The potency of peptides for GLP-1R induced arrestin recruitment is determined using the PathHunter Enzyme Fragment Complementation approach substantially as described (von Degenfeld et al., FASEB J., 2007 (14):3819-26 and Hamdouchi et al., J. Med Chem., 2016 59(24):10891-10916). CHO-K1 cells expressing Pro-Link-tagged Human GLP-1R and enzyme-acceptor-tagged β-arrestin-2 may be obtained from DiscoveRx and prepared as assay-ready frozen cells. Test peptides are solubilized in DMSO and serial dilutions are perfomed using the Echo acoustic dispenser (LabCyte). Assay media is the PathHunter Cell Assay Buffer (DiscoveRx) containing 0.1% w/v hydrolyzed Casein (Sigma). 100 nl of peptide is dispensed into 10 µl of assay media in a 384 well plate and then 10 µl of cells in assay media are added to give 5000 cells per well. Plates are incubated for 90 minutes in a 37'C/5% C02 incubator and 10 µl of PathHunter detection reagent is added (DiscoveRx) and plates are incubated at room temperature for 60 minutes. Luminescence signal is measured. Peptide concentration-response curves fit to a four-parameter logistic model to calculate potency as an EC₅₀. Data normalization to % stimulation is performed using DMSO and GLP-1(7-36) as minimum and maximum controls (Campbell et al, Assay Guidance Manual 2017). The potency of a sample peptide to stimulate GLP-1R induced β-arrestin recruitment is reported in **Table 14**. The assay results identify a peptide that is a partial agonist on the GLP-1R with respect to β-arrestin-2 recruitment.

**Table 14**

| **Example** | **hGLP1R GTPgS Rel EC50 nM (SEM, n)** | **hGLP1R GTPgS % Top (SEM, n)** | **hGLP1R B-Arrestin2 Rel EC50 uM (SEM, n)** | **hGLP1R B-Arrestin2 % Top (SEM, n)** |
|---|---|---|---|---|
| | 0.475 (0.0322, n=115) | 99.2 (0.659, n=115) | 0.00274 (0.000359, n=42) | 104 (3.45, n=42) |
| 1 | 0.235 (0.0201, n=5) | 91.1 (1.77, n=5) | 0.005 | 105 |
| 2 | 0.642 (0.0294, n=2) | 95.9 (0.553, n=2) | 0.00882 (0.00269, n=2) | 96.1 (0.742, n=2) |
| 3 | 0.421 (0.181, n=2) | 95.4 (2.20, n=2) | | |
| 4 | 0.245 (0.0638, n=3) | 86.9 (5.93, n=3) | 0.00480 (0.000138, n=2) | 92.4 (14.0, n=2) |
| 5 | 0.196 (0.0375, n=3) | 91.3 (6.90, n=3) | | |
| 266 | 0.865 (0.328, n=2) | 63.4 (1.31, n=2) | 0.016 | 17.1 |
| 267 | 0.867 | 62.3 | 0.00901 | 16.5 |
| 272 | 0.651 (0.0427, n=2) | 66.5 (0.741, n=2) | >12.0 | ND |
| 298 | 1.03 | 57.3 | | |
| | | | | |
| 300 | 0.405 | 85.6 | 0.0054 | 38.4 |
| 301 | 0.435 (0.0848, n=3) | 91.4 (3.63, n=3) | 0.00267 | 93.7 |
| 302 | 0.268 | 98.6 | 0.00219 | 98.4 |
| 303 | 0.547 (0.0998, n=2) | 74.3 (2.99, n=2) | 0.0179 | 47.7 |
| 304 | 0.561 | 77.1 | | |
| 305 | 0.389 | 76.3 | | |
| 306 | 0.378 | 76.1 | | |
| 315 | 0.601 | 44.2 | 0.0199 | 25.4 |
| 316 | 0.766 (0.0469, n=2) | 56.7 (3.14, n=2) | 0.00608 | 26.1 |
| 317 | 0.536 | 53.7 | | |
| 318 | 0.415 | 58.4 | | |
| **288** | 0.666 (0.104, n=3) | 66.7 (4.09, n=3) | 0.00674 (0.00278, n=3) | 21.3 (1.94, n=3) |
| 319 | 0.657 | 65.7 | | |
| 323 | 0.79 | 81.9 | | |
| 324 | 0.475 | 84.5 | | |
| **289** | 0.404 (0.0247, n=3) | 83.7 (3.81, n=3) | 0.0124 (0.00151, n=3) | 51.3 (6.05, n=3) |
| 325 | 0.414 | 97.9 | | |
| 326 | 0.663 | 61.6 | | |
| 327 | 0.287 | 75.6 | 0.00379 | 41.6 |
| 328 | 0.481 | 66.3 | | |
| 329 | 0.343 | 83.6 | 0.00473 | 63.4 |
| 330 | 1.05 (0.275, n=2) | 47.8 (1.16, n=2) | >10.9 | ND |
| 331 | 0.375 (0.0274, n=4) | 80.9 (2.54, n=4) | 0.0128 | 44.3 |
| 332 | 0.453 (0.0479, n=4) | 81.5 (4.65, n=4) | 0.0171 | 45.6 |
| 333 | 0.442 (0.00535, n=2) | 83.4 (0.439, n=2) | 0.0548 | 58.2 |
| 334 | 0.432 | 70.2 | >10.3 | ND |
| 335 | 0.285 | 89.3 | 0.00531 | 73 |
| 336 | 0.377 | 90.2 | 0.00778 | 82.5 |
| **290** | 0.466 (0.0664, n=9) | 66.2 (3.12, n=9) | 0.0238 (0.00530, n=4) | 20.0 (1.15, n=4) |
| 337 | 0.322 | 59.5 | 0.0174 | 34.1 |
| 338 | | | 0.0189 | 47 |
| 339 | 0.326 (0.0357, n=3) | 74.1 (7.97, n=3) | 0.0107 (0.00238, n=2) | 44.0 (2.40, n=2) |
| 340 | 0.450 (0.0182, n=5) | 67.4 (5.12, n=5) | 0.0107 (0.00711, n=4) | 20.1 (2.31, n=4) |
| 341 | 0.496 | 78.9 | 0.0188 | 21.9 |
| 342 | 0.414 | 77.2 | 0.035 | 20.7 |
| 343 | 0.522 | 74.9 | 0.0455 | 41.4 |
| 344 | 0.423 | 85.8 | 0.0343 | 46 |
| 345 | 0.684 | 62 | 0.00308 (0.000666, n=2) | 74.7 (2.86, n=2) |
| 346 | 0.737 (0.201, n=3) | 56.6 (3.94, n=3) | 0.00325 (0.00105, n=3) | 19.3 (0.767, n=3) |
| 347 | 0.759 | 46.9 | 0.00542 (0.00152, n=2) | 24.6 (2.81, n=2) |
| 348 | 0.66 | 47.7 | 0.00346 (n=1/2) | 19.8 |
| 349 | 0.464 (0.0290, n=4) | 64.5 (1.68, n=4) | 0.0151 (0.00111, n=2) | 18.4 (0.337, n=2) |
| 350 | 0.589 | 64.1 | 0.0108 | 21.8 |
| 351 | 0.563 | 66.5 | 0.0196 | 23.5 |
| 352 | 0.552 (0.0267, n=2) | 63.5 (1.51, n=2) | 0.00421 (n=1/2) | 17.4 |
| 353 | 1.96 | 63.1 | 0.559 (0.108, n=2) | 23.0 (0.408, n=2) |
| **291** | 0.466 (0.0476, n=6) | 65.0 (2.15, n=6) | >10.0 (n=1/4) | ND |
| 354 | 0.967 | 53.3 | 0.255 (n=1/2) | 22.7 |
| 355 | 1.76 | 50.6 | 0.363 | 18.3 |
| 356 | | | >10.5 | ND |
| 357 | | | 0.118 | 18.2 |
| 358 | 0.414 (0.00356, n=2) | 72.3 (1.27, n=2) | 0.00938 (0.00413, n=3) | 21.4 (2.47, n=3) |
| 359 | 0.496 | 69.7 | 0.0841 | 28.4 |
| 360 | | | 0.0395 | 25.8 |
| 361 | | | 0.269 (n=1/2) | 20.2 |
| 362 | | | >11.0 | ND |
| 363 | 0.943 | 69.6 | 0.135 | 22.1 |
| 364 | | | >12.0 | ND |
| **292** | 0.429 (0.0190, n=4) | 71.5 (3.33, n=4) | 0.00774 (0.00199, n=4) | 29.0 (3.96, n=4) |
| **293** | 0.368 (0.0304, n=4) | 70.6 (0.715, n=4) | 0.00719 (0.00168, n=4) | 29.5 (6.23, n=4) |
| 365 | 0.464 (0.0178, n=3) | 66.9 (1.35, n=3) | 0.00703 (0.00233, n=4) | 20.8 (1.78, n=4) |
| 366 | 0.409 (0.0308, n=3) | 67.6 (3.55, n=3) | 0.00557 (0.00363, n=2) | 21.2 (0.163, n=2) |
| 367 | 0.289 | 89.7 | 0.00666 (0.00118, n=2) | 70.6 (8.61, n=2) |
| 368 | 0.495 | 68.9 | 0.0479 (0.0205, n=2) | 21.3 (4.03, n=2) |
| 369 | 0.381 | 58.8 | 0.0414 (n=1/2) | 19.9 |
| 370 | 0.428 | 63.7 | 0.00990 (0.00149, n=2) | 23.0 (0.470, n=2) |
| 371 | 0.27 | 62.5 | 0.0142 (0.00333, n=2) | 21.7 (0.711, n=2) |
| 372 | 0.379 | 69.1 | 0.00981 (0.00630, n=2) | 32.9 (1.65, n=2) |
| 373 | 0.336 | 65.6 | 0.00954 (0.00348, n=2) | 22.9 (5.88, n=2) |
| 374 | 0.345 | 67.1 | 0.0218 | 34.7 |
| 375 | 0.419 | 70.4 | 0.0114 | 24.2 |
| 376 | 0.326 | 72.6 | 0.0123 | 25.6 |
| 377 | 0.356 | 68.4 | 0.00532 | 16.7 |
| 378 | 0.359 | 68.6 | >10.2 | ND |
| 379 | 0.239 | 71 | 0.0181 | 31.9 |
| 380 | 0.188 | 66.8 | 0.0137 | 35 |
| 381 | 0.273 | 73.6 | 0.0155 | 25.8 |
| Comparator Tirzepatide | 0.442 (se=0.0311, n=9( | 62.9 (se=1.28, n=9) | >10.5 (n=1/5) | ND |

| **Example** | **hGLPIR B-Arrestin2 Rel EC50 uM (SEM, n)** | **hGLPIR B-Arrestin2 % Top (SEM, n)** |
|---|---|---|
| | 0.00274 (0.000359, n=42) | 104 (3.45, n=42) |
| 1 | 0.005 | 105 |
| 2 | 0.00882 (0.00269, n=2) | 96.1 (0.742, n=2) |
| 3 | | |
| 4 | 0.00480 (0.000138, n=2) | 92.4 (14.0, n=2) |
| 266 | 0.016 | 17.1 |
| 267 | 0.00901 | 16.5 |
| 272 | >12.0 | ND |
| 300 | 0.0054 | 38.4 |
| 301 | 0.00267 | 93.7 |
| 302 | 0.00219 | 98.4 |
| 303 | 0.0179 | 47.7 |
| 315 | 0.0199 | 25.4 |
| 316 | 0.00608 | 26.1 |
| **288** | 0.00674 (0.00278, n=3) | 21.3 (1.94, n=3) |
| **289** | 0.0124 (0.00151, n=3) | 51.3 (6.05, n=3) |
| 327 | 0.00379 | 41.6 |
| | | |
| 329 | 0.00473 | 63.4 |
| 330 | >10.9 | ND |
| 331 | 0.0128 | 44.3 |
| 332 | 0.0171 | 45.6 |
| 333 | 0.0548 | 58.2 |
| 334 | >10.3 | ND |
| 335 | 0.00531 | 73 |
| 336 | 0.00778 | 82.5 |
| **290** | 0.0238 (0.00530, n=4) | 20.0 (1.15, n=4) |
| 337 | 0.0174 | 34.1 |
| 338 | 0.0189 | 47 |
| 339 | 0.0107 (0.00238, n=2) | 44.0 (2.40, n=2) |
| 340 | 0.0107 (0.00711, n=4) | 20.1 (2.31, n=4) |
| 341 | 0.0188 | 21.9 |
| 342 | 0.035 | 20.7 |
| 343 | 0.0455 | 41.4 |
| 344 | 0.0343 | 46 |
| 345 | 0.00308 (0.000666, n=2) | 74.7 (2.86, n=2) |
| 346 | 0.00325 (0.00105, n=3) | 19.3 (0.767, n=3) |
| 347 | 0.00542 (0.00152, n=2) | 24.6 (2.81, n=2) |
| 348 | 0.00346 (n=1/2) | 19.8 |
| 349 | 0.0151 (0.00111, n=2) | 18.4 (0.337, n=2) |
| 350 | 0.0108 | 21.8 |
| 351 | 0.0196 | 23.5 |
| 352 | 0.00421 (n=1/2) | 17.4 |
| 353 | 0.559 (0.108, n=2) | 23.0 (0.408, n=2) |
| **291** | >10.0 (n=1/4) | ND |
| 354 | 0.255 (n=1/2) | 22.7 |
| 355 | 0.363 | 18.3 |
| 356 | >10.5 | ND |
| 357 | 0.118 | 18.2 |
| 358 | 0.00938 (0.00413, n=3) | 21.4 (2.47, n=3) |
| 359 | 0.0841 | 28.4 |
| 360 | 0.0395 | 25.8 |
| 361 | 0.269 (n=1/2) | 20.2 |
| 362 | >11.0 | ND |
| 363 | 0.135 | 22.1 |
| 364 | >12.0 | ND |
| **292** | 0.00774 (0.00199, n=4) | 29.0 (3.96, n=4) |
| **293** | 0.00719 (0.00168, n=4) | 29.5 (6.23, n=4) |
| 365 | 0.00703 (0.00233, n=4) | 20.8 (1.78, n=4) |
| 366 | 0.00557 (0.00363, n=2) | 21.2 (0.163, n=2) |
| 367 | 0.00666 (0.00118, n=2) | 70.6 (8.61, n=2) |
| 368 | 0.0479 (0.0205, n=2) | 21.3 (4.03, n=2) |
| 369 | 0.0414 (n=1/2) | 19.9 |
| 370 | 0.00990 (0.00149, n=2) | 23.0 (0.470, n=2) |
| 371 | 0.0142 (0.00333, n=2) | 21.7 (0.711, n=2) |
| 372 | 0.00981 (0.00630, n=2) | 32.9 (1.65, n=2) |
| 373 | 0.00954 (0.00348, n=2) | 22.9 (5.88, n=2) |
| 374 | 0.0218 | 34.7 |
| 375 | 0.0114 | 24.2 |
| 376 | 0.0123 | 25.6 |
| 377 | 0.00532 | 16.7 |
| 378 | >10.2 | ND |
| 379 | 0.0181 | 31.9 |
| 380 | 0.0137 | 35 |
| 381 | 0.0155 | 25.8 |

### Composition for Oral Administration

A peptide is dissolved in Tris buffer (pH 8.0, 50 mM). A Permeation enhancer ("PE") is prepared as follows: C10 is dissolved in Tris buffer (pH 8.0, 50 mM), LC, DPC, C12-maltoside and Rhamnolipid are each dissolved in phosphate buffered saline ("PBS") (1X, pH 7.2). A solution of peptide, a PE, and a protease inhibitor is mixed to reach a final peptide concentration of 300 uM, PE at 100 mM (5% w/v for Rhamnolipid) and 1% (v/v) for the protease inhibitor. .

A peptide is incubated at 37 °C in 1% (v/v) rat small intestinal fluid or 50% (v/v) pig small intestinal fluid with and without a peptidase inhibitor. At different time points, samples are taken out, followed by quenching with 1% TFA in 50% ACN/water to stop the enzyme activity. The intact peptide at different time points is analyzed by high-performance liquid chromatography (HPLC) equipped with an ultraviolet (UV) detector or LC-MS/MS and normalized to the amount of peptide before mixing with the enzyme solution. A study using a petide of Example 2 and a peptide of Example 4 are reported in **Table 15**.

**Table 15.**

| | % peptide intact | | | | |
|---|---|---|---|---|---|
| Recombinant protease inhibitor (concentration) | Small intestinal fluid | 0 min | 15 min | 30 min | 60 min |
| rSBTI (5 mg/mL) + Peptide (Example 4) | 50% v/v pig | 100.00 | 96.96 | 96.28 | 88.57 |
| Peptide Example 4 (no PI; control) | 50% v/v pig | 100.00 | 72.52 | 41.44 | 18.98 |
| rSBTI (5 mg/mL) + Peptide Example 2 | 50% v/v pig | 100.00 | 103.02 | 112.69 | 87.33 |
| Peptide Example 2 (no PI; control) | 50% v/v pig | 100.00 | 2.42 | 1.90 | 3.09 |
| rSBTCI (0.5 mg/mL) + Peptide Example 2 | 50% v/v pig | 100.00 | 131.71 | 126.53 | 123.70 |

Table 15 results support that an oral formulation composition for a peptide of Example 4 may be prepared using a PE and no PI.

### Oral Formulation Composition

Examples of formulation compositions for a peptide of this invention are provided by Table 16. The formulation compositions for peptides of this invention are in no way limited by the examples provided.

**Table 16**

| Formulation | Formulation composition | Concentration |
|---|---|---|
| 1 | Peptide (Example 1; or Example 4 or Example 3) | 2.4 mg/mL |
| | C10 | 250 mM |
| | SBTI | 75 mg/mL |
| 2 | Peptide (Example 1) | 2.4 mg/mL |
| | LC | 500 mM |
| | Citric acid | 500 mM |
| 3 | Peptide (Example 1) | 2.4 mg/mL |
| | NaTDC | 250 mM |
| | SBTI | 75 mg/mL |
| 4 | Peptide (Example 1, Example 2, or Example 4) | 2.4 mg/mL |
| | C10 | 250 mM |
| | SBTI | 12 mg/mL |
| 5 | Peptide (Example 1 or Example 2) | 2.4 mg/mL |
| | C10 | 125 mM |
| | SBTI | 12 mg/mL |
| | Peptide (Example 1) | 2.4 mg/mL |
| 6 | C10 | 125 mM |
| | SBTI | 24 mg/mL |
| 7 | Peptide (Example 4) | 2.4 mg/mL |
| | C10 | 250 mM |
| | SFTI | 12 mg/mL |

The effect of formulation composition on a peptide exposure is evaluated in rats via intrajejunal (IJ) administration using liquid formulations. To prepare liquid formulations for a rat IJ administration, a peptide, C10 or NaTDC and SBTI is dissolved in 50 mM Tris buffer pH 8.0 and mixed to achieve final desired concentration. For LC/citric acid formulation, LC and citric acid are dissolved in water and mixed with a peptide dissolved in Tris buffer. Formulation compositions provided in Table 16 may be administered as an oral composition.

### Enteric Capsules

An enteric capsule composition may be desired for certain peptides of this invention and may be prepared using methods for example, as set forth by Table 17. Enteric compositions may be prepared by blending ingredients together and filling the blend in enteric capsules.
An enteric composition of Table 17 is prepared adding half of the stated amount of sodium decanoate to a mortar. SBTI (for Examples 382-385) or SFTI (for Examples 386 and 387), and a peptide (peptides of Examples 1-4), as shown in Table 17. A remaining half of the sodium decanoate is added. A mixture is gently blended together using pestle, and spatula. If desired, additional mixing using pestle provides a homogenous blend. A capsule may be manually filled by individually weighing the required amount of blend, filling in capsules, and securely closing the capsule caps to the capsule bodies.
Dissolution testing of a single capsule is completed using known methods. A peptide of this invention may be formulated as an entric oral composition.

**Table 17: Composition of Individual Enteric Capsule for Formulation**

| **PeptideComponent** | | | | | | |
|---|---|---|---|---|---|---|
| | **Enteric Example 382** | **Enteric Example 383** | **Enteric Example 384** | **Enteric Example 385** | **Enteric Example 386** | **Enteric Example 387** |
| **Example 2** | **12.50** | | | | **12.50** | |
| **Example 4** | | **12.50** | | | | **12.50** |
| **Example 1** | | | **12.50** | | | |
| **Example 3** | | | | **12.50** | | |
| **Sodium decanoate (C10)** | **250.00** | **250.00** | **250.00** | **250.00** | **250.00** | **250.00** |
| **SBTI** | **62.50** | **62.50** | **62.50** | **62.50** | | |
| **SFTI** | | | | | **62.50** | **62.50** |
| **Total Capsule Fill Weight** | **325.00** | **325.00** | **325.00** | **325.00** | **325.00** | **325.00** |
| **Capsule Size** | **Size 00** | **Size 00** | **Size 00** | **Size 00** | **Size 00** | **Size 00** |

### Amino Acid Sequences

SEQ ID NO:1
   GIP (Human)
   YAEGTFISDYSIAMDKIHQQDFVNWLLAQKGKKNDWKHNITQ
SEQ ID NO:2
   GLP-1 (7-36) (Human)
   HAEGTFTSDVSSYLEGQAAKEFIAWLVKGR-NH₂
SEQ ID NO:3 
R1X1 X2 X3GT X6TSD X10 X11 X12 X13 X14D X16X17AX19 X20 X21 X22X23 X24 X25 X26 X27
X28 X29 X30X31
SEQ **ID** NO:4
   PX₃₂ X₃₃ X₃₄-R₂
SEQ ID NO:5
   PX₃₂X₃₃X₃₄X₃₅X₃₆X₃₇X₃₈X₃₉-R₂
SEQ ID NO:6
   PX₃₂ X₃₃ X₃₄ X₃₅X₃₆ X₃₇ X₃₈ X₃₉ X₄₀-R₂
SEQ ID NO:7
   K[(2-[2-(2-Amino-ethoxy)-ethoxy]-acetyl)₂-(γ-Glu)-CO-(CH₂)_{q}-CO₂H] X₃₂ X₃₃ X₃₄-R₂
SEQ ID NO:8 
K[(2-[2-(2-Amino-ethoxy)-ethoxy]-acetyl)2-(γ-Glu)-CO-(CH2)q-CO2H] X32 X33 X34
X35X36 X37 X38 X39-R2
SEQ ID NO:9 
K[(2-[2-(2-Amino-ethoxy)-ethoxy]-acetyl)2-(γ-Glu)-CO-(CH2)q-CO2H] X32 X33 X34
X35X36 X37 X38 X39 X40-R2
SEQ ID NO:10
   **Example 1** 
Y-Aib-EGT-αMeF(2F)-TSDYSI-αMeL-LDEK((2-[2-(2-Amino-ethoxy)-ethoxy]-acetyl)2-
(γ-Glu)-CO-(CH2)18-CO2H)AQ-Aib-EFI-(D-Glu)-YLIEGGPSSGAPPPS-NH2
SEQ ID NO:11
   **Example 2** 
Y-Aib-EGT-αMeF(2F)-TSDYSI-αMeL-LD-Orn-K((2-[2-(2-Amino-ethoxy)-
ethoxy]-acetyl)2-(γ-Glu)-CO-(CH2)16-CO2H)AQ-Aib-EFI-(D-Glu)-
YLIEGGPSSGAPPPS-NH2
SEQ ID NO:12
   **Example 3** 
Y-Aib-EGT-αMeF(2F)-TSDYSI-αMeL-LD-Orn-K((2-[2-(2-Amino-ethoxy)-ethoxy]-
acetyl)2-(γ-Glu)-CO-(CH2)18-CO2H)AQ-Aib-EFI-(D-Glu)-YLIEGGPSSGAPPPS-NH2
SEQ ID NO:13
   **Example 4** 
Y-Aib-EGT-αMeF(2F)-TSD-4Pal-SI-αMeL-LD-Orn-K((2-[2-(2-Amino-ethoxy)-ethoxy]-
acetyl)2-(γ-Glu)-CO-(CH2)16-CO2H)AQ-Aib-EFI-(D-Glu)-αMeY-LIEGGPSSGAPPPS-
NH2
SEQ ID NO:14
   **Example 5** 
Y-Aib-EGT-αMeF(2F)-TSDVSI-αMeL-LD-Orn-K((2-[2-(2-Amino-ethoxy)-ethoxy]-
acetyl)2-(γ-Glu)-CO-(CH2)16-CO2H)AQ-Aib-EFI-(D-Glu)-αMeY-LIEGGPSSGAPPPS-
NH2
SEQ ID NO:297
   PSSG-R₂
SEQ ID NO:298
   PSSGAPPPS-R₂
SEQ ID NO:299
   PSSG
SEQ ID NO:300
   PSSG-NH₂
SEQ ID NO:301
   PSSGAPPPS
SEQ ID NO:302
   PSSGAPPPS-NH₂

### NUMBERED EMBODIMENTS

1. A compound of the formula:
   R1X1X2X3GTX6TSDX10X11X12X13X14DX16X17AX19X20X21X22X23X24X25X26X27 X₂₈X₂₉X₃₀X₃₁ (SEQ ID NO:3) wherein:
   R₁ is a modification of the N-terminal amino group wherein the modification is selected from the group consisting of Ac and absent;
   X₁ is selected from the group consisting of Y, H, D-Tyr, F, desH, and desY;
   X₂ is selected from the group consisting of Aib, αMeP, A, P, and D-Ala_{;}
   or X₁ and X₂ combine to form desH-*ψ*[NHCO]-Aib;
   X₃ is selected from the group consisting of E, N, Aad, and cTA;
   X₆ is selected from the group consisting of F, αMeF, and αMeF(2F);
   X₁₀ is selected from the group consisting of A, L, H, 3Pal, 4Pal, V, Y, E, αMeF, αMeF(2F), I, αMeY, Q, D-His, D-Tyr, cTA, and K(2-[2-(2-amino-ethoxy)-ethoxy]-acetyl)₂-(γ-Glu)-CO-(CH₂)qCO₂H;
   X₁₁ is selected from the group consisting of S, αMeS, and D-Ser;
   X₁₂ is selected from the group consisting of I, S, D-Ile, and K(2-[2-(2-amino-ethoxy)-ethoxy]-acetyl)₂-(γ-Glu)-CO-(CH₂)qCO₂H;
   X₁₃ is selected from the group consisting of Nle, Aib, L, αMeL, and K(2-[2-(2-amino-ethoxy)-ethoxy]-acetyl)₂-(γ-Glu)-CO-(CH₂)qCO₂H;
   X₁₄ is selected from the group consisting of L and K, wherein K is conjugated to a C₁₆-C₂₂ fatty acid wherein said fatty acid is optionally conjugated to said K via a linker;
   X₁₆ is selected from the group consisting of K, E, Orn, Dab, Dap, S, T, H, Aib, αMeK, R, and K(2-[2-(2-amino-ethoxy)-ethoxy]-acetyl)₂-(γ-Glu)-CO-(CH₂)qCO₂H;
   X₁₇ is selected from the group consisting of K, Q, I, and an amino acid conjugated to a C₁₆-C₂₂ fatty acid wherein said fatty acid is optionally conjugated to said amino acid via a linker;
   X₁₉ is selected from the group consisting of Q, A, and K(2-[2-(2-amino-ethoxy)-ethoxy]-acetyl)₂-(γ-Glu)-CO-(CH₂)qCO₂H;
   X₂₀ is selected from the group consisting of Aib, Q, H, R, K, αMeK, and K(2-[2-(2-amino-ethoxy)-ethoxy]-acetyl)₂-(γ-Glu)-CO-(CH₂)qCO₂H;
   X₂₁ is selected from the group consisting of H, Aad, D, Aib, T, A, E, I, and K(2-[2-(2-amino-ethoxy)-ethoxy]-acetyl)₂-(γ-Glu)-CO-(CH₂)qCO₂H;
   X₂₂ is selected from the group consisting of F and αMeF;
   X₂₃ is selected from the group consisting of I, L, A, G, F, H, E, V, and K(2-[2-(2-amino-ethoxy)-ethoxy]-acetyl)₂-(γ-Glu)-CO-(CH₂)qCO₂H;
   X₂₄ is selected from the group consisting of S, Aad, D-Glu, E, Aib, H, V, A, Q, D, P, and K(2-[2-(2-amino-ethoxy)-ethoxy]-acetyl)₂-(γ-Glu)-CO-(CH₂)qCO₂H;
   X₂₅ is selected from the group consisting of Y and αMeY;
   X₂₆ is selected from the group consisting of L, αMeL, and K(2-[2-(2-amino-ethoxy)-ethoxy]-acetyl)₂-(γ-Glu)-CO-(CH₂)qCO₂H;
   X₂₇ is selected from the group consisting of L, I, and K(2-[2-(2-amino-ethoxy)-ethoxy]-acetyl)₂-(γ-Glu)-CO-(CH₂)qCO₂H;
   X₂₈ is selected from the group consisting of E, A, S, D-Glu, and K(2-[2-(2-amino-ethoxy)-ethoxy]-acetyl)₂-(γ-Glu)-CO-(CH₂)qCO₂H;
   X₂₉ is selected from the group consisting of Aib, G, A, and K(2-[2-(2-amino-ethoxy)-ethoxy]-acetyl)₂-(γ-Glu)-CO-(CH₂)qCO₂H;
   X₃₀ is selected from the group consisting of C, G, G-R₂ and K(2-[2-(2-amino-ethoxy)-ethoxy]-acetyl)₂-(γ-Glu)-CO-(CH₂)_{q}-CO₂H;
   X₃₁ is absent or is selected from the group consisting of PX₃₂X₃₃X₃₄-R₂ (SEQ ID NO:4), PX₃₂X₃₃X₃₄X₃₅X₃₆X₃₇X₃₈X₃₉-R₂ (SEQ ID NO:5),
   PX₃₂X₃₃X₃₄X₃₅X₃₆X₃₇X₃₈X₃₉X₄₀-R₂ (SEQ ID NO:6)_{,} K[(2-[2-(2-amino-ethoxy)-ethoxy]-acetyl)₂-(γ-Glu)-CO-(CH2)q-CO2H] X₃₂X₃₃X₃₄-R₂ (SEQ ID NO:7)_{,} K[(2-[2-(2-amino-ethoxy)-ethoxy]-acetyl)₂-(γ-Glu)-CO-(CH₂)_{q}-CO₂H]
   X₃₂X₃₃X₃₄X₃₅X₃₆X₃₇X₃₈X₃₉-R₂ (SEQ ID NO:8)_{,} and K[(2-[2-(2-amino-ethoxy)-ethoxy]-acetyl)₂-(γ-Glu)-CO-(CH2)q-CO2H] X₃₂X₃₃X₃₄X₃₅X₃₆X₃₇X₃₈X₃₉X₄₀-R₂ (SEQ ID NO:9); wherein:
      X₃₂ is S or K[(2-[2-(2-amino-ethoxy)-ethoxy]-acetyl)₂-(γ-Glu)-CO-(CH₂)_{q}-CO₂H];
      X₃₃ is S or K[(2-[2-(2-amino-ethoxy)-ethoxy]-acetyl)₂-(γ-Glu)-CO-(CH₂)_{q}-CO₂H];
      X₃₄ is selected from the group consisting of G, C, and K[(2-[2-(2-amino-ethoxy)-ethoxy]-acetyl)₂-(γ-Glu)-CO-(CH₂)_{q}-CO₂H];
      X₃₅ is A or K[(2-[2-(2-amino-ethoxy)-ethoxy]-acetyl)₂-(γ-Glu)-CO-(CH₂)_{q}-CO₂H];
      X₃₆ is P or K[(2-[2-(2-amino-ethoxy)-ethoxy]-acetyl)₂-(γ-Glu)-CO-(CH₂)_{q}-CO₂H];
      X₃₇ is P or K[(2-[2-(2-amino-ethoxy)-ethoxy]-acetyl)₂-(γ-Glu)-CO-(CH₂)_{q}-CO₂H];
      X₃₈ is P or K[(2-[2-(2-amino-ethoxy)-ethoxy]-acetyl)₂-(γ-Glu)-CO-(CH₂)_{q}-CO₂H];
      X₃₉ is selected from the group consisting of C, S, and K[(2-[2-(2-amino-ethoxy)-ethoxy]-acetyl)₂-(γ-Glu)-CO-(CH₂)_{q}-CO₂H];
      X₄₀ is selected from the group consisting of C and K[(2-[2-(2-amino-ethoxy)-ethoxy]-acetyl)₂-(γ-Glu)-CO-(CH₂)_{q}-CO₂H];
   q is selected from the group consisting of 14, 15, 16, 17, 18, 19, and 20; and R₂ is a modification of the C-terminal group, wherein the modification is NH₂ or absent;
   or a pharmaceutically acceptable salt thereof;
   wherein if X₃₀ is G-R₂, then X₃₁ is absent;
   wherein no more than one of X₁₀, X₁₂, X₁₃, X₁₄, X₁₆, X₁₇, X₁₉, X₂₀, X₂₁, X_{23,} X₂₄, X₂₆, X₂₇, X₂₈, X₂₉, X₃₀, X₃₁, X₃₂, X₃₃, X₃₄, X₃₅, X₃₆, X₃₇, X₃₈, X₃₉, and X₄₀ may be a substituent that contains a fatty acid; and
   wherein no more than one of X_{30,} X_{34,} X_{39,} and X₄₀ may be C; and
   wherein if one of X_{30,} X_{34,} X_{39,} and X₄₀ is C, then none of X₁₀, X₁₂, X₁₃, X₁₄, X₁₆, X17, X19, X20, X21, X23, X24, X26, X27, X28, X29, X30, X31, X32, X33, X34, X35, X36, X37, X_{38,} X_{39,} and X₄₀ is a substituent that contains a fatty acid.
2. A compound, or a pharmaceutically acceptable salt thereof, according to Embodiment 1 wherein X₁ and X₂ do not combine to form desH-*ψ*[NHCO]-Aib.
3. A compound, or a pharmaceutically acceptable salt thereof, according to Embodiment 1 or Embodiment 2 wherein X₁₇ is an amino acid conjugated to a C₁₆-C₂₂ fatty acid.
4. A compound, or a pharmaceutically acceptable salt thereof, according to any one of Embodiments 1 to 3 wherein:
   X₁ is Y;
   X₂ is Aib;
   X₃ is E;
   X₁₀ is selected from the group consisting of A, L, H, 3Pal, 4Pal, V, and Y;
   X₁₁ is S;
   X₁₂ is I;
   X₁₄ is L;
   X₁₆ is selected from the group consisting of K, E, Orn, Dab, and Dap;
   X₁₇ is K conjugated to a C₁₆-C₂₂ fatty acid wherein said fatty acid is conjugated to K via a linker;
   X₁₉ is Q;
   X₂₀ is Aib;
   X₂₁ is selected from the group consisting of H, Aad, D, Aib, T, A, and E;
   X₂₂ is F;
   X₂₃ is I;
   X₂₄ is selected from the group consisting of S, Aad, D-Glu, and E;
   X₂₆ is L;
   X₂₇ is selected from the group consisting of L and I; and
   X₂₈ is selected from the group consisting of E and A.
5. A compound, or a pharmaceutically acceptable salt thereof, according to any one of Embodiments 1 to 4 wherein: 
X30 is G and X31 is PX32X33X34X35X36X37X38X39-R2 (SEQ ID NO:5), wherein X32
is S, X33 is S, X34 is G, X35 is A, X36 is P, X37 is P, X38 is P and X39 is S (SEQ ID
NO:298).
6. A compound, or a pharmaceutically acceptable salt thereof, according to any one of Embodiments 1, 2 or 5 wherein X₁₇ is K.
7. A compound, or a pharmaceutically acceptable salt thereof, according to embodiment 4 wherein the linker comprises one to two amino acids.
8. A compound, or a pharmaceutically acceptable salt thereof, according to embodiment 7 wherein the linker comprises from one to two (2-[2-(2-Amino-ethoxy)-ethoxy]-acetyl) moieties.
9. A compound, or a pharmaceutically acceptable salt thereof, according to embodiment 8 wherein the linker comprises one to two amino acids independently selected from the group consisting of Glu and γ-Glu.
10. A compound, or a pharmaceutically acceptable salt thereof, according to embodiment 9 wherein the linker has the following formula:
   {(2-[2-(2-Amino-ethoxy)-ethoxy]-acetyl)ₐ-(γ-Glu)_{b}, wherein a is 1 or 2; and b is 1 or 2.
11. A compound, or a pharmaceutically acceptable salt thereof, according to any one of embodiments 1 to 10 wherein X₁₇ is K(2-[2-(2-Amino-ethoxy)-ethoxy]-acetyl)ₐ-(γ-Glu)_{b}-CO-(CH₂)_{q}-CO₂H, wherein: a is 1 or 2; b is 1 or 2; and q is selected from the group consisting of 14 to 20.
12. A compound, or a pharmaceutically acceptable salt thereof, according to embodiment 11 wherein a is 1.
13. A compound, or a pharmaceutically acceptable salt thereof, according to embodiment 11 wherein a is 2.
14. A compound, or a pharmaceutically acceptable salt thereof, according to any one of embodiments 11 to 13 wherein b is 1.
15. A compound, or a pharmaceutically acceptable salt thereof, according to any one of embodiments 11 to 13 wherein b is 2.
16. A compound, or a pharmaceutically acceptable salt thereof, according to any one of embodiments 11 to 15 wherein q is 18.
17. A compound, or a pharmaceutically acceptable salt thereof, according to any one of Embodiments 11 to 15 wherein q is 16.
18. A compound, or a pharmaceutically acceptable salt thereof, according to any one of Embodiments 1 to 11 wherein X₁₇ is K(2-[2-(2-amino-ethoxy)-ethoxy]-acetyl)₂-(γ-Glu)-CO-(CH₂)_{q}-CO₂H.
19. A compound, or a pharmaceutically acceptable salt thereof, according to any one of Embodiments 1 to 18 wherein X₂₇ is I.
20. A compound, or a pharmaceutically acceptable salt thereof, according to any one of Embodiments 1 to 18 wherein X₂₇ is L.
21. A compound, or a pharmaceutically acceptable salt thereof, according to any one of Embodiments 1 to 19 wherein:
   R₁ is absent;
   X₁ is Y;
   X₂ is Aib;
   X₃ is E;
   X₆ is αMeF(2F);
   X₁₀ is selected from the group consisting of Y, 4-PaI, and V;
   X₁₁ is S;
   X₁₂ is I;
   X₁₃ is selected from the group consisting of L, Aib, and αMeL;
   X₁₄ is L;
   X₁₆ is selected from the group consisting of E, K, and Orn;
   X₁₇ is K;
   X₁₉ is Q;
   X₂₀ is Aib
   X₂₁ is selected from the group consisting of E, A, and T;
   X₂₂ is F;
   X₂₃ is I;
   X₂₄ is D-Glu;
   X₂₆ is L;
   X₂₇ is I;
   X₂₈ is E;
   X₂₉ is G;
   X₃₀ is G; and
   X₃₁ is PX₃₂X₃₃X₃₄X₃₅X₃₆X₃₇X₃₈X₃₉-R₂ (SEQ ID NO:5)_{,} wherein X₃₂ is S, X₃₃ is S,
   X₃₄ is G, X₃₅ is A, X₃₆ is P, X₃₇ is P, X₃₈ is P and X₃₉ is S (SEQ ID NO:298).
22. A compound, or a pharmaceutically acceptable salt thereof, according to any one of Embodiments 1 to 21 wherein X₂₅ is Y.
23. A compound, or a pharmaceutically acceptable salt thereof, according to any one of Embodiments 1 to 22 wherein X₁₃ is αMeL.
24. A compound, or a pharmaceutically acceptable salt thereof, according to any one of embodiments 1 to 23 wherein X₁₆ is Orn.
25. A compound, or a pharmaceutically acceptable salt thereof, according to any one of embodiments 1 to 23 wherein X₁₆ is E.
26. A compound, or a pharmaceutically acceptable salt thereof, according to any one of embodiments 1 to 25 wherein X₁₀ is Y.
27. A compound, or a pharmaceutically acceptable salt thereof, according to any one of embodiments 1 to 25 wherein X₁₀ is V.
28. A compound, or a pharmaceutically acceptable salt thereof, according to any one of embodiments 1 to 25 wherein X₁₀ is 4Pal.
29. A compound, or a pharmaceutically acceptable salt thereof, according to Embodiment 11 wherein X₁₃ is αMeL; a is 2; b is 1; q is 16; and X₂₅ is Y.
30. A compound, or a pharmaceutically acceptable salt thereof, according to Embodiment 11 wherein X₁₃ is αMeL; a is 2; b is 1; q is 18; and X₂₅ is Y.
31. A compound, or a pharmaceutically acceptable salt thereof, according to Embodiment 11 wherein X₁₃ is αMeL; a is 2; b is 1; q is 14; and X₂₅ is Y.
32. A compound, or a pharmaceutically acceptable salt thereof, according to any one of embodiments 1 to 24 wherein X₁₀ is Y; and X₁₆ is Orn.
33. A compound, or a pharmaceutically acceptable salt thereof, according to any one of embodiments 1 to 23 wherein X₁₀ is Y; and X₁₆ is E.
34. A compound, or a pharmaceutically acceptable salt thereof, according to any one of embodiments 1 to 23 wherein X₁₀ is 4Pal; and X₁₆ is Orn.
35. A compound, or a pharmaceutically acceptable salt thereof, according to any one of embodiments 1 to 23 wherein X₁₀ is V; and X₁₆ is Orn.
36. A compound according to Embodiment 1 wherein the compound is selected from the group consisting of SEQ ID NO:10, SEQ ID NO:11, SEQ ID NO:12, SEQ ID NO:13, and SEQ ID NO:14, or a pharmaceutically acceptable salt thereof.
37. A compound according to Embodiment 36 wherein the compound is SEQ ID NO:13, or a pharmaceutically acceptable salt thereof.
38. A compound according to Embodiment 36 wherein the compound is SEQ ID NO:11, or a pharmaceutically acceptable salt thereof.
39. A compound, or a pharmaceutically acceptable salt thereof according to Embodiment 1
   wherein:
   X₁₀ is selected from the group consisting of A, L, H, 3Pal, 4Pal, V, Y, αMeF, αMeF(2F), I, αMeY, Q, D-His, E, cTA, and D-Tyr;
   X₁₂ is selected from the group consisting of I, D-Ile, and S;
   X₁₃ is selected from the group consisting of Nle, Aib, L, and αMeL;
   X₁₄ is L;
   X₁₆ is selected from the group consisting of K, E, Orn, Dab, Dap, S, T, H, Aib, αMeK, and R;
   X₁₇ is selected from the group consisting of K, Q, and I;
   X₁₉ is selected from the group consisting of Q and A;
   X₂₀ is selected from the group consisting of Aib, Q, H, R, K, and αMeK;
   X₂₁ is selected from the group consisting of H, Aad, D, Aib, T, A, E, and I;
   X₂₃ is selected from the group consisting of I, L, A, G, F, H, E, and V;
   X₂₄ is selected from the group consisting of S, Aad, D-Glu, E, Aib, H, V, A, Q, D, and P;
   X₂₆ is selected from the group consisting of L and αMeL;
   X₂₇ is selected from the group consisting of L and I;
   X₂₈ is selected from the group consisting of E, A, S, and D-Glu; and
   X₂₉ is selected from the group consisting of Aib, G, and A.
40. A compound, or a pharmaceutically acceptable salt thereof according to Embodiment 39 wherein:
   X₁ is Y;
   X₂ is Aib;
   X₃ is E;
   X₁₀ is selected from the group consisting of A, L, H, 3Pal, 4Pal, V, and Y;
   X₁₁ is S;
   X₁₂ is I;
   X₁₆ is selected from the group consisting of K, E, Orn, Dab, and Dap;
   X₁₉ is Q;
   X₂₀ is selected from the group consisting of Aib and K;
   X₂₁ is selected from the group consisting of H, Aad, D, Aib, T, A, and E;
   X₂₂ is F;
   X₂₃ is I;
   X₂₄ is selected from the group consisting of S, Aad, D-Glu, and E;
   X₂₆ is L; and
   X₂₈ is selected from the group consisting of E and A.
41. A compound, or a pharmaceutically acceptable salt thereof, according to any one of Embodiments 39 or 40 wherein one of X₃₀, X₃₄, and X₃₉ is C.
42. A compound, or a pharmaceutically acceptable salt thereof, according to Embodiment 41 wherein the compound is modified using time-extension technology.
43. A compound, or a pharmaceutically acceptable salt thereof, according to any one of Embodiments 39 to 42 wherein q is 18.
44. A compound, or a pharmaceutically acceptable salt thereof, according to any one of Embodiments 39 to 42 wherein q is 16.
45. A compound, or a pharmaceutically acceptable salt thereof, according to any one of Embodiments 39 to 44 wherein X₂₇ is L.
46. A compound, or a pharmaceutically acceptable salt thereof, according to any one of Embodiments 39 to 45 wherein X₃₁ is PX₃₂X₃₃X₃₄X₃₅X₃₆X₃₇X₃₈X₃₉-R₂.
47. A compound, or a pharmaceutically acceptable salt thereof, according to any one of Embodiments 39 to 45 wherein X₃₁ is absent.
48. A compound, or a pharmaceutically acceptable salt thereof, according to Embodiment 47 wherein R₂ is NH₂.
49. A compound, or a pharmaceutically acceptable salt thereof, according to Embodiment 1
   wherein:
   X₁₄ is L;
   X₁₇ is selected from the group consisting of K, Q, and I;
   X₃₀ is selected from the group consisting of G-R₂ and G; and
   q is selected from the group consisting of 16, 18, and 20;
   wherein if X₃₀ is G, then X₃₁ is selected from the group consisting of:
      PX₃₂X₃₃X₃₄-R₂ (SEQ ID NO:4), wherein:
      X₃₂ is S, X₃₃ is S, X₃₄ is G and R₂ is absent (SEQ ID NO:299) or
      X₃₂ is S, X₃₃ is S, X₃₄ is G and R₂ is NH₂ (SEQ ID NO:300);
         and
      PX₃₂X₃₃X₃₄X₃₅X₃₆X₃₇X₃₈X₃₉-R₂ (SEQ ID NO:5), wherein:
         X₃₂ is S, X₃₃ is S, X₃₄ is G, X₃₅ is A, X₃₆ is P, X₃₇ is P, X₃₈ is P, X₃₉ is S and R₂ is absent (SEQ ID NO:301) or
         X₃₂ is S, X₃₃ is S, X₃₄ is G, X₃₅ is A, X₃₆ is P, X₃₇ is P, X₃₈ is P, X₃₉ is S and R₂ is NH₂ (SEQ ID NO:302); and
   wherein one of X₁₀, X₁₂, X₁₃, X_{14,} X₁₆, X₁₉, X₂₀, X₂₁, X_{23,} X₂₄, X₂₆, X₂₇, X₂₈, and
   X₂₉ is K(2-[2-(2-amino-ethoxy)-ethoxy]-acetyl)₂-γGlu-CO-(CH₂)qCO₂H.
50. A compound, or a pharmaceutically acceptable salt thereof, according to Embodiment 49 wherein:
   X₁ is Y;
   X₂ is Aib;
   X₃ is E;
   X₁₀ is selected from the group consisting of A, L, H, 3Pal, 4Pal, V, Y, E, cTA, and K(2-[2-(2-amino-ethoxy)-ethoxy]-acetyl)₂-(γ-Glu)-CO-(CH₂)qCO₂H;
   X₁₁ is S;
   X₁₂ is selected from the group consisting of I, D-Ile, and K(2-[2-(2-amino-ethoxy)-ethoxy]-acetyl)₂-(γ-Glu)-CO-(CH₂)qCO₂H;
   X₁₄ is selected from the group consisting of L and K(2-[2-(2-amino-ethoxy)-ethoxy]-acetyl)₂-(γ-Glu)-CO-(CH₂)qCO₂H;
   X₁₆ is selected from the group consisting of K, E, Orn, Dab, Dap, and K(2-[2-(2-amino-ethoxy)-ethoxy]-acetyl)₂-(γ-Glu)-CO-(CH₂)qCO₂H;
   X₁₇ is selected from the group consisting of K and I;
   X₁₉ is selected from the group consisting of Q and K(2-[2-(2-amino-ethoxy)-ethoxy]-acetyl)₂-(γ-Glu)-CO-(CH₂)qCO₂H;
   X₂₀ is selected from the group consisting of Aib and K(2-[2-(2-amino-ethoxy)-ethoxy]-acetyl)₂-(γ-Glu)-CO-(CH₂)qCO₂H;
   X₂₁ is selected from the group consisting of H, Aad, D, Aib, T, A, E, and K(2-[2-(2-amino-ethoxy)-ethoxy]-acetyl)₂-(γ-Glu)-CO-(CH₂)qCO₂H;
   X₂₂ is F;
   X₂₃ is I;
   X₂₄ is selected from the group consisting of S, Aad, D--Glu, E, and K(2-[2-(2-amino-ethoxy)-ethoxy]-acetyl)₂-(γ-Glu)-CO-(CH₂)qCO₂H;
   X₂₆ is selected from the group consisting of L, and K(2-[2-(2-amino-ethoxy)-ethoxy]-acetyl)₂-(γ-Glu)-CO-(CH₂)qCO₂H;
   X₂₇ is selected from the group consisting of L and I;
   X₂₈ is selected from the group consisting of E, A, and K(2-[2-(2-amino-ethoxy)-ethoxy]-acetyl)₂-(γ-Glu)-CO-(CH₂)qCO₂H;
   X₃₀ is G; and
   X₃₁ is selected from the group consisting of PX₃₂X₃₃X₃₄-R₂ (SEQ ID NO:4), PX₃₂X₃₃X₃₄X₃₅X₃₆X₃₇X₃₈X₃₉-R₂ (SEQ ID NO:5),
      X₃₂ is S;
      X₃₃ is S;
      X₃₄ is G;
      X₃₅ is A;
      X₃₆ is P;
      X₃₇ is P;
      X₃₈ is P; and
      X₃₉ is S.
51. A compound, or a pharmaceutically acceptable salt thereof, according to any one of Embodiments 49 to 50 wherein X₂₀ is K(2-[2-(2-amino-ethoxy)-ethoxy]-acetyl)₂-(γ-Glu)-CO-(CH₂)_{q}-CO₂H.
52. A compound, or a pharmaceutically acceptable salt thereof, according to any one of Embodiments 49 to 51 wherein PX₃₂X₃₃X₃₄X₃₅X₃₆X₃₇X₃₈X₃₉-R₂ is selected from the group consisting of PSSGAPPPS (SEQ ID NO:301) and PSSGAPPPS-NH₂ (SEQ ID NO:302).
53. A compound, or a pharmaceutically acceptable salt thereof, according to any one of Embodiments 49 to 52 wherein q is 18.
54. A compound, or a pharmaceutically acceptable salt thereof, according to any one of Embodiments 49 to 52 wherein q is 16.
55. A compound, or a pharmaceutically acceptable salt thereof, according to Embodiment 1
   wherein:
   X₁ is selected from the group consisting of Y, F, D-Tyr, and desY,
   X₆ is F; and
   X₁₃ is selected from the group consisting of Aib, L, and αMeL.
56. A compound, or pharmaceutically acceptable salt thereof, according to Embodiment 55 wherein
   R₁ is absent;
   X₂ is Aib;
   X₃ is E;
   X₁₀ is Y;
   X₁₁ is S;
   X₁₂ is I;
   X₁₄ is L;
   X₁₆ is selected from the group consisting of K, E, Orn, Dab, Dap, S, T, H, Aib, αMeK, and R
   X₁₇ is an amino acid conjugated to a C₁₆-C₂₂ fatty acid wherein said fatty acid is optionally conjugated to said amino acid via a linker;
   X₁₉ is Q;
   X₂₀ is selected from the group consisting of Aib, Q, H, and K;
   X₂₁ is selected from the group consisting of H, D, T, A, and E;
   X₂₂ is F;
   X₂₃ is I;
   X₂₄ is selected from the group consisting of D-Glu and E;
   X₂₆ is L;
   X₂₇ is I;
   X₂₈ is selected from the group consisting of E, A, S, and D-Glu;
   X₂₉ is selected from the group consisting of Aib, G, and A;
   X₃₀ is selected from the group consisting of C, G, and G-R₂;
   X₃₁ is absent or is selected from the group consisting of PX₃₂X₃₃X₃₄-R₂ (SEQ ID NO:4), PX₃₂X₃₃X₃₄X₃₅X₃₆X₃₇X₃₈X₃₉-R₂ (SEQ ID NO:5)_{,} and
   PX₃₂X₃₃X₃₄X₃₅X₃₆X₃₇X₃₈X₃₉X₄₀-R₂ (SEQ ID NO:6); wherein:
      X₃₂ is S;
      X₃₃ is S;
      X₃₄ is selected from the group consisting of G and C;
      X₃₅ is A;
      X₃₆ is P;
      X₃₇ is P;
      X₃₈ is P;
      X₃₉ is selected from the group consisting of C and S; and
      X₄₀ is C.
57. A compound, or a pharmaceutically acceptable salt thereof, according to any one of Embodiments 55 to 56 wherein X₁₇ is K(2-[2-(2-amino-ethoxy)-ethoxy]-acetyl)₂-(γ-Glu)-CO-(CH₂)_{q}-CO₂H.
58. A compound, or a pharmaceutically acceptable salt thereof, according to any one of Embodiments 55 to 57 wherein PX₃₂X₃₃X₃₄X₃₅X₃₆X₃₇X₃₈X₃₉-R₂ is selected from the group consisting of PSSGAPPPS (SEQ ID NO:301) and PSSGAPPPS-NH₂ (SEQ ID NO:302).
59. A compound, or pharmaceutically acceptable salt thereof, according to any one of Embodiments 55 to 58 wherein
   X₂₈ is A;
   X₂₉ G;
   X₃₀ is G;
   X₃₁ is PX₃₂X₃₃X₃₄X₃₅X₃₆X₃₇X₃₈X₃₉-R₂ (SEQ ID NO:5)
      X₃₄ is G; and
      X₃₉ is S.
60. A compound, or pharmaceutically acceptable salt thereof, according to any one of Embodiments 55 to 59 wherein X₁ is selected from the group consisting of Y and D-Tyr; and X₁₃ is αMeL.
61. A compound, or pharmaceutically acceptable salt thereof, according to any one of Embodiments 55 to 60 wherein q is 16.
62. A compound, or pharmaceutically acceptable salt thereof, according to any one of Embodiments 55 to 60 wherein q is 18.
63. A compound, or pharmaceutically acceptable salt thereof, according to Embodiment 1 wherein the compound is selected from the group consisting of SEQ ID NO:303, SEQ ID NO:304, SEQ ID NO:305, SEQ ID NO:306, SEQ ID NO:307, SEQ ID NO:308, and SEQ ID NO:392.
64. A compound, or pharmaceutically acceptable salt thereof, according to Embodiment 63 wherien the compound is SEQ ID NO:305.
65. A compound, or pharmaceutically acceptable salt thereof, according to Embodiment 63 wherien the compound is SEQ ID NO:307.
66. A compound, or pharmaceutically acceptable salt thereof, according to Embodiment 63 wherien the compound is SEQ ID NO:308.
67. A compound, or pharmaceutically acceptable salt thereof, according to Embodiment 63 wherien the compound is SEQ ID NO:392.
68. A compound, or pharmaceutically acceptable salt thereof, according to any one of Embodiments 1 to 67 wherein the compound is a partial agonist on the GLP-1R.
69. A compound, or pharmaceutically acceptable salt thereof, according to Embodiment 68 wherein the compound stimulates GLP-1R induced activation of Gαₛ in the GIPR and GLP-1R HEK293 Cell Membrane Guanosine 5'-(gamma-thio) Triphosphate-[³⁵S] (GTPγS) Binding Assay.
70. A compound, or pharmaceutically acceptable salt thereof, according to any one of Embodiments 68 or 69 wherein the compound is a partial agonist on the GLP-1R with respect to the β-arrestin-2 recruitment assay.
71. A method for treating a condition selected from the group consisting of type 2 diabetes mellitus, obesity, NAFLD, nonalcoholic steatohepatitis, dyslipidemia, and metabolic syndrome, comprising administering to a patient in need thereof, an effective amount of a compound, or a pharmaceutically acceptable salt thereof, according to any one of Embodiments 1 to 70.
72. A method for treating obesity, comprising administering to a patient in need thereof, an effective amount of a compound, or a pharmaceutically acceptable salt thereof, according to any one of Embodiments 1 to 70.
73. A method for providing therapeutic weight loss, comprising administering to a subject in need thereof, an effective amount of a compound, or a pharmaceutically acceptable salt thereof, according to any one of Embodiments 1 to 70.
74. A method for treating type 2 diabetes mellitus comprising administering to a subject in need thereof, an effective amount of the compound, or a pharmaceutically acceptable salt thereof, according to any one of Embodiments 1 to 70.
75. A pharmaceutical composition comprising the compound, or a pharmaceutically acceptable salt thereof, according to any one of Embodiments 1 to 70 and at least one pharmaceutically acceptable carrier, diluent, or excipient.
76. A pharmaceutical composition according to Embodiment 75 wherein the composition is administered as a subcutaneous injection.
77. A pharmaceutical composition according to Embodiment 75 wherein the composition is administered orally.
78. A pharmaceutical composition according to Embodiment 77 wherein the composition comprises a permeation enhancer and at least one pharmaceutically acceptable carrier, diluent, or excipient.
79. A pharmaceutical composition according to Embodiment 78 wherein the permeation enhancer is selected from the group consisting of sodium decanoate ("C10"), sodium taurodeoxycholate ("NaTDC"), lauroyl carnitine ("LC"), dodecyl maltoside ("C12-maltoside"), dodecyl phosphatidylcholine ("DPC"), sodium taurodeoxycholate ("NaTDC"), and a Rhamnolipid.
80. A pharmaceutical composition according to Embodiment 79 wherein the permeation enhancer is selected from the group consisting of C10 and LC.
81. A pharmaceutical composition according to Embodiment 80 wherein the permeation enhancer is C10.
82. A pharmaceutical composition according to any one of Embodiments 77 to 81 wherein the composition comprises a permeation enhancer and a protease inhibitor, and at least one pharmaceutically acceptable carrier, diluent, or excipient.
83. A pharmaceutical composition according to Embodiment 82 wherein the protease inhibitor is selected from the group consisting of soybean trypsin inhibitor ("SBTI"), soybean trypsin-chymotrypsin inhibitor ("SBTCI"), ecotin, sunflower trypsin inhibitor ("SFTI"), leupeptin, citric acid, ethylenediaminetetraacetic acid ("EDTA"), sodium glycocholate and 4-(2-aminoethyl) benzenesulfonyl fluoride hydrochloride ("AEBSF").
84. A pharmaceutical composition according to Embodiment 83 wherein the protease inhibitor is selected from the group consisting of SBTI, SBTICI, and SFTI.
85. A pharmaceutical composition according to Embodiment 84 wherein the protease inhibitor is SBTI.
86. A pharmaceutical composition according to any one of Embodiments 77 to 85 wherein the composition is a monolithic formulation.
87. A pharmaceutical composition according to any one of Embodiments 77 to 85 wherein the composition is a multiparticulate formulation.
88. A pharmaceutical composition according to any one of Embodiments 77 to 85 wherein the composition is a capsule or tablet.
89. A pharmaceutical composition according to Embodiment 86 wherein the composition is an enteric capsule or tablet.
90. A compound, or a pharmaceutically acceptable salt thereof, according to any one of Embodiments 1 to 70 for use as a medicament.
91. A compound, or a pharmaceutically acceptable salt thereof, according to any one of Embodiments 1 to 70 for use in treating a disease selected from the group consisting of treating type 2 diabetes mellitus, obesity, NAFLD, NASH, dyslipidemia and metabolic syndrome.
92. A compound, or a pharmaceutically acceptable salt thereof, according to any one of Embodiments 1 to 70 for use in treating treating type 2 diabetes mellitus.
93. The use of a compound, or a pharmaceutically acceptable salt thereof, according to any one of Embodiments 1 to 70 in the manufacture of a medicament for treating a condition selected from the group consisting of type 2 diabetes mellitus, obesity, NAFLD, NASH, dyslipidemia and metabolic syndrome.
94. The use of a compound, or a pharmaceutically acceptable salt thereof, according to any one of Embodiments 1 to 70 in the manufacture of a medicament for treating type 2 diabetes mellitus.
95. A method for treating diabetes comprising administering an effective amount of a compound showing partial agonism of 75% or less in the GLP-1R HEK293 Cell Membrane Guanosine 5'-(gamma-thio) Triphosphate-[³⁵S] (GTPγS) Binding Assay, and an effective amount of a compound that is a GIP agonist.
96. A method according to Embodiment 95 wherein the diabetes is type 2 diabetes.
97. A method according to any one of Embodiments 95 or 96 wherein a compound showing partial agonism in the GLP-1R HEK293 Cell Membrane Guanosine 5'-(gamma-thio) Triphosphate-[³⁵S] (GTPγS) Binding Assay is co-administered with a compound having GIP agonist activity.
98. A method according to any one of Embodiments 95 to 97 wherein a compound showing partial agonism in the GLP-1R HEK293 Cell Membrane Guanosine 5'-(gamma-thio) Triphosphate-[³⁵S] (GTPγS) Binding Assay is co-administered with a compound of a compound showing 35% or less in the GLP-CHO Cell β-Arrestin.Recruitment Assay.
99. A method according to any one of Embodiments 95 to 98 wherein a compound showing partial agonism in the GLP-1R HEK293 Cell Membrane Guanosine 5'-(gamma-thio) Triphosphate-[³⁵S] (GTPγS) Binding Assay is administered as an active agent within one week before or after a compound having GIP agonist activity.
100. A method according to any one of Embodiments 95 to 98 wherein a compound showing partial agonism in the GLP-1R HEK293 Cell Membrane Guanosine 5'-(gamma-thio) Triphosphate-[³⁵S] (GTPγS) Binding Assay is administered as an active agent within one week before or after a compound having GIP agonism and showing showing 35% or less in the GLP-CHO Cell β-Arrestin.Recruitment Assay.

## Claims

1. A compound of the formula:
R1X1X2X3GTX6TSDX10X11X12X13X14DX16X17AX19X20X21X22X23X24X25X26X27X28X2 ₉X₃₀X₃₁ (SEQ ID NO:3) wherein:
R₁ is absent;
X₁ is D-Tyr;
X₂ is Aib;
X₃ is E;
X₆ is F;
X₁₀ is Y;
X₁₁ is S;
X₁₂ is I;
X₁₃ is αMeL;
X₁₄ is L;
X₁₆ is K;
X₁₇ is K conjugated to a C16-C22 fatty acid via a linker, wherein the linker is (2-[2-(2-amino-ethoxy)-ethoxy]-acetyl)ₐ-(γ-Glu)_{b}, wherein a is 1 or 2 and b is 1 or 2;
X₁₉ is Q;
X₂₀ is Aib;
X₂₁ is E;
X₂₂ is F;
X₂₃ is I;
X₂₄ is E;
X₂₅ is αMeY;
X₂₆ is L;
X₂₇ is I;
X₂₈ is A;
X₂₉ is G;
X₃₀ is G;
X₃₁ is PX₃₂X₃₃X₃₄X₃₅X₃₆X₃₇X₃₈X₃₉-R₂ (SEQ ID NO:5), wherein:
X₃₂ is S;
X₃₃ is S;
X₃₄ is G;
X₃₅ is A;
X₃₆ is P;
X₃₇ is P;
X₃₈ is P;
X₃₉ is S;
R₂ is a modification of the C-terminal group, wherein the modification is NH₂;
or a pharmaceutically acceptable salt thereof.

2. The compound, or a pharmaceutically acceptable salt thereof, according to claim 1, wherein the C16-C22 fatty acid is a saturated C16-C22 diacid.

3. The compound, or a pharmaceutically acceptable salt thereof, according to claim 1 or claim 2, wherein a is 1 and b is 1.

4. The compound or a pharmaceutically acceptable salt thereof, according to any one of claims 1 to 3, wherein the C16-C22 fatty acid is CO-(CH₂)_{q}-CO₂H, wherein q is 16 or 18.

5. The compound or a pharmaceutically acceptable salt thereof, according to claim 4, wherein a is 1, b is 1 and q is 18 (SEQ ID NO:387).

6. The compound, or a pharmaceutically acceptable salt thereof, according to any one of claims 1 to 5, for use as a medicament.

7. The compound, or a pharmaceutically acceptable salt thereof, according to any one of claims 1 to 5, for use in treating a condition selected from the group consisting of type 2 diabetes mellitus, obesity, nonalcoholic fatty liver disease (NAFLD), nonalcoholic steatohepatitis, dyslipidemia, and metabolic syndrome.

8. The compound, or a pharmaceutically acceptable salt thereof, according to any one of claims 1 to 5, for use in treating obesity.

9. The compound, or a pharmaceutically acceptable salt thereof, according to any one of claims 1 to 5, for use in providing therapeutic weight loss.

10. A pharmaceutical composition comprising the compound, or a pharmaceutically acceptable salt thereof, according to any one of claims 1 to 5 and at least one pharmaceutically acceptable carrier, diluent, or excipient.
